# EUROPEAN PATENT APPLICATION

(11) **EP 2 481 735 A1**
(43) Date of publication of application: **01.08.2012**
(21) Application number: 12165102.0
(22) Date of filing: 29.04.2008
(51) Int. Cl.: C07D 401/06, C07D 401/14, C07D 405/06, C07D 409/14, C07D 413/14, C07D 417/06, C07D 417/14, C07D 471/04, A61K 31/4709, A61P 31/04

(54) **Substituted heterocyclic derivatives and compositions and their pharmaceutical use as antibacterials**

(30) Priority: 09.05.2007 US 916906 P
(62) Divisional of application: 08737576.2
(71) Applicant: Pfizer Inc., New York, NY 10017 (US)
(72) Inventor: Brickner, Steven Joseph, Groton, CT 06340 (US); Zhang, Zhijun, Groton, CT 06340 (US); Chen, Jinshan Michael, Groton, CT 06340 (US); Li, Zhengong Bryan, Groton, CT 06340 (US); Marfat, Anthony, Groton, CT 06340 (US); Reilly, Usa, Groton, CT 06340 (US); Mitton-Fry, Mark Joseph, Groton, CT 06340 (US); Plotkin, Michael Aaron, Groton, CT 06340 (US); Robinson, Shaughnessy, Groton, CT 06340 (US); Subramanyam, Chakrapani, Groton, CT 06340 (US)
(74) Representative: Lane, Graham Mark Hamilton

(57) **Abstract**

Compounds of the general Formula (I), 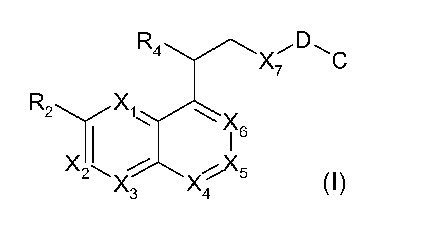 wherein X¹, X², X³, X⁴, X⁵, X⁶, X⁷, R¹, R², R⁴, R⁵, R⁶, R⁷, R⁸, R⁹, R¹⁰, C, D, Y¹, n, m, p and q are defined as in the description, their preparation and their use as antimicrobial agents.

## Description

### BACKGROUND

Antibacterial resistance is a global clinical and public health problem that has emerged with alarming rapidity in recent years. Resistance is a problem in the community as well as in health care settings, where transmission of bacteria is greatly amplified. Because many pathogens exhibit multiple drug resistance, physicians are now confronted with infections for which there is no effective therapy. In particular, infection with multi-drug resistant Gram-positive pathogens such as, methicillin-resistant *Staphylococcus aureus* (MRSA) and vancomycin-resistant enterococcus (VRE), is associated with increased patient morbidity and mortality as well as greater health care costs. Thus increasing antibacterial resistance represents a significant clinical, social and economic challenge and is a principle motivation in the search for new antibacterial agents.

Type II topoisomerases regulate the conformational changes in DNA by catalyzing the breaking and rejoining of DNA strands during replication. Bacterial type II topoisomerases, i.e. DNA gyrase and/or topoisomerase IV, are paralogous enzymes with significant amino acid sequence similarities; however, each enzyme plays a critical, but distinct, role during replication. Inhibiting the catalytic activities of bacterial DNA gyrase and/or topoisomerase IV (topo IV) is an attractive strategy for developing new antibiotics, since both gyrase and topo IV are necessary for DNA replication and, ultimately, bacterial cell growth and division.

### SUMMARY

One aspect of the present disclosure relates to compounds having the structure of Formula I, 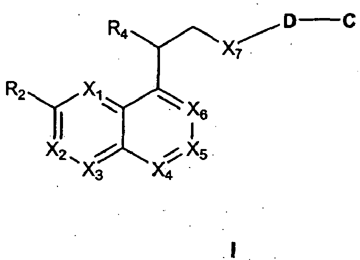 or a pharmaceutically acceptable salt or prodrug thereof or a hydrate or solvate of such compound, salt or prodrug wherein:
at least one of X₁, X₂, X₃, X₄, X₅, or X₆ is selected from N or *N*-oxide and the remaining are independently selected from N or CR₁;
each R₁ is independently selected from hydrogen, halogen, cyano, (C₁-C₆)alkyl, (C₁-C₆)alkoxy, halo(C₁-C₆)alkyl, amino, hydroxyl, thiol, or (C₁-C₆)alkylthio;
R₂ is independently selected from hydrogen, hydroxyl, halogen, amino, (C₁-C₆)alkyl, (C₁-C₆)alkylthio, (C₃-C₁₀)cycloalkyl, (C₃-C₁₀)cycloalkoxy, (C₃-C₁₀)cycloalkyl(C₁-C₆)alkyl, (C₁-C₆)alkyl(C₃-C₁₀)cycloalkyl, (C₂-C₉)heterocycloalkyl, (C₂-C₉)heterocyclo(C₁-C₆)alkyl, (C₆-C₁₀)aryloxy, (C₂-C₉)heterocycloxy, (C₂-C₉)heterocyclo(C₁-C₆)alkoxy, (C₁-C₆)alkoxy, (C₁-C₆)alkoxy(C₁-C₆)alkyl, fluoromethyl, difluoromethyl, trifluoromethyl, fluoromethoxy, difluoromethoxy, trifluoromethoxy, (C₃-C₁₀)cycloalkyloxy, (C₃-C₁₀)cycloalkylthio, (C₁-C₆)acyloxy, cyano, or nitro, where any of the aforementioned groups (with the exception of hydrogen, halogen, cyano, hydroxyl, and nitro) is optionally substituted with at least one moiety selected from (C₁-C₆)alkyl, (C₃-C₁₀)cycloalkyl, (C₁-C₆)alkoxy, (C₃-C₁₀)cycloalkoxy, (C₆-C₁₀)aryl, (C₅-C₉)heteroaryl, carboxyl, (C₁-C₆)alkyloxycarbonyl, (C₃-C₁₀)cycloalkyloxycarbonyl, (C₁-C₆)acyl, halogen, halo(C₁-C₆)alkyl, halo(C₁₋C₆)alkoxy, (C₁-C₆)alkylsulfonyl, aminocarbonyl, mono- or di-(C₁-C₆)alkyl)aminocarbonyl, hydroxyl, (C₂-C₉)heterocycloxy, (C₆-C₁₀)aryloxy, or (C₁-C₆)acyloxy;
X₇ is selected from O, NR₅, CH₂, -S-, SO, or SO₂ or -CR₅H-;
R₄ is selected from hydrogen, hydroxyl, (C₁-C₆)alkoxy, fluoro, NH₂, ((C₁-C₆)alkyl)NH- , ((C₁-C₆)alkyl)₂N-, (C₂-C₉)heterocycloalkyl, cyano, or (C₁-C₆)alkylthio;
R₅ is selected from hydrogen, (C₁-C₆)alkyl, (C₁-C₆)alkyloxycarbonyl, aminocarbonyl, (C₁-C₆)alkylsulfonyl, or (C₁-C₆)alkylcarbonyl;
D is 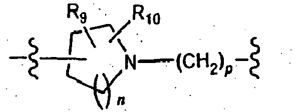
C is selected from 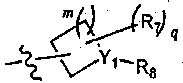 wherein 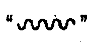 indicates a point of attachment;
Y₁ is CR₆ where R₆ is selected from hydrogen, hydroxyl, halogen, (C₁-C₆)alkyl or R₇; or
Y₁ is N;
wherein one of the carbon ring atoms of each of the foregoing C ring groups, together with the group to which it is attached, may optionally be replaced by -C(O)-;
each R₇ is independently selected from hydrogen, halogen, hydroxyl, (C₁₋C₆)alkyl, (C₁-C₆)alkoxy, trifluoromethyl, trifluoromethoxy, or amino provided that when Y₁ is N and R₇ is hydroxyl, (C₁-C₆)alkoxy, amino, trifluoromethoxy, or halogen R₇ may not be located on an atom adjacent to Y₁;
R₈ is selected from (C₆-C₁₀)aryl, (C₆-C₁₀)aryloxy, (C₆-C₁₀)aryl(C₁-C₆)alkyl, (C₆-C₁₀)aryl(C₁-C₆)alkoxy, (C₃-C₁₀)cycloalkyl, (C₃-C₁₀)cycloalkoxy, (C₃-C₁₀)cycloalkyl(C₁-C₆)alkoxy, C₃-C₁₀)cycloalkyl(C₁-C₆)alkyl, (C₅-C₉)heteroaryl(C₁-C₆)alkyl, (C₅-C₉)heteroaryl, (C₅-C₉)heteroaryl(C₁-C₆)alkoxy, (C₅-C₉)heteroaryloxy, (C₃-C₁₀)cycloalkoxy(C₁-C₆)alkyl, (C₂-C₉)heterocycloalkyl, (C₂-C₉)heterocycloxy, (C₂-C₉)heterocyclo(C₁-C₆)alkyl, (C₂-C₉)heterocyclo(C₁-C₆)alkoxy, where any of the aforementioned groups may be optionally substituted with 1 to 4 moieties each independently selected from halogen, (C₁-C₆)alkyl, (C₁-C₆)alkoxy, (C₁-C₆)alkoxy(C₁-C₆)alkyl, carboxyl, halo(C₁-C₆)alkyl, halo(C₁-C₆)alkoxy, thiol, (C₁-C₆)alkylthio, hydroxyl, nitro, cyano, amino, mono- or di-(C₁-C₆)alkylamino, (C₆-C₁₀)aryl, (C₅-C₉)heteroaryl, (C₁-C₆)alkoxycarbonyl, (C₁-C₆)alkoxycarbonyl(C₁-C₆)alkyl, (C₃-C₁₀)cycloalkyl, (C₁-C₆)alkylcarbonyl, (C₁-C₆)alkylsulfinyl, (C₁-C₆)alkylsulfonyl, aminocarbonyl, mono- and di-(C₁-C₆)alkylaminocarbonyl, (C₁-C₆)acylthio, or (C₁-C₆)acyloxy;
or R₇ and R₈ together with the atoms to which they are bonded form a three to eight membered saturated or unsaturated or aromatic ring system that may be monocyclic or bicyclic, wherein said ring system may optionally contain at least one heteroatom selected from nitrogen, oxygen or sulfur, and wherein said ring system may be optionally substituted with 1 to 4 moieties each independently selected from hydroxyl, halogen, cyano, (C₁-C₆)alkyl, halo(C₁-C₆)alkyl, (C₁-C₆)alkoxy, halo(C₁-C₆)alkoxy, (C₃-C₁₀)cycloalkyl, (C₃-C₁₀)cycloalkoxy, formyl, (C₁-C₆)acyl, (C₁-C₆)alkoxycarbonyl, (C₂-C₉)heterocycloalkyl, (C₆-C₁₀)aryl, or (C₅-C₉)heteroaryl;
R₉ is selected from carboxyl, (C₁-C₆)alkoxycarbonyl, aminocarbonyl, (C₁-C₆)alkylaminocarbonyl, (C₁-C₆)alkylsulfonylaminocarbonyl, hydroxyl, hydroxymethyl, or tetrazole;
R₁₀ is selected from hydrogen, halogen, hydroxyl, (C₁-C₆)alkyl or halo(C₁-C₆)alkyl ;
*n* is 0, 1, 2, or 3;
*m* is 0, 1, 2, or 3;
*p* is 0 or 1; and
*q* is 0, 1 or 2.

Certain other aspects of the disclosure relate to specific embodiments of compounds of Formula I wherein **D** is selected from:

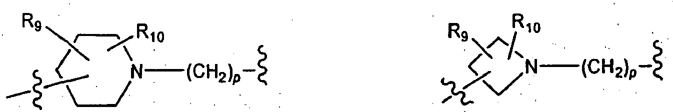

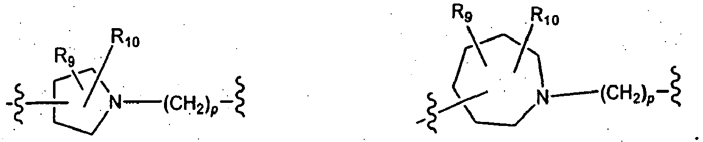

Certain other aspects of the disclosure relate to specific embodiments of compounds of Formula I wherein **C** is selected from: 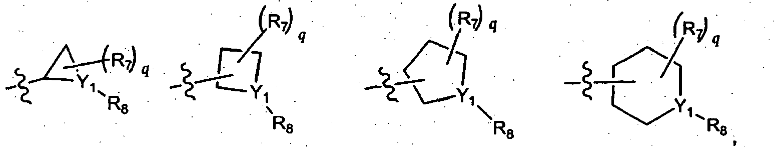 wherein one of the carbon ring atoms of each of the foregoing C ring groups, together with the group to which it is attached, may optionally be replaced by -C(O)-;

Further aspects of the disclosure relate to specific embodiments of compounds of Formula I wherein **D** is selected from: 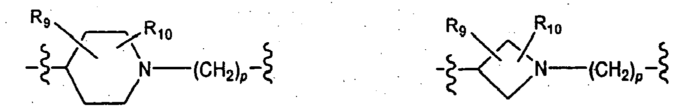 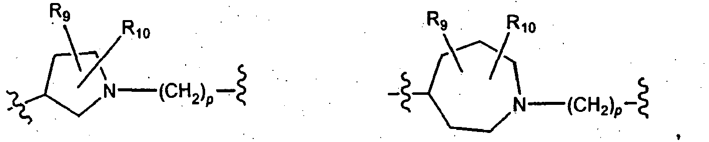 and **C** is selected from:

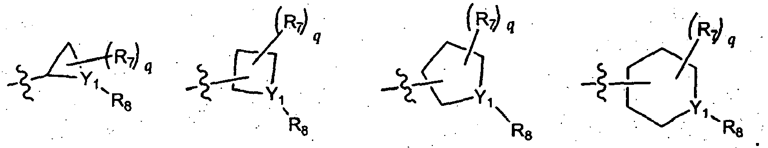

Still other aspects of the disclosure relate to specific embodiments of compounds of Formula I wherein any one or two of X₁, X₂, X₃, X₄, X₅ or X₆ is selected from N or *N*-oxide wherein if any one of X₁, X₂, X₃, X₄, X₅ or X₆ is *N-*oxide the remaining are selected from N or CR₁; **D** is selected from: 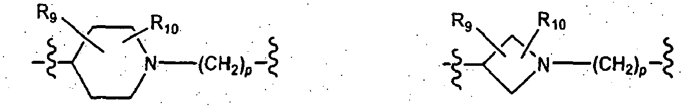 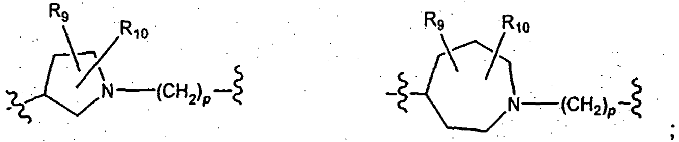 and C is selected from:

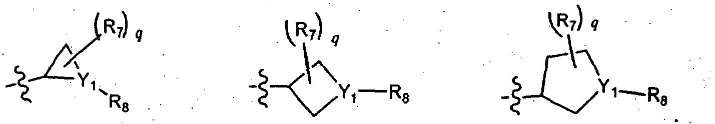

Additional aspects of the disclosure relate to specific embodiments of compounds of Formula I wherein at least X₄ is N or *N-*oxide; R₂ is (C₁-C₆)alkoxy or difluoromethoxy; R₄ is selected from hydrogen, hydroxyl, cyano, (C₁-C₆)alkoxy, fluoro, NH₂, ((C₁-C₆)alkyl)NH-, ((C₁-C₆)alkyl)₂N or (C₂-C₉)heterocycloakyl; and
R₈ is selected from: (C₆-C₁₀)aryl, (C₆-C₁₀)aryl(C₁-C₆)alkyl, (C₆-C₁₀)aryloxy, (C₆-C₁₀)aryl(C₁-C₆)alkoxy, (C₃-C₁₀)cycloalkyl, (C₃-C₁₀)cycloalkyl(C₁-C₆)alkyl, (C₃-C₁₀)cycloalkyl(C₁-C₆)alkoxy, (C₅-C₉)heteroaryl, (C₅-C₉)heteroaryl(C₁-C₆)alkyl, (C₂-C₉)heterocycloalkyl, (C₂-C₉)heterocyclo(C₁-C₆)alkyl, (C₂-C₉)heterocyclo(C₁-C₆)alkoxy or (C₅-C₉)heteroaryloxy, where any of the aforementioned groups is optionally substituted with 1 to 4 moieties each independently selected from halogen, (C₁-C₆)alkyl, (C₁-C₆)alkoxy, (C₁-C₆)alkoxy(C₁-C₆)alkyl, cyano, halo(C₁-C₆)alkyl, halo(C₁-C₆)alkoxy, or hydroxyl; or
R₇ and R₈ together with the atoms to which they are bonded form at least a 5 membered spirocyclic ring or at least a 5 membered carbocyclic, heterocyclic, aromatic or heteroaromatic ring, wherein any of the aforementioned ring systems may be monocyclic or bicyclic, wherein said ring system may optionally contain at least one heteroatom selected from nitrogen, oxygen or sulfur, and wherein said ring system is optionally substituted with 1 to 4 moieties each independently selected from halogen, cyano, (C₁-C₆)alkyl, (C₃-C₁₀)cycloalkyl, (C₃-C₁₀)cycloalkyl(C₁-C₆)alkyl, (C₂-C₉)heterocycloalky, (C₆-C₁₀)aryl, or (C₅-C₉)heteroaryl.

Yet another group of embodiments include compounds of Formula I wherein: at least X₄ is N or *N*-oxide; R₂ is (C₁-C₆)alkoxy or difluoromethoxy; Y₁ is N;
C is selected from 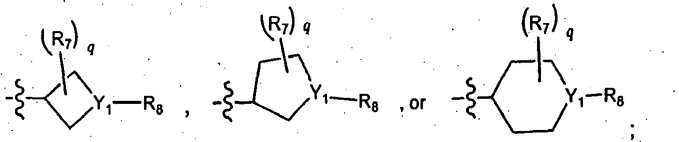 and R₈ is (C₅-C₉)heteroaryl, (C₅-C₉)heteroaryl(C₁-C₆)alkyl, (C₂-C₉)heterocycloalkyl, (C₃-C₁₀)cycloalkyl, or (C₆-C₁₀)aryl(C₁-C₆)alkyl where any of the aforementioned groups is optionally substituted with 1 to 4 moieties each independently selected from halo, cyano, (C₁-C₆)alkyl, (C₁-C₆)alkoxy, halo(C₁-C₆)alkyl, halo(C₁-C₆)alkoxy or hydroxyl.

Other aspects of the present disclosure relate to methods for preparing compounds of Formula I, intermediates and starting materials such as substituted 3-fluoroquinolines.

Additional aspects of the present disclosure relate to the use of compounds of Formula I in treating and/or preventing bacteria infections in mammals, including humans.

still other aspects of the present disclosure relate to pharmaceutical compositions comprising a therapeutically effective amount of at least one compound of Formula I, or a pharmaceutically acceptable salt, prodrug or hydrate or solvate of such compound, prodrug or salt, either alone or in combination with a second agent, and a pharmaceutically acceptable carrier, vehicle, diluent or excipient. The pharmaceutical composition comprising a combination of at least one compound of Formula I and a second agent may be administered as part of the same or separate dosage forms, via the same or different routes of administration, and on the same or different administration schedules according to standard pharmaceutical practice.

Further aspects of the present disclosure relate to methods of treating and/or preventing infections in mammals, including humans, comprising administering to said mammal in need of such treatment a therapeutically effective amount of at least one compound of the present invention or a pharmaceutically acceptable salt, prodrug or hydrate or solvate of such compound, prodrug or salt, either alone or in combination with a second agent, and a pharmaceutically acceptable carrier, vehicle, diluent or excipient.

The compounds and the prodrugs, salts, hydrates, solvates, pharmaceutical compositions and combinations thereof as described herein are useful for the treatment or prevention of infections associated with a variety of Gram-positive pathogens, including multi-drug resistant organisms and infections that require long-term therapy (>28 days).

In one embodiment, the invention relates to a compound of Formula I selected from any one of the compounds exemplified in Examples 1-229, or pharmaceutically acceptable salts, hydrates, solvates or prodrugs thereof.

In another embodiment, the invention relates to a compound of Formula I selected from the group consisting of:
(3R,4R)-4-(3-hydroxy-3-(6-methoxyquinolin-4-yl)propyl)-(3-phenylcyclobutyl)piperidine-3-carboxylic acid;
(3R,4R)-4-(3-(3-fluoro-6-methoxyquinolin-4-yl)-3-hydroxypropyl)-1-(3-phenylcyclobutyl)piperidine-3-carboxylic acid;
(3R,4R)-4-(3-(3-chloro-6-methoxyquinolin-4-yl)-3-hydroxypropyl)-1-(3-phenylcyclobutyl)piperidine-3-carboxylic acid;
(3R,4R)-1-(3-(2,6-difluorophenyl)cyclobutyl)-4-(3-hydroxy-3-(6-methoxyquinolin-4-yl)propyl)piperidine-3-carboxylic acid;
(3R,4R)-1-(3-(2,6-difluorophenyl)cyclobutyl)-4-(3-(3-fluoro-6-methoxyquinolin-4-yl)-3-hydroxypropyl)piperidine-3-carboxylic acid;
(3R,4R)-4-(3-(3-chloro-6-methoxyquinolin-4-yl)-3-hydroxypropyl)-1-(3-(2,6-difluorophenyl)cyclobutyl)piperidine-3-carboxylic acid;
(3R,4R)-1-(3-(2,6-difluorophenyl)cyclobutyl)-4-(3-(3-fluoro-6-methoxy-1,5-naphthyridin-4-yl)-3-hydroxypropyl)piperidine-3-carboxylic acid;
(3R,4R)-1-(3-(2,5-difluorophenyl)cyclobutyl)-4-((S)-3-hydroxy-3-(6-methoxyquinolin-4-yl)propyl)piperidine-3-carboxylic acid;
(3R,4R)-1-(3-(2,5-difluorophenyl)cyclobutyl)-4-((S)-3-(3-fluoro-6-methoxyquinolin-4-yl)-3-hydroxypropyl)piperidine-3-carboxylic acid;
(3R,4R)-4-(3-(3-chloro-6-methoxyquinolin-4-yl)-3-hydroxypropyl)-1-(3-(2,5-difluorophenyl)cyclobutyl)piperidine-3-carboxylic acid;
(3R,4R)-1-(3-(2,5-difluorophenyl)cyclobutyl)-4-(3-(3-fluoro-6-methoxy-1,5-naphthyridin-4-yl)-3-hydroxypropyl)piperidine-3-carboxylic acid;
(3R,4R)-1-[3-(3,5-difluorophenyl)cyclobutyl]-4-[(3S)-3-hydroxy-3-(6-methoxyquinolin-4-yl)propyl]piperidine-3-carboxylic acid;
(3R,4R)-1-[3-(3,5-difluorophenyl)cyclobutyl]-4-[(3S)-3-(3-fluoro-6-methoxyquinolin-4-yl)-3-hydroxypropyl]piperidine-3-carboxylic acid;
3-(3-(3-chloro-6-methoxyquinolin-4-yl)propyl)-1-(3-(2,5-difluorophenyl)cyclobutyl)pyrrolidine-3-carboxylic acid; and
(3R,4R)-1-[3-(3,5-difluorophenyl)cyclobutyl]-4-[3-(3-fluoro-6-methoxy-1,5-naphthyridin-4-yl)-3-hydroxypropyl]piperidine-3-carboxylic acid.

### Definitions

The carbon atom content of the various hydrocarbon-containing moieties herein may be indicated by a prefix designating the minimum and maximum number of carbon atoms in the moiety. For example, (Cₐ-C_{b})alkyl indicates an alkyl moiety of the integer "a" to the integer "b" carbon atoms, inclusive.

As used herein, the terms "alkyl" and "(C₁-C₆)alkyl" refer to monovalent hydrocarbon radicals containing the requisite number of carbon atoms as described above, having straight or branched moieties or combinations thereof. As used herein, alkyl groups may be optionally substituted with between one to four substituents. Non-limiting examples of alkyl groups include, e.g. methyl, ethyl, n-propyl, isopropyl, n-butyl, isobutyl, sec-butyl, t-butyl, etc. Of course, other alkyl groups will be readily apparent to those of skill in the art given the benefit of the present disclosure.

As used herein, the terms "alkoxy" and "(C₁-C₆)alkoxy" refer to monovalent hydrocarbon radicals containing the requisite number of carbon atoms as described above, having straight or branched moieties or combinations thereof, bonded to an oxygen atom. Non-limiting examples of alkoxy groups include, e.g. methoxy, ethoxy, *tert*-butoxy, etc. Of course, other alkoxy groups will be readily apparent to those of skill in the art given the benefit of the present disclosure.

As used herein, the term "aromatic" refers to monocyclic and polycyclic ring systems containing 4n+2 pi electrons, wherein n is an integer. As used herein, aromatic refers to and includes ring systems that contain only carbon atoms (i.e. "aryl") and ring systems that contain at least one heteroatom selected from N, O or S (i.e. "heteroaromatic" or "heteroaryl"). As used herein, aromatic ring systems may be optionally substituted with between one to four substituents.

As used herein, the terms "aryl" and "(C₆-C₁₀)aryl" refer to monocyclic and polycyclic aromatic hydrocarbon ring systems which may be optionally substituted with between one to four substituents. Non-limiting examples include phenyl and napthyl.

As used herein, the terms "carbocyclic" and "carbocycle" refers to monocyclic and polycyclic ring systems that contain only carbon atoms in the ring(s), without regard to aromaticity, and may be optionally substituted with between one to four substituents. As used herein, carbocyclic refers to and includes ring systems that are saturated or unsaturated, aryl or non-aryl, as well as ring systems having aromatic and/or non-aromatic portions. The term carbocyclic further includes bridged, fused and spirocyclic ring systems. Non-limiting examples of carbocylic groups include, e.g. cyclopropyl, cyclobutyl, 1,3-dimethylcyclopentyl, cyclohexyl, phenyl, napthyl, cyclohexenyl, 2,3-dihydro-indenyl, spiro[3.4]octanyl, bicyclo[2.2.1]heptanyl, etc. Of course, other carbocyclic groups will be readily apparent to those of skill in the art given the benefit of the present disclosure.

As used herein, the terms "halo" and "halogen" include fluorine, chlorine, bromine, and iodine atoms and substituents.

As used herein, the terms "haloalkyl" and "halo(C₁-C₆)alkyl" refer to alkyl groups, as defined above, having one or more hydrogen atoms replaced by halogen atoms, as defined above. It should be understood that where there is more than one halogen atom present in a haloalkyl group, the halogen atoms may be the same or different and/or may be located on the same or different carbon atoms. Non-limiting examples of haloalkyl groups include, e.g. difluoromethyl, trifluoromethyl, chloromethyl, 3-bromo-2-chloro-propyl, 2, 2-dibromoethyl, 2-bromo-2-chloro-ethyl, etc. Of course, other haloalkyl groups will be readily apparent to those of skill in the art given the benefit of the present disclosure.

As used herein, the terms "haloalkoxy" and "halo(C₁-C₆)alkoxy" refer to haloalkyl groups, as defined above, bonded to an oxygen atom. Non-limiting examples of haloalkoxy groups include, e.g. difluoromethoxy, trifluoromethoxy, chloromethoxy, 2,2-dibromoethoxy, 3-bromo-2-chloro-propoxy, etc. Of course, other haloalkoxy groups will be readily apparent to those of skill in the art given the benefit of the present disclosure.

As used herein, the terms "cycloalkyl" and "(C₃-C₁₀)cycloalkyl" refer to monocyclic and polycyclic hydrocarbon ring systems that may be optionally substituted with between one to four substituents. The term cycloalkyl includes ring systems that are saturated or unsaturated as well as polycyclic ring systems with unsaturated or aromatic portions. It should be understood that the term cycloalkyl further refers to and includes fused polycyclic structures such as, for example, bicyclo[3.2.1]octanyl, bicyclo[5.2.0]nonanyl and the like, as well as spirocyclic ring systems such as, for example, spiro[3.4]octanyl, spiro[3.5]nonyl and the like. Other non-limiting examples of cycloalkyl groups include, e.g. cyclopropyl, methylcyclopropyl, cyclobutyl, cyclobutenyl, isopropylcyclobutyl, cyclopentyl, 1,3-dimethylcyclopentyl, cyclohexyl, cyclohexenyl, cycloheptyl, 2,3-dihydro-1*H*-inden-2-yl, norbornyl, decahydronaphthalenyl, etc. Of course, other cycloalkyl groups will be readily apparent to those of skill in the art given the benefit of the present disclosure.

As used herein, the terms "cycloalkoxy" and "(C₃-C₁₀)cycloalkoxy" refer to a cycloalkyl group, as defined above, bonded to an oxygen atom. As used herein, a cycloalkoxy group may be optionally substituted with between one to four substituents. Non-limiting examples of cycloalkoxy groups include, e.g. cyclopropyloxy, cyclobutyloxy, cyclopentyloxy, etc. Of course, other cycloalkoxy groups will be readily apparent to those of skill in the art given the benefit of the present disclosure.

As used herein, the terms "heterocycloalkyl", "(C₂-C₉)heterocycloalkyl", "heterocycle" and "heterocyclic" refer to monocyclic and polycyclic ring systems containing at least one heteroatom selected from N, O, or S, and include ring systems that are saturated or unsaturated as well as polycyclic ring systems with unsaturated and/or aromatic portions. It should be understood that polycyclic heterocycloalkyl groups further include fused, bridged and spirocyclic ring systems. As used herein, a heterocycloalkyl group may be optionally substituted with between one to four substituents. Non-limiting examples of heterocycloalkyl groups include, e.g. oxiranyl, thiaranyl, aziridinyl, oxetanyl, thiatanyl, azetidinyl, tetrahydrofuranyl, tetrahydrothiophenyl, pyrrolidinyl, dihydrofuranyl, tetrahydropyranyl, pyranyl, tetrahydrothiopyranyl, thiopyranyl, piperidinyl, 1,4-dioxanyl, 1,4-oxathianyl, morpholinyl, thiomorpholinyl, 1,4-dithianyl, piperazinyl, 1,4-azathianyl, oxepanyl, thiepanyl, azepanyl, 1,4-dioxepanyl, 1,4-oxathiepanyl, 1,4-oxaazepanyl, 1,4-dithiepanyl, 1,4-thieazepanyl, 1,4- diazepanyl, 1,2-tetrahydrothiazin-2-yl, 1,3-tetrahydrothiazin-3-yl, tetrahydrothiadiazinyl, 1,2-tetrahydrodiazin-2-yl, 1,3-tetrahydrodiazin-1-yl, tetrahydroazepinyl, chromanyl, chromenyl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, 7-oxa-1-aza-spiro[4.4]nonanyl, 3-azabicyclo[3.1.0]hexanyl, indolinyl, octahydro-1H-indolyl, octahydro-2H-pyrido[1,2-a]pyrazinyl, 3-azabicyclo[4.1.0]heptanyl, etc. Of course, other heterocycloalkyl groups will be readily apparent to those of skill in the art given the benefit of the present disclosure.

As used herein, the terms "heterocycloxy" and "(C₂-C₉)heterocycloxy" refer to a heterocycloalkyl group, as defined above, bonded to an oxygen atom. As used herein, a heterocycloxy group may be optionally substituted with between one to four substituents. Non-limiting examples include, e.g. pyrrolidin-3-yloxy, piperidin-4-yloxy, azepan-4-yloxy, etc. Of course, other heterocycloxy groups will be readily apparent to those of skill in the art given the benefit of the present disclosure.

As used herein, the terms "heteroaryl", "(C₅-C₉)heteroaryl", and "heteroaromatic" refer to monocyclic and polycyclic aromatic ring systems containing at least one heteroatom selected from N, O, or S and may be optionally substituted with between one to four substituents. Non-limiting examples include, e.g., pyrrolyl, furanyl, thiophenyl, thienyl, pyrazolyl, imidazolyl, isoxazolyl, oxazolyl, isothiazolyl, thiazolyl, 1,2,3-triazolyl, 1,2,4-triazolyl, tetrazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridinyl, pyridazinyl, pyrimidinyl, pyrazinyl, 1,2,3-triazinyl, 1,3,5-triazinyl, pyrazolo[3,4-b]pyridinyl, cinnolinyl, pteridinyl, purinyl, 6,7-dihydro-5H-[1]pyrindinyl, benzo[b]thiophenyl, 5, 6, 7, 8-tetrahydro-quinolin-3-yl, benzoxazolyl, benzothiazolyl, benzisothiazolyl, benzisoxazolyl, benzimidazolyl, thianaphthenyl, isothianaphthenyl, benzofuranyl, isobenzofuranyl, isoindolyl, indolyl, indolizinyl, indazolyl, isoquinolyl, quinolyl, phthalazinyl, quinoxalinyl, quinazolinyl, benzoxazinyl, etc. Of course, other heteroaryl groups will be readily apparent to those of skill in the art given the benefit of the present disclosure.

As used herein, " 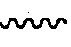 " indicates a point of attachment.

As used herein, the term "*N*-oxide" refers to an oxide of a tertiary amine or an oxide of an aromatic amine such as, for example, pyridine and can be represented as >N⁺-O⁻; >N=O; or >N→ O.

As used herein, the term "oxo" refers to a carbonyl group.

As used herein, the term "formyl" refers to a moiety of the formula HCO-.

As used herein, the term "(C₁-C₆)acyl" refers to a (C₁-C₆)alkylcarbonyl group, where (C₁-C₆)alkyl is as defined as above.

As used herein, the term "(C₁-C₆)acyloxy" refers to a (C₁-C₆)acyl group as defined above, bonded to an oxygen atom.

As used herein, the terms "epoxide" and "oxirane" refer to a specific heterocycloalkyl moiety having 3 ring members consisting of an oxygen atom and two carbon atoms.

As used herein, the term "thiol" refers to a moiety of the formula -SH.

The phrase "pharmaceutically acceptable" indicates that the designated carrier, vehicle, diluent, excipient, salt or prodrug is generally chemically and/or physically compatible with the other ingredients comprising a formulation, and is physiologically compatible with the recipient thereof.

The term "substituted" indicates that a hydrogen atom on a molecule has been replaced with a different atom or group of atoms. The atom or group of atoms replacing the hydrogen atom is denoted as a "substituent." It should be understood that the term "moiety" refers to substituent(s) when used in the phrase "optionally substituted by between one to four moieties ..." As used herein, non-limiting examples of substituents include, e.g. halogen, (C₁-C₆)alkyl, (C₁-C₆)alkoxy, (C₁-C₆)alkoxy(C₁-C₆)alkyl, carboxyl, formyl, (C₁-C₆)acyl, halo(C₁-C₆)alkyl, halo(C₁-C₆)alkoxy, thio, (C₁-C₆)alkylthio, hydroxyl, nitro, cyano, amino, mono- or di-(C₁-C₆)alkylamino, (C₆-C₁₀)aryl, (C₅-C₉)heteroaryl, (C₁-C₆)alkoxycarbonyl, (C₃-C₁₀)cycloalkyl, (C₃-C₁₀)cycloalkoxy, (C₂-C₉)heterocycloalkyl, (C₂-C₉)heterocycloxy, (C₁-C₆)alkoxycarbonyl(C₁-C₆)alkyl, (C₁-C₆)alkoxycarbonyl, (C₁-C₆)alkylsulfinyl, (C₁₋C₆)alkylsulfonyl, aminocarbonyl, mono- and di-(C₁-C₆)alkylaminocarbonyl, (C₁-C₆)acylthio, or (C₁-C₆)acyloxy, etc. Of course, other substituent groups will be readily apparent to those of skill in the art given the benefit of the present disclosure.

The terms "treating", "treated", and "treatment" as used herein include preventative (e.g., prophylactic), ameliorative, palliative and curative uses and/or results.

The phrases "therapeutic" and "therapeutically effective amount" as used herein denote an amount of a compound, composition or medicament that (a) treats or prevents a particular disease, condition or disorder; (b) attenuates, ameliorates or eliminates one or more symptoms of a particular disease, condition or disorder; (c) prevents or delays the onset of one or more symptoms of a particular disease, condition or disorder described herein. It should be understood that the terms "therapeutic" and "therapeutically effective" encompass any one of the aforementioned effects (a)-(c), either alone or in combination with any of the others (a)-(c).

Certain compounds of Formula I have two or more asymmetric centers and therefore can exist in a number of stereoisomeric configurations. Consequently, the compounds of the present invention can occur as mixtures of enantiomers and as individual (pure) enantiomers, as well as diastereomers and mixtures of different diastereomers. The present invention includes all such enantiomers and diastereomers and mixtures thereof in all ratios. In addition, compounds of Formula I include a cycloalkyl group about which geometric *cis*/*trans* isomers are possible. The scope of the present invention includes all stereoisomers, as well as all geometric isomers and tautomeric forms ("tautomers") of the compounds of Formula I, and all mixtures thereof in any ratio. It will be appreciated by one skilled in the art that a single compound may exhibit more than one type of isomerism.

Compounds of the present invention may be resolved into the pure enantiomers by methods known to those skilled in the art, for example by formation of diastereoisomeric salts which may be separated, for example, by crystallization; formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support with a bound chiral ligand or in the presence of a chiral solvent. It will be appreciated that where the desired stereoisomer is converted into another chemical entity by one of the separation procedures described above, a further step is required to liberate the desired enantiomeric form. Alternatively, the specific stereoisomers may be synthesized by using an optically active starting material, by asymmetric synthesis using optically active reagents, substrates, catalysts or solvents, or by converting one stereoisomer into the other by asymmetric transformation or inversion.

Wherein said compounds of the present invention contain one or more additional stereogenic centers, those skilled in the art will appreciate that all diastereoisomers and diastereoisomeric mixtures of the compounds illustrated and discussed herein are within the scope of the present invention. These diastereoisomers may be isolated by Methods known to those skilled in the art, for example, by crystallization, gas-liquid or liquid chromatography. Alternatively, intermediates in the course of the synthesis may exist as racemic mixtures and be subjected to resolution by methods known to those skilled in the art, for example by formation of diastereoisomeric salts which may be separated, for example, by crystallization; formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support with a bound chiral ligand or in the presence of a chiral solvent. It will be appreciated that where the desired stereoisomer is converted into another chemical entity by one of the separation procedures described above, a further step is required to liberate the desired enantiomeric form. Alternatively, specific stereoisomers may be synthesized by asymmetric synthesis using optically active reagents, substrates, catalysts or solvents, or by converting one stereoisomer into the other by asymmetric transformation or inversion.

Where a compound of the invention contains an alkenyl or alkenylene group, geometric cis/trans (or Z/E) isomers are possible. When such bonds are present, the compounds of the invention exist as cis and trans configurations and as mixtures thereof. Cis/trans isomers may be separated by conventional techniques well known to those skilled in the art, for example, chromatography and fractional crystallization.

Where structural isomers are interconvertible via a low energy barrier, tautomeric isomerism ('tautomerism') can occur. This can take the form of proton tautomerism in compounds of the invention containing, for example, an imino, keto, or oxime group, or so-called valence tautomerism in compounds which contain an aromatic moiety. It follows that a single compound may exhibit more than one type of isomerism. All such tautomeric forms are included within the scope of the present invention. Tautomers exist as mixtures of a tautomeric set in solution. In solid form, usually one tautomer predominates. Even though one tautomer may be described, the present invention includes all tautomers of the present compounds.

It should be understood that pharmaceutical compositions and methods of treatment that employ or contain compounds of Formula I, either by themselves or in combination with additional agents, similarly encompass all stereoisomers, geometric isomers and tautomeric forms of the compounds, and mixtures thereof in any ratio.

The compounds of the present invention may exist in unsolvated as well as solvated forms with pharmaceutically acceptable solvents such as water, ethanol, and the like. It should be understood that pharmaceutically acceptable solvents includes isotopically substituted solvents such as D₂O, d₆-DMSO and the like. The term 'solvate' is used herein to describe a complex comprising the compound of the invention and one or more pharmaceutically acceptable solvent molecules. It is intended that the present invention embrace unsolvated forms, solvated forms and mixtures of solvated forms.

Certain compounds of the present invention and/or their salts and/or solvates may exist in more than one crystal form. Polymorphs of compounds represented by Formula I are encompassed in the present invention and may be prepared by crystallization of a compound of formula I under different conditions such as, for example, using different solvents or different solvent mixtures; crystallization at different temperatures; various modes of cooling ranging from very fast to very slow during crystallization. Polymorphs may also be obtained by heating or melting a compound of Formula I followed by gradual or fast cooling. The presence of polymorphs may be determined by solid NMR spectroscopy, IR spectroscopy, differential scanning calorimetry, powder x-ray diffraction or other techniques.

The present invention also includes all pharmaceutically acceptable isotopically-labelled compounds, which are identical to those described by Formula I but wherein one or more atoms are replaced by atoms having an atomic mass or mass number different from the atomic mass or mass number usually found in nature. Examples of isotopes that may be incorporated into compounds of the invention include isotopes of hydrogen, carbon, chlorine, fluorine, iodine, nitrogen, oxygen, and sulfur, such as ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, and ³⁵S, respectively. It should be understood that compounds of the present invention, prodrugs thereof, and pharmaceutical acceptable salts of the compounds or of the prodrugs which contain the aforementioned isotopes and/or other isotopes of other atoms are within the scope of the invention. Certain isotopically labeled compounds of the present invention such as, for example, those incorporating a radioactive isotope such as ³H and ¹⁴C, are useful in drug and/or substrate tissue distribution studies. Tritium, *i*.*e*. ³H, and carbon-14, *i.e.* ¹⁴C, are particularly preferred due their ease of preparation and detection. Further, substitution with heavier isotopes such as deuterium, *i.e.* ²H, can afford certain therapeutic advantages resulting from greater metabolic stability, for example, increased *in vivo* half-life or reduced dosage requirements, and hence may be preferred in some circumstances. Isotopically labeled compounds of Formula I of this invention and prodrugs thereof can generally be prepared by carrying out the procedures disclosed in the Schemes and/or in the Examples by substituting a readily available isotopically labeled reagent for a non-isotopically labeled reagent.

The compounds of the invention may be isolated and used *per se* or in the form of their pharmaceutically acceptable salts or solvates. Pharmaceutically acceptable salts, as used herein in relation to the compounds of the present invention, include pharmacologically acceptable inorganic and organic salts of said compound. These salts can be prepared *in situ* during the final isolation and/or purification of a compound (or prodrug), or by separately reacting the compound (or prodrug) with a suitable organic or inorganic acid and isolating the salt thus formed. A pharmaceutically acceptable salt of a compound of Formula I may be readily prepared by mixing together solutions of the compound of Formula I and the desired acid or base, as appropriate. The salt may precipitate from solution and be collected by filtration or may be recovered by evaporation of the solvent. The degree of ionization in the salt may vary from completely ionized to almost non-ionized.

Representative salts include, but are not limited to, acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate and the like. Other examples of representative salts include alkali or alkaline earth metal cations such as sodium, lithium, potassium, calcium, magnesium, and the like, as well as non-toxic ammonium, quaternary ammonium and amine cations including, but not limited to, ammonium, tetramethylammonium, tetraethylammonium, lysine, arginine, benzathine, choline, tromethamine, diolamine, glycine, meglumine, olamine and the like. The invention further includes mixtures of salt forms.

In accordance with the present invention, compounds with multiple basic nitrogen atoms can form salts with a varying number of equivalents of acid. A practitioner of ordinary skill will readily appreciate that all such salts are within the scope of the invention.

Compounds of the present invention may be administered as prodrugs. The term "prodrug" refers to a compound that is transformed *in vivo* to yield a compound of Formula I or a pharmaceutically acceptable salt or solvate of the compound. The transformation may occur by various mechanisms, such as via hydrolysis in blood.

A prodrug of a compound of Formula I may be formed in a conventional manner with one or more functional groups in the compound, such as an amino, hydroxyl or carboxyl group. For example, if a compound of the present invention contains a carboxylic acid functional group, a prodrug can comprise: (1) an ester formed by the replacement of a hydrogen of the acid group with a group such as (C₁-C₆)alkyl or (C₆-C₁₀) aryl; (2) an activated ester formed by the replacement of the hydrogen of the acid group with groups such as -(CR₂)COOR', where CR₂ is a spacer and R can be groups such as H or methyl and R' can be groups such as (C₁-C₆)alkyl or (C₆-C₁₀) aryl; and/or (3) a carbonate formed by the replacement of the hydrogen of the acid with groups such as CHROCOOR' where R can be groups such as H or methyl and R' can be groups such as (C₁-C₆)alkyl or (C₆-C₁₀)aryl. Similarly, if a compound of the present invention contains an alcohol functional group, a prodrug can be formed via the replacement of the hydrogen of the alcohol with groups such as (C₁-C₆)alkanoyloxymethyl or (C₁-C₆)alkanoyloxyaryl or by forming an ester via condensation with, for example, an amino acid. Where a compound of Formula I contains a primary or secondary amino group, a prodrug may comprise, for example, an amide formed by the replacement of one or both of the hydrogen atoms of the amino group with (C₁-C₁₀)alkanoyl or (C₆-C₁₀)aroyl. Other prodrugs of amines are well known to those skilled in the art. Alternatively, certain compounds of Formula I may themselves act as prodrugs of other compounds of Formula I. Discussions regarding prodrugs and their the use can be found in, for example, "Prodrugs as Novel Delivery Systems," T. Higuchi and W. Stella, Vol. 14 of the ACS Symposium Series, and Bioreversible Carriers in Drug Design, Pergamon Press, 1987 (ed. E B Roche, American Pharmaceutical Association). Further examples of replacement groups in accordance with the foregoing examples and examples of other prodrug types may be found in the aforementioned references.

### DETAILED DESCRIPTION

The following provides additional non-limiting details of compounds of Formula I, including subgenuses and various species (embodiments) encompassed by Formula I.

In general, the compounds of Formula I may be prepared by methods that include processes known in the chemical arts, particularly in light of the description contained herein in combination with the knowledge of the skilled artisan. Although other reagents, compounds or methods can be used in practice or testing, generalized methods for the preparation of the compounds of Formula I are illustrated by the following descriptions, Preparations, and reaction Schemes. Other processes for the preparation of compounds of Formula I are described in the experimental section. The methods disclosed herein, including those outlined in the Schemes, Preparations, and Examples are for intended for illustrative purposes and are not to be construed in any manner as limitations thereon. Various changes and modifications will be obvious to those of skill in the art given the benefit of the present disclosure and are deemed to be within the spirit and scope of the present disclosure as further defined in the appended claims.

Unless otherwise indicated, the variables X₁, X₂, X₃, X₄, X₅, X₆, X₇, R₁, R₂, R₄, R₅, R₆, R₇, R₈, R₉, R₁₀, Y₁, *n, m, p* and *q* that appear in the Preparations and Schemes are defined as above or as defined in the Claims. Unless expressly defined otherwise, all technical and scientific terms used herein have the same meaning as commonly understood by one having ordinary skill in the art to which this disclosure belongs. Although specific embodiments of the present disclosure will be described with reference to the Schemes, Preparations and Examples, it should be understood that such embodiments are by way of example only and are merely illustrative of a small number of the many possible specific embodiments which can represent applications of the principles of the present disclosure.

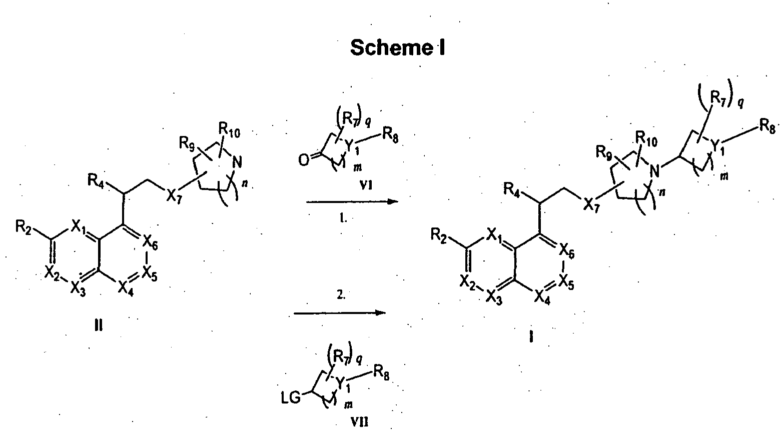

In Scheme **1** various compounds of Formula **I** may be prepared by condensing amine **II** with an appropriately substituted cyclic ketone **VI** as shown in reaction **1.** Such reductive amination reactions are well-known in the art. See, for example, Clinton F. Lane, Synthesis, 1975, 135-146. Typically, **II** and **VI** are combined with glacial acetic acid and 4A molecular sieves in an appropriate solvent or mixture of solvents, such as tetrahydrofuran and methanol. The reaction is allowed to stir at ambient temperature for between about 1 to about 6 hours, for example 3 hours, before the addition of a reducing agent such as sodium cyanoborohydride. The reaction mixture is again allowed to stir at 25°C for about 12 to about 18 hours, for example overnight. Where R₉ represents (C₁-C₆)alkoxycarbonyl, conversion to the corresponding carboxylic acid may be achieved using a strong inorganic base such as lithium hydroxide or sodium hydroxide in an appropriate solvent or solvent mixture such as for example tetrahyrofuran/methanol/water (1:1:1) for a sufficient period of time, usually between about 4 to 16 hours or, for example, overnight. Saponification is typically effected at a temperature of between about ambient temperature to about 100°C. For compounds where X₇ is O and R₄ is OH, the condensation reaction may be effected in an aprotic solvent such as N,N-dimethylformamide in the presence of a resin supported reducing agent such as MP-cyanoborohydride. The reaction is typically heated in a microwave at about 60°C to about 100°C for a suitable time, such as, for example about 1 hour.

Alternatively, various compounds of Formula I may be prepared via alkylation of amine **II** using an appropriately functionalized cyclic intermediate **VII**, where LG represents a suitable leaving group such as mesylate, as shown in reaction 2. Typically, where R₄ is oxo, **II** and **VII** are combined with a suitable organic base, such as triethyl amine, in the presence of potassium carbonate and an appropriate solvent or mixture of aprotic solvents, such as tetrahydrofuran and N,N-dimethylformamide. The reaction is allowed to stir for about 5 days at a temperature of between about 50°C to about 65°C. Conversion of R₄ to the corresponding alcohol is effected using a reducing agent such as sodium borohydride in a suitable solvent such as methanol. The reaction is allowed to stir at ambient temperature for a sufficient period of time, usually between about 1 hour to about 5 hours, before being quenched with the addition of water. Such reductions are well-known in the art. Where R₉ represents (C₁-C₆)alkoxycarbonyl, conversion to the corresponding carboxylic acid may be achieved using the general saponification conditions described above.

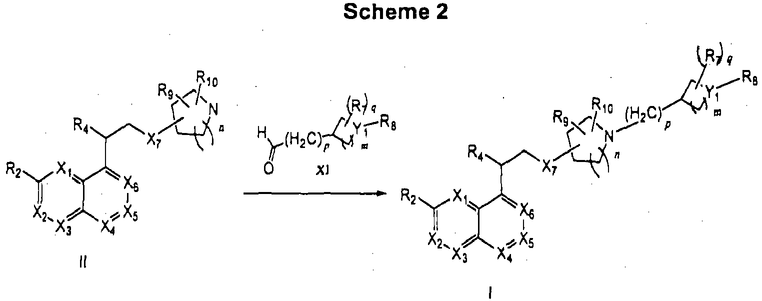

In Scheme 2, various compounds of Formula I may be prepared by condensing cyclic amine **II** with an appropriately substituted aldehyde **XI** using reductive amination procedures analogous to those described in reaction 1 of Scheme 1.

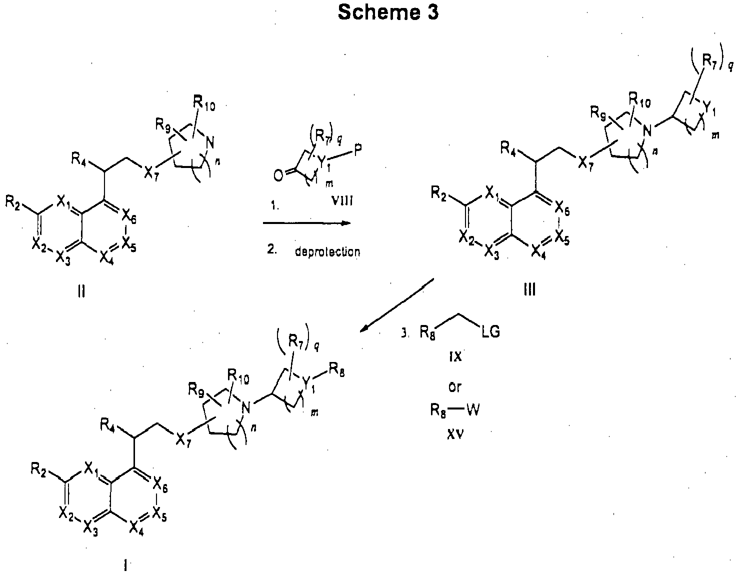

In Scheme 3, various compounds of Formula I may be prepared via alkylation of cyclic intermediate **III.** Intermediate **III** is the condensation product of cyclic amine **II** and cyclic ketone **VIII**, where Y₁ is nitrogen and P is a nitrogen protecting group such as, for example, *tert*-butoxycarbonyl. In reaction 1, the reductive amination is performed as described in reaction 1 of Scheme 1, after which the protecting group is removed according to procedures well-known in the art (reaction 2). In reaction 3, deprotected cyclic intermediate **III** is coupled with the appropriately substituted compound **IX,** where LG represents a suitable leaving group such as, for example, chlorine, in the presence of potassium hydrogen phosphate in a suitable solvent such as dimethyl sulfoxide. The reaction is allowed to stir for a suitable time, such as between about 48 to about 72 hours, at a temperature of between about 20° C to about 120°C. Alternatively, where W represents oxo, the deprotected cyclic intermediate **III** is condensed with the appropriately substituted oxo compound **XV** via a redactive amination reaction as described above. Where R₉ represents (C₁-C₆)alkoxycarbonyl, conversion to the corresponding carboxylic acid may be effected using the saponification conditions analogous to those described in Scheme 1.

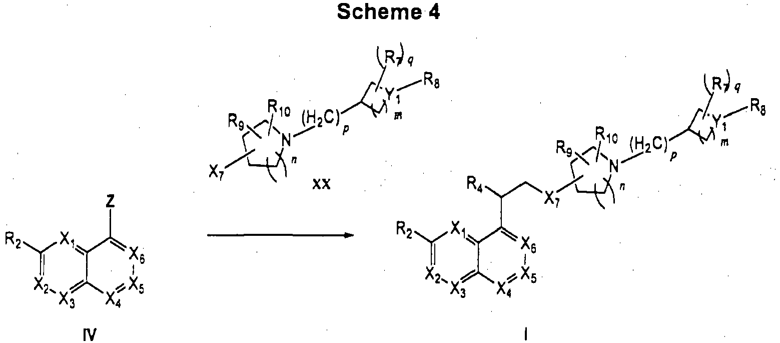

In Scheme 4, various compounds of Formula I may be prepared via reaction of cyclic intermediate **XX** with the appropriate bicyclic intermediate **IV** where Z may represent a number of functional groups. For example, where n is 3 and X₇ is NH₂, Z may represent an epoxide. The epoxide-opening reaction can be affected in a suitable solvent such as *tert*-butanol, at a temperature of between about 60° C to about 90° C. The reaction is allowed to proceed for a suitable time, such as between about 8 to about 18 hours. Where R₉ represents (C₁-C₆)alkoxycarbonyl, conversion to the corresponding carboxylic acid may be affected using a saponification procedure analogous to those described in Scheme 1. Alternatively, for compounds where, for example, m is 2 and X₇ is NH₂, Z may represent 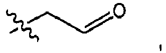 compounds of Formula I may be prepared via reductive animation. Typically, intermediates **IV** and **XX** are combined with acetic acid and 4A molecular sieves in an appropriate solvent or mixture of solvents, such as tetrahydrofuran and methanol and stirred at ambient temperature for about 1 hour to about 6 hours before a resin-supported hydride reagent such as MP-Cyanoborohydride is added. The reaction mixture is then stirred at ambient temperature for about 12 to about 18 hours, for example overnight. Where R₉ represents (C₁-C₆)alkoxycarbonyl, conversion to the corresponding carboxylic acid may be affected using a saponification procedure analogous to those described in Scheme 1.

Intermediate II depicted in Schemes 1-3 may be prepared by known methods or the non-limiting methods depicted in Schemes 5-7 below.

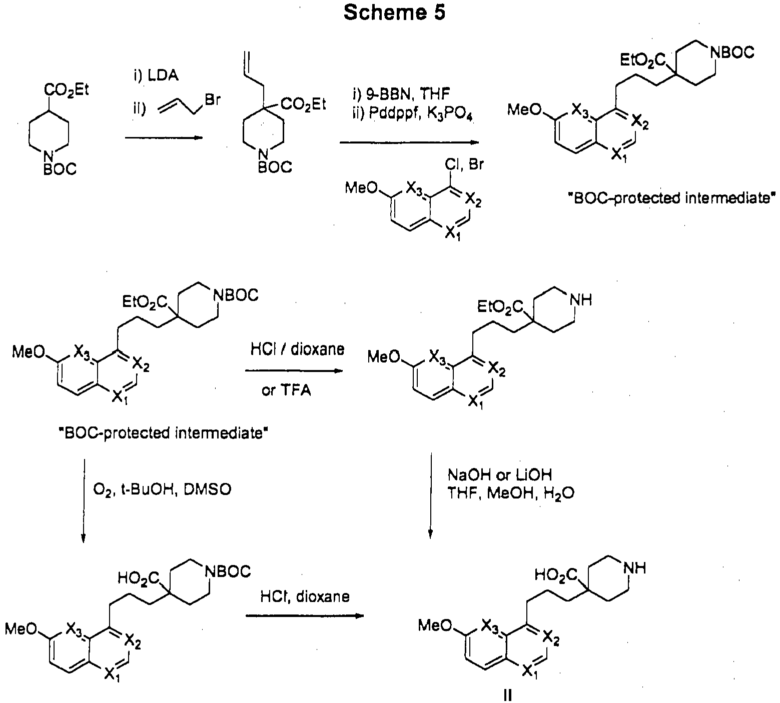

In Scheme 5, a BOC-protected ethyl piperidine-4-carboxylate is reacted first with lithium diisopropylamide (LDA) followed by reaction with allyl bromide. The product of this reaction is then reacted first with 9-borabicyclo (3.3.1) nonane (9-BBN) followed by reaction with the desired quinoline or quinoline analog in the presence of a palladium catalyst to form the BOC-protected intermediate. The BOC-protected intermediate may deprotected by reaction with acid followed by treatment with base (e.g., NaOH of LiOH) to form intermediate II. Alternatively, the BOC-protected intermediate may be oxidized then deprotected by reaction with acid to form intermediate II.

Scheme 6 depicts another method for making intermediates of formula II.

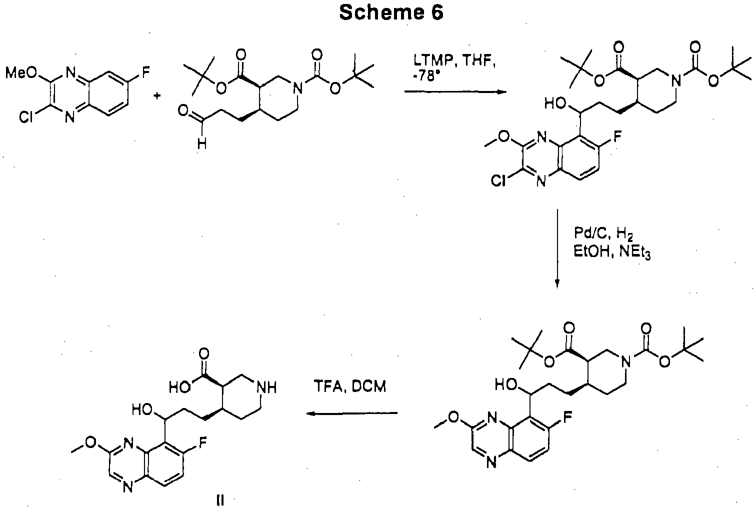

In Scheme 6 a compound such as 2-chloro-6-fluoro-3-methoxyquinoxaline (prepared according to J. Med. Chem. 1990, 33, 2240-2254) is allowed to react with an aldehyde such as (3R,4R)-di-tert-butyl 4-(3-oxopropyl)piperidine-1,3-dicarboxylate in the presence of lithium 2,2,6,6-tetramethylpiperidide (LTMP) to provide the 5-substituted quinoxaline shown. The 5-substituted quinoxaline is then dechlorinated under hydrogen atmosphere using palladium supported on carbon in the presence of triethylamine. The product is then hydrolyzed with trifluoroacetic acid (TFA) to provide (3R,4R)-4-(3-(6-fluoro-3-methoxyquinoxalin-5-yl)-3-hydroxypropyl)piperidine-3-carboxylic acid, an intermediate of formula II.

Scheme 7 depicts a method that may be used to make fluoroquinolines of formula II.

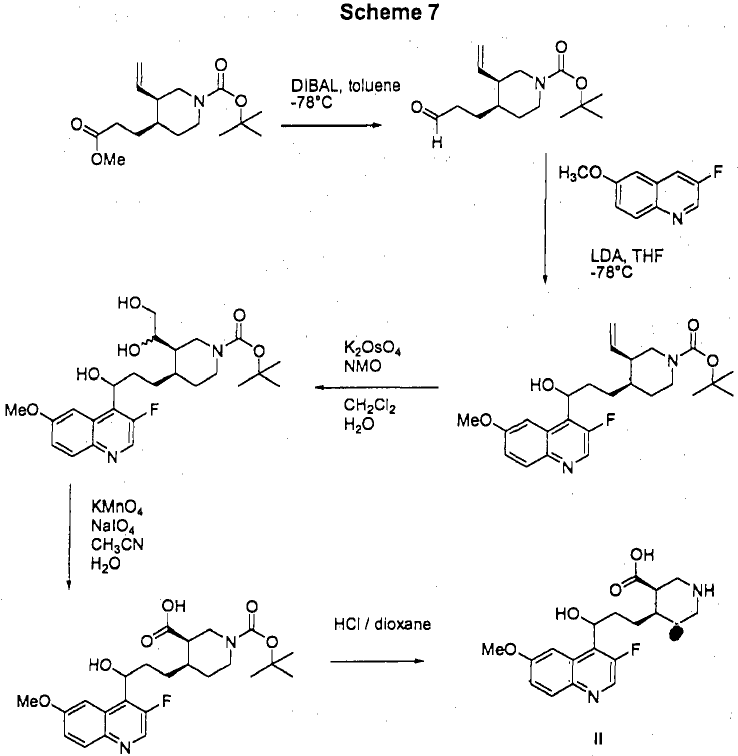

In Scheme 7, the aldehyde formed by the reaction of (3R,4R)-tert-butyl 4-(3-methoxy-3-oxopropyl)-3-vinylpiperidine-1-carboxylate with diisobutyl aluminum hydride (DIBAL) is allowed to react with a fluoroquinoline such as the 3-fluoro-6-methoxyquinoline shown in Scheme 7. Subsequent dihyrodroxylation with N-methylmorpholinoxide (NMO) and catalytic K₂OsO₄ is followed by cleavage with NalO₄ and catalytic KMnO₄ and finally by acid treatment in dioxane to provide the flouroquinoline intermediate II.

The compounds of formula I may be also prepared by prepared according the non-limiting procedure depicted in Scheme 8.

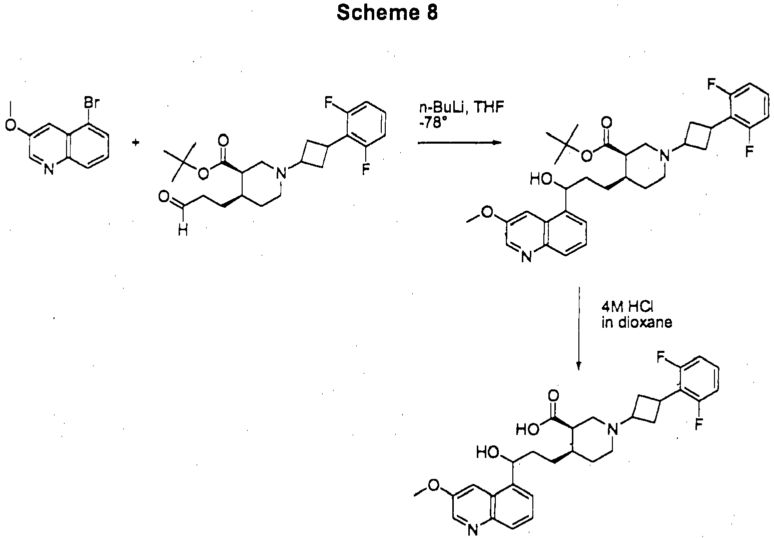

In Scheme 8 an aldehyde such as the (3R,4R)-tert-butyl 1-(3-(2,6-difluorophenyl)cyclobutyl)-4-(3-oxopropyl)piperidine-3-carboxylate is allowed to react with 5-bromo-3-methoxyquinoline to form a compound of formula I which is substituted at the 3 position of the piperidine ring with a t-butyl ester group. If desired, the ester can be hydrolyzed under acidic conditions to form the carboxylic acid analog of the compound of formula I.

Generalized methods for the preparation of intermediates **VI** and **XI** are described below in Preparations A and B. Preparation C describes a generalized method for the preparation of substituted 3-fluoroquinolines.

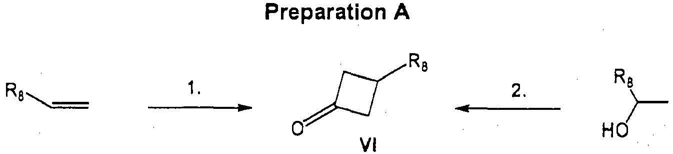

Preparation A illustrates two general routes for the preparation of cyclobutanone **VI.** In reaction 1, N,N-dimethylacetamide is treated with trifluoromethanesulfonic anhydride at a suitable temperature, such as about - 15°C, in an inert solvent such as 1,2 dichloroethane, before the simultaneous addition of the appropriate olefin and 2,4,6-collidine. The resulting reaction mixture is allowed to warm to ambient temperature and typically is heated to about 95°C, for between about 12 hours to about 72 hours. An alternative procedure for the preparation of cyclobutanone **VI** is illustrated in reaction 2, where after pretreating *N,N*-dimethylacetamide with trifluoromethanesulfonic anhydride as described for reaction 1, the appropriate alcohol and 2,4,6-collidine are simultaneously added to the reaction mixture. The reaction mixture is then typically heated to reflux for about 16 hours to 24 hours.

The preparation of various cyclobutanones of formula **VI** has also been described in J. Org. Chem. 1978, 43, 2879 and Organic Synthesis, Coll. Vol.8, p. 306 (1993); Vol. 69, p.199 (1990), and others are commercially available.

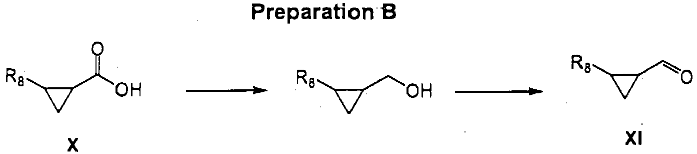

Preparation B illustrates the general preparation of aldehyde **XI** from a suitable carboxylic acid **X** according to known methods. Typically, carboxylic acid **X** is reduced to the corresponding alcohol using a reducing agent such as lithium aluminum hydride in a suitable solvent such as tetrahydrofuran. The reaction is conducted at a temperature of between about 50°C to about 70°C, for between about 6 to about 18 hours, for example, overnight. The alcohol product is then treated with an oxidizing agent such as Dess-Martin periodinane [1,1,1-tris(acetyloxy)-1,1-dihydro-1,2-benziodoxol-3-(1H)-one] in a suitable solvent such as methylene chloride or in a suitable solvent mixture such as methylene chloride and water. The reaction mixture is stirred at ambient temperature for a time period of between about 1 to about 18 hours, for example, overnight.

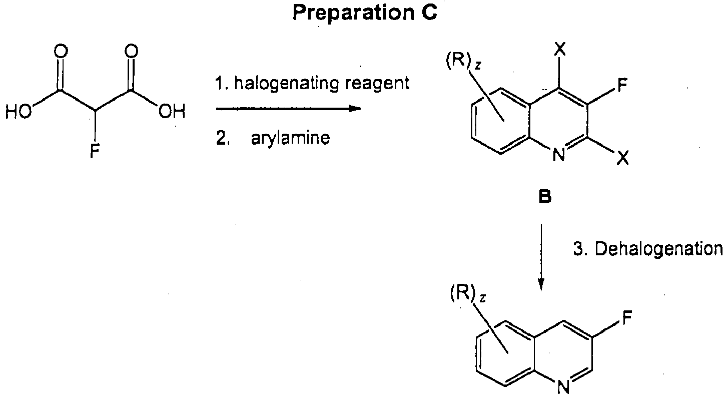

Preparation C illustrates a general reaction sequence for the preparation of substituted 3-fluoroquinolines which are precursors to specific intermediates of formula **IV.** Typically, 2-fluoromalonic acid is treated with a halogenating reagent, such as phosphorus oxychloride, and an appropriately functionalized arylamine, such as p-anisidine, to form the corresponding polyhalo-quinoline intermediate **B.** The 2-fluoromalonic acid may be prepared according to any number of known methods such as, for example, saponification of a diester of 2-fluoromalonate, such as dimethyl-2-fluoromalonate, using an inorganic base, such as lithium hydroxide, in a suitable solvent, such as methanol. Such saponification reactions are well known in the art. Various halogenating reagents may be used for this reaction which include but are not limited to: phosphorus oxychloride (POCl₃), oxalyl chloride (COCl)₂, thionyl chloride (SOCl₂), phosphorus pentachloride (PCl₅), sulfuryl chloride (SO₂Cl₂), phosphorus oxybromide (POBr₃), phosphorus pentabromide (PBr₅), oxalyl bromide (COBr)₂, and thionyl bromide (SOBr₂). Generally, the halogenating reagent is used as the solvent for the reaction and the functionalized arylamine is added to the reaction in portions. Once the addition is complete, the cyclization reaction occurs at a suitable temperature, such as between about 40°C to about 110°C. The resulting polyhalo-quinoline intermediate **B,** where X is halogen, z is 1, 2, 3, or 4 and each R may be independently selected from hydrogen, hydroxyl, amino, mono-or di(C₁-C₆)alkylamino, (C₁-C₆)alkyl, (C₁-C₆)alkyl optionally substituted with 1 or 2 halogen atoms, (C₁-C₆)alkoxy, (C₁-C₆)alkoxy(C₁-C₆)alkyl, (C₆-C₁₀)aryl, (C₅-C₉ )heteroaryl, (C₃-C₁₀)cycloalkyl, (C₆-C₁₀)aryloxy, (C₅-C₉)heteroaryloxy, is then dehalogenated in reaction 3 in the presence of a reagent such as, a metal catalyst or metal hydride, in a suitable solvent or mixture of solvents. The dehalogenation reaction is effected at a suitable temperature, such as for example, about 25°C, for an appropriate time, such as about 24 hours to about 55 hours. Exemplary reagents for the dehalogenation reaction include, but are not limited to, metal catalysts such as: palladium-on-carbon (Pd/C), palladium hydroxide-on-carbon (Pd(OH)₂/C), Raney Nickel, as well as metal hydrides such as, lithium aluminum hydride (LiAlH₄). Depending on the reagent used, hydrogen gas (H₂) may be needed to effect the reaction. For example, where z is one, R is methoxy and each X is chlorine, the dehalogenation reaction is conducted using Raney Nickel, 150 psi of hydrogen in a solution of methanol and ammonia. Alternatively, polyhalo-quinoline intermediate **B** may be dehalogenated without the use of hydrogen gas by using a metal hydride such as lithium aluminum hydride.

Various intermediates of formulas **II** and **IV** may be prepared using or adapting methods known in the art. For example, compounds of formula **IV** where Z is an epoxide may be prepared according to procedures described in Tetrahedron Letters 2004, 45, 3783 and Tetrahedron 1992, 48, 10515. Other epoxides of formula **IV** may be prepared from the corresponding carboxylic acids, which are either commercially available or are accessible via standard routes for the preparation of carboxy-heteroaromatics. For example, the carboxylic acid derivatives of various heteroaromatics such as quinazolines, napthyridines and pyridazines may be prepared using routes analogous to those described in Heterocyclic Compounds, 6, 324 (1957) and Comprehensive Heterocyclic Chemistry, Vols 2 and 3. Alternatively, various intermediates of formulas **II** and **IV** may be prepared in a manner that is analogous to procedures described in US 04/0198756, US 04/0198755, US 05/0032800, WO 00/21948, WO 99/37635 and/or WO 05/097781.

Other useful derivatives of formula **IV** where Z is hydroxyl may be prepared from the corresponding amino compounds or by other methods known in the art. For example, 4-hydroxy cinnolines may be prepared as described by Osbom and Schofield, J. Chem. Soc., 2100 (1955). Procedures for the conversion of hydroxy derivatives to halo and/or amino compounds are also well known in the art and are described in standard reference books such as, e.g., Compendium of Organic Synthetic Methods, Vols. I-VI (Wiley-Interscience). Intermediates of formulas **II** and **IV** not specifically described herein may in general be obtained by methods described in the references above in combination with the knowledge of one skilled in the art.

One of ordinary skill in the art will appreciate that in some cases protecting groups may be required during synthesis. After a particular target molecule or intermediate is made or at some specific step later in a synthetic route, the protecting group can be removed by methods well known to those of ordinary skill in the art, such as described in Greene and Wuts, Protective Groups in Organic Synthesis, (3rd Ed, John Wiley & Sons, 1999).

The compounds of the present disclosure intended for pharmaceutical use may be administered alone or in combination with one or more other compounds of the invention or in combination with one or more other drugs (or as any combination thereof). Generally, the compound(s) will be administered as a formulation in association with one or more pharmaceutically acceptable excipients. The term "excipient" is used herein to describe any ingredient other than the compound(s) of the invention and includes ingredients such as vehicles, carriers, diluents, preservatives and the like. The choice of excipient(s) will largely depend on factors such as the particular mode of administration, the effect of the excipient(s) on solubility and stability, and the nature of the dosage form. A pharmaceutical composition of the invention, for example, includes forms suitable for oral administration as a tablet, capsule, pill, powder, sustained release formulations, solution, suspension, or for parenteral injection as a sterile solution, suspension or emulsion. Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

In one preferred embodiment, the compounds of the invention may be administered orally. Oral administration may involve swallowing, so that the compound enters the gastrointestinal tract, or buccal or sublingual administration may be employed by which the compound enters the blood stream directly from the mouth. Formulations suitable for oral administration include solid formulations, such as tablets, capsules containing particulates, liquids, or powders; lozenges (including liquid-filled), chews; multi- and nano-particulates; gels, solid solution, liposome, films (including muco-adhesive), ovules, sprays and liquid formulations. Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet. The compounds of the invention may also be used in fast-dissolving, fast-disintegrating dosage forms such as those described in Expert Opinion in Therapeutic Patents, 11 (6), 981-986 by Liang and Chen (2001).

In another preferred embodiment, the compounds of the invention may be administered by parenteral injection. Exemplary parenteral administration forms include sterile solutions, suspensions or emulsions of the compounds of the invention in sterile aqueous media, for example, aqueous propylene glycol or dextrose. In another embodiment, the parenteral administration form is a solution. Such parenteral dosage forms can be suitably buffered, if desired.

Dosage regimens of the compounds and/or pharmaceutical composition of the invention may be adjusted to provide the optimum desired response. For example, a single bolus may be administered, several divided doses may be administered over time or the dose may be proportionally reduced or increased as indicated by the exigencies of the therapeutic situation. The appropriate dosing regimen, the amount of each dose administered and/or the intervals between doses will depend upon the compound of the invention being used, the type of pharmaceutical composition, the characteristics of the subject in need of treatment and the severity of the condition being treated.

Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a patient may also be determined, as can the temporal requirements for administering each agent to provide a detectable therapeutic benefit to the patient. Accordingly, while certain dose and administration regimens are exemplified herein, these examples in no way limit the dose and administration regimen that may be provided to a patient in practicing the present invention.

In general, a total daily dose for the compounds of the present disclosure is in the range of about 1.0 mg/day to about 5.0 grams/day, preferably about 100 mg/day to about 2.0 grams/day, of the compound of Formula I/saft/solvate/prodrug. The total daily dose may be administered in single or multiple doses. These dosages are based on an average human subject having a weight of about 65kg to 70kg. The physician or the individual responsible for dosing will readily be able to determine doses for subjects whose weight falls outside this weight range, such as infants and the elderly.

It should be noted that variation in the dosage will depend on the compound employed, the mode of administration, the treatment desired and the disorder (severity and type) to be treated or alleviated. The present invention also encompasses sustained release compositions and 'flash' formulations, i.e. providing a medication to dissolve in the mouth.

It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. For example, doses may be adjusted based on pharmacokinetic or pharmacodynamic parameters, which may include clinical effects such as toxic effects and/or laboratory values. Thus, the present invention encompasses intra-patient dose-escalation as determined by the skilled artisan. Determining appropriate dosages and regiments for administration of the chemotherapeutic agent are well-known in the relevant art and would be understood to be encompassed by the skilled artisan once provided the teachings disclosed herein.

A pharmaceutical composition of the invention may be prepared, packaged, or sold in bulk, as a single unit dose, or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, a pharmaceutical composition of the invention may comprise between 0.1% and 100% (w/w) active ingredient. In addition to the active ingredient, a pharmaceutical composition of the invention may further comprise one or more additional pharmaceutically active agents.

### PREPARATIVE EXAMPLES

The compounds described below and/or listed in Tables 2 through 10 are non-limiting Examples of compounds encompassed by Formula I that were prepared and characterized according to one or more of the procedures outlined below. The preparation of various intermediates is also described.

In the discussions below, the following abbreviations are used: BOC (*tert*-butoxycarbonyl), DMF (N,N-dimethylformamide), MeOH (methanol), MTBE (*tert*-butyl methyl ether), THF (tetrahydrofuran), DMAP (4-dimethylaminopyridine), DMSO (dimethyl sulfoxide), DCM (dichloromethane), CDCl₃, (deuterochloroform), D₆-DMSO (deuterodimethylsulfoxide), CD₃OD (deuteromethanol), EtOAc (ethyl acetate), Aq. (aqueous), EtOH (ethanol), DAST, ((diethylamino)sulfur trifluoride), sat. (saturated), AcOH (acetic acid), 25°C, *t*-BuOH (*tert*-butanol), TBDMS-CL (*tert*-butyldimethylsilyl chloride), TFFH (Fluoro-*N,N,N*'- tetramethylformamidinium hexafluorophosphate), NMP (1-methyl-2-pyrrolidinone), TFA (trifluoroacetic acid), ACN (acetonitrile), STAB (sodium triacetoxyborohydride), h (hours), TEA (triethyl amine), RP (reverse phase); DEA (diethylamine); MP-cyanoborohydride (Macroporous Polymer-bound cyanoborohydride) PS-IBX (*o*-iodoxybenzoic acid; resin supported); MP (medium pressure); MCX (Mixed-mode strong Cation exchange).

H¹ Nuclear magnetic Resonance (NMR) spectra were acquired on either a Varian AS-500 or VXR-400 instrument and were in all cases consistent with the proposed structures. Characteristic chemical shifts (δ) are given in parts-per-million using conventional abbreviations for the designation of major peaks: e.g., s, singlet; d, doublet; t, triplet; q, quartet; m, multiplet; br, broad.

The mass spectra (MS) data and retention times (minutes) included in Tables 2-9 were obtained using an automated Gilson LC-MS spectrometer, eluting with various mixtures of solvent A (98% H2O, 2% acetonitrile, 0.01% formic acid) and "solvent B" (acetonitrile with 0.005% formic acid) according to one of the following three protocols, standard, polar and nonpolar. Standard conditions are as follows: (1 mL/min flow rate) Time = 0 min: 95%A, 5% B; 1.05 min: 80%A, 20%B; 2.30 min: 50%A, 50%B, 3.55 min: 100%B; 3.76 min: run is over, return to starting conditions. Polar conditions are as follows: (1 mL/min flow rate) Time = 0 min: 100%A; 2.00 min: 80%A, 20%B; 3.50 min: 100%B; 3.75 min: 100%A; 3.76 min: run is over, return to starting conditions. Non-polar conditions are as follows: (1.3 mL/min flow rate) Time = 0 min: 100%A; 1.00 min: 20%A, 80%B; 2.25 min: 100%B; 3.75 min: 100%A; 3.76 min: run is over, return to starting conditions. The mass spectrum of the major eluting component was then obtained in positive or negative ion mode scanning a molecular weight range from 165 AMU to 1100 AMU.

Unless otherwise noted, HPLC data was acquired on a Hewlett Packard 1100 series using a Waters Symmetry C8 5µm 4.6 x 50 mm column. Preparatory HPLC purification was performed on a model SIL10A from Shimazu Scientific Instruments using either Exterra prep ms C₁₈ OBD 5*µ*m 19 x 50, Exterra prep ms C₁₈ OBD 5*µ*m 30 x 50 or Exterra prep ms C18 5*µ*m 50 x 100 columns. Chiral preparatory HPLC purifications can be performed using columns such as: Chiralcel OD-H, ChiralPakAD-H, and Chiralcel OJ-H. Microwave experiments were performed using a Biotage Initiator microwave apparatus. Chromatography refers to and includes column chromatography performed using 32-63 mm silica gel and a MP chromatography system such as ISCO or under nitrogen pressure (flash chromatography) conditions. Room or ambient temperature refers to 20-25°C. Unless stated otherwise, all nonaqueous reactions were run under a nitrogen atmosphere and commercial reagents were utilized without further purification. The terms 'concentration' or 'concentration at reduced pressure' or *'in* vacuo' mean that a rotary evaporator and/or vacuum pump were used.

In general, the Examples were prepared as mixtures of diastereomers where the absolute configuration at 1 or more centers may be undetermined or unconfirmed. Where included, ratios of diastereomers and isomeric products were measured directly from integration of ¹H NMR absorptions of protons common to the components or were determined using ¹⁹F NMR in similar fashion. Where possible, diastereomeric ratios were corroborated using HPLC.

### Preparation 1: (trans)-2-phenyl-cyclopropanecarbaldehyde (racemic)

Step 1: To a suspension of LiAlH₄ (702mg, 18.5mmol) in 60mL anhydrous THF was added a solution of (*trans*)-2-phenyl-1-cyclopropanecarboxylic acid (2.0g, 12.33mmol) in 10ml anhydrous THF. The reaction was stirred at 25°C overnight and subsequently quenched by the sequential addition of 0.7mL H₂O, 0.4ml of 6N aq. NaOH solution, and 2mL H₂O. The resulting suspension was stirred for 15 minutes and filtered to remove solids. The filtrate was concentrated and the residue dissolved in CHCl₃ and poured into H₂O. The aqueous layer was extracted with CHCl₃ (3 x). The organic extracts were combined, dried over MgSO₄, filtered and concentrated to afford 1.75 g of a clear oil.

Step 2: The product of Step 1 (1.65g, 11.1mmol) and Dess-Martin periodinane (5.2g, 12.25mmol) were combined in 25 mL DCM. The reaction was stirred at 25°C for 5 hours, diluted with DCM, poured into 1 N aq. NaOH solution and the layers separated. The aqueous layer was extracted with DCM (3 x). The organic extracts were combined, dried over MgSO₄, filtered and concentrated onto silica gel. The crude material was purified by chromatography (gradient elution from 1% to 100% EtOAc in heptane) to afford the title compound (1.3g) as a clear oil that solidified upon standing. ¹H NMR (400 MHz, CDCl₃) 1.49-1.55 (m, 1 H), 1.69-1.75 (m, 1H), 2.13-2.19 (m, 1H), 2.58-2.65 (m, 1 H), 7.08-7.12 (m, 2H), 7.18-7.31 (m, 3H), 9.31 (d, 1H).

### Preparation 2: General Procedures for the Preparation of 3-Substituted Cyclobutanones

### Method A:

1,2 dichloroethane (10 mL) and N,N-dimethylacetamide (10 mmol) were combined and cooled to -15 °C. To this was added, trifluoromethanesulfonic anhydride (11 mmol) drop-wise over 5 min, forming an opaque suspension. The reaction was stirred for an additional 15 min at - 15 °C before the simultaneous addition of the appropriate styrene or olefin (10 mmol) and 2,4,6-Collidine (10 mmol) via syringe. The mixture was allowed to warm to 25°C and then heated to reflux for 16 hours, whereupon the reaction was quenched with the addition of H₂O (10 mL) and stirred for 2 hours at 25°C. The organic layer was separated and the aqueous layer extracted with DCM (4 x 10 mL). The organics were combined, dried over MgSO₄, concentrated, and purified by MP chromatography (gradient elution using 0-15% EtOAC: Heptane) to afford the corresponding 3-cyclobutanone product.

### Method B:

Step 1: To a solution of 2,5-difluorobenzaldehyde (40.0 g) in anhydrous THF (140 mL) was added MeMgBr (3.0 M in diethyl ether, 103 mL) over 1 h at -78 °C under nitrogen, via dropping funnel. The reaction mixture was stirred at -78 °C for 1h, quenched with aqueous saturated NH₄Cl, warmed to 25°C and extracted with DCM (2 x 300 mL). The organic extracts were combined, dried over MgSO₄, filtered, concentrated and dried under vacuum to provide a 43g of a pale yellow oil (>90% purity by NMR).

Step 2: A solution of fresh N,N-dimethylacetamide (26.3 mL, HPLC grade) and molecular sieve (4A, 10 g, dried overnight in oven) in anhydrous 1,2-dichloroethane (100 mL) was cooled to -15°C under N₂. Tf₂O (50 mL, fresh bottle) was slowly added over 30 min via dropping funnel, resulting in a pale yellow suspension. The mixture was stirred for 15 min before the addition of a solution of 1-(2,5-difluorophenyl)ethanol (22.4 g, crude product of Step 1) and anhydrous 2,4,6-collidine (37.6 mL) in 1,2-dichloroethane(80 mL). The resultant mixture was warmed to r.t and refluxed for 48 h. The reaction was cooled to 25°C, treated with water (450 mL) and stirred for 3-4 h. The organic layer was separated and the aqueous layer extracted with DCM (100 mL). The organics were combined and concentrated. Purification on a 120 g ISCO silica gel column and elution with gradient EtOAc/Heptane (0% in 3 min, 0-50% in 47 min) provided 20 g of yellow oil. The material thus obtained was re-purified on a 120 g ISCO column, eluting with a EtOAc/Heptane gradient (0% in 3 min, 0-50% in 47 min) to afford 7.2 g of desired cyclobutanone product as a yellow oil.

Table 1 provides additional non-limiting cyclic ketones of general formula **VI** that were prepared in a manner analogous to that described in Preparation 2 using appropriate starting materials. Other cyclobutanones, such as 3-phenylcyclobutanone and 3-(4-chlorophenyl)cyclopentanone, are commercially available or can be prepared by the methods described in Example 225-230.

**Table 1. Non-limiting examples of cyclic ketones of general formula VI.**

| Cyclic ketone | ¹H NMR |
|---|---|
| 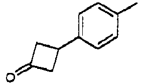 | ¹H NMR (400 MHz, CHLOROFORM-d) δ ppm 2.29 - 2.41 (m, 3 H) 3.16 - 3.32 (m, 2 H) 3.41 - 3.54 (m, 2 H) 3.62 (m, 1 H) 7.10 - 7.24 (m, 4 H) |
| 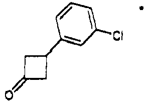 | ¹H NMR (400 MHz, CHLOROFORM-d) δ ppm 3.12 - 3.31 (m, 2 H) 3.42 - 3.55 (m, 2 H) 3.65 (m, Hz, 1 H) 7.16 - 7.30 (m, 4 H) |
| 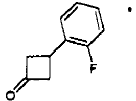 | ¹H NMR (400 MHz, CHLOROFORM-d) δ ppm 3.20 - 3.35 (m, 2 H) 3.43 - 3.57 (m, 2 H) 3.72 - 3.88 (m, 1 H) 7.00 - 7.16 (m, 2 H) 7.17 - 7.31 (m, 2 H) |
| 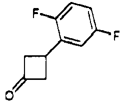 | ¹H NMR (400 MHz, CHLOROFORM-d) δ ppm 3.20 - 3.34 (m, 2 H) 3.41 - 3.56 (m, 2 H) 3.67 - 3.81 (m, 1 H) 6.84 - 7.07 (m, 3 H) |
| 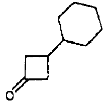 | ¹H NMR (400 MHz, CHLOROFORM-d) δ ppm 1.16 -1.40 (m, 4 H) 1.58 - 1.75 (m, 6 H) 2.05 (m, 1 H) 2.68 -2.78 (m, 2 H) 2.88 (m, 1 H) 2.97 - 3.08 (m, 2 H) |
| 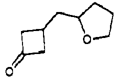 | ¹H NMR (400 MHz, CHLOROFORM-d) 1.68 (dd, J=13.50, 5.19 Hz, 1 H) 1.65 - 1.73 (m, 1 H) 1.77 - 1.90 (m, 1 H) 1.82 (dd, J=11.84, 6.85 Hz, 2 H) 1.95 (dd, J=8.10, 4.78 Hz, 1 H) 2.49 (d, J=8.31 Hz, 1 H) 2.61 -2.77 (m, 2 H) 2.83 (dd, J=17.65, 6.02 Hz, 1 H) |
| | 3.04 - 3.22 (m, 3 H) 3.71 - 3.84 (m, 1 H). |
| 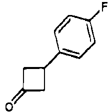 | ¹H NMR (400 MHz, CHLOROFORM-d) δ ppm 3.11 -3.27 (m, 2 H) 3.41 - 3.52 (m, 2 H) 3.64 (m, 1 H) 7.01 (t, J=8.52 Hz, 2 H) 7.24 (dd, J=8.52, 5.19 Hz, 2 H) |
| 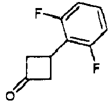 | ¹H NMR (400 MHz, CHLOROFORM-d) δ ppm 3.48 (d, J=8.31 Hz, 4 H) 3.93 (dq, J=8.52, 8.38 Hz, 1 H) 6.84 -6.94 (m, 2 H) 7.14 - 7.24 (m, 1 H) |
| 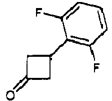 | ¹H NMR (400 MHz, CHLOROFORM-d) δ ppm 1.01 -1.19 (m, 3 H)1.49 - 1.60 (m, 1 H) 1.55 (m, 2 H) 1.97 (m, 1 H) 2.14 (m, 1 H) 2.39 - 2.49 (m, 2 H) 3.09 - 3.14 (m, 1 H) 3.26 (m, 1 H) |
| 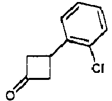 | ¹H NMR (400 MHz, CHLOROFORM-d) δ ppm 3.17 -3.33 (m, 2 H) 3.42 - 3.57 (m, 2 H) 3.94 (m, 1 H) 7.19 -7.41 (m, 4 H) |
| 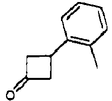 | ¹H NMR (400 MHz, CHLOROFORM-d) δ ppm 2.32 (s, 3 H) 3.16 - 3.30 (m, 2 H) 3.36 - 3.50 (m, 2 H) 3.76 (m, 1 H) 7.14 - 7.30 (m, 4 H) |
| 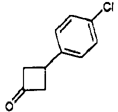 | ¹H NMR (400 MHz, CHLOROFORM-d) δ ppm 3.11 -3.27 (m, 2 H) 3.40 - 3.55 (m, 2 H) 3.65 (m, 1 H) 7.19 -7.27 (m, 2 H) 7.27 - 7.35 (m, 2 H) |
| 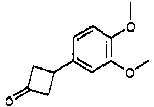 | ¹H NMR (400 MHz, CHLOROFORM-d) δ ppm 3.14 -3.27 (m, 2 H) 3.41 - 3.52 (m, 2 H) 3.63 (m, 1 H) 3.86 (s, 3 H) 3.88 (s, 3H) 6.78 (s, 1 H) 6.83 (s, 2 H) |
| 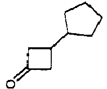 | ¹H NMR (500 MHz, CHLOROFORM-d) δ ppm 1.16 -1.35 (m, 2 H) 1.57- 1.73(m,4H) 1.80 (m, 2 H) 1.96 (m, 1 H) 2.28 (m, 1 H) 2.68 - 2.77 (m, 1 H) 3.06 - 3.14 (m, 1 H) |
| 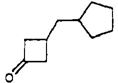 | ¹H NMR (400 MHz, CHLOROFORM-d) δ ppm 1.06 -1.18 (m, 1 H) 1.19 - 1.36 (m, 4 H) 1.47 - 1.81 (m, 6 H) 2.39 (m, 1 H) 2.57 - 2.72 (m, 2 H) 3.03 - 3.16 (m, 2 H) |
| 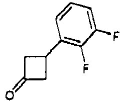 | ¹H NMR (400 MHz, CHLOROFORM-d) δ ppm 3.21 -3.40 (m, 2 H) 3.40 - 3.56 (m, 2 H) 3.82 (m, 1 H) 6.94 -7.16 (m, 3 H) |
| 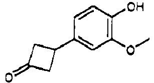 | ¹H NMR (400 MHz, CHLOROFORM-d) δ ppm 3.08 -3.29 (m, 2 H) 3.41 - 3.55 (m, 2 H) 3.64 (m, 1 H) 3.83 (s, 3 H) 6.86 (d, J=5.40 Hz, 2 H) 7.00 (m, 1 H) |
| 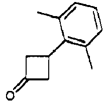 | ¹H NMR (400 MHz, CHLOROFORM-d) δ ppm 2.40 (s, 6 H) 3.38 - 3.56 (m, 4 H) 4.08 (m, 1 H) 6.99 - 7.08 (m, 3 H) |
| 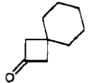 | ¹H NMR (400 MHz, CHLOROFORM-d) δ ppm 1.28 -1.42 (br, m., 6 H) 1.47 (m, 4 H) 2.56 (s, 4 H) |
| 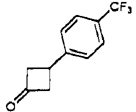 | ¹H NMR (400 MHz, CHLOROFORM-d) δ ppm 3.15 -3.32 (m, 2 H) 3.45 - 3.59 (m, 2 H) 3.73 (m, 1 H) 7.41 (d, J=7.89 Hz, 2 H) 7.60 (d, J=8.31 Hz, 2 H) |
| 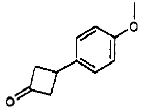 | ¹H NMR (400 MHz, CHLOROFORM-d) δ ppm 3.13 -3.23 (m, 2 H) 3.38 - 3.49 (m, 2 H) 3.61 (m, 1 H) 3.78 (s, 3 H) 6.87 (d, J=8.72 Hz, 2 H) 7.20 (d, J=8.72 Hz, 2 H) |
| 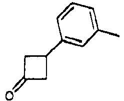 | ¹H NMR (400 MHz, CHLOROFORM-d) δ ppm 2.35 (s, 3 H) 3.16 - 3.29 (m, 2 H) 3.41 - 3.52 (m, 2 H) 3.63 (m, 1 H) 7.01 - 7.14 (m, 2 H) 7.08 (d, J=4.15 Hz, 1 H) 7.24 (t, J=7.48 Hz, 1 H) |
| 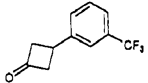 | ¹H NMR (400 MHz, CHLOROFORM-d) δ ppm 3.18 -3.31 (m, 2 H) 3.47 - 3.59 (m, 2 H) 3.74 (m, 1 H) 7.43 -7.58 (m, 4 H) |
| 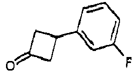 | ¹H NMR (400 MHz, CHLOROFORM-d) δ ppm 3.15 -3.30 (m, 2 H) 3.43 - 3.55 (m, 2 H) 3.67 (m, 1 H) 6.90 -7.02 (m, 2 H) 7.06 (d, J=7.48 Hz, 1 H) 7.25 - 7.36 (m, 1 H) |
| 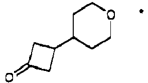 | ¹H NMR (400 MHz, CHLOROFORM-d) δ ppm 1.12 -1.36 (m, 4 H) 1.64 (m, 1 H) 2.05 (m, 1 H) 2.73 (m, 2 H) 3.08 (m, 2 H) 3.36 (m, 2H) 3.99 (m, 2H) |
| 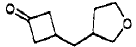 | ¹H NMR (400 MHz, CHLOROFORM-d) 1.48 - 1.65 (m, 2 H) 2.00 - 2.12 (m, 2 H) 2.19 (d, J=7.48 Hz, 1 H) 2.15 (d, J=7.06 Hz, 1 H) 2.36 (d, J=7.89 Hz, 1 H) 2.69 (td, J=13.60, 6.85 Hz, 2 H) 3.19 (d, J=7.48 Hz, 1 H) 3.10 -3.22 (m, 1 H) 3.70 - 3.83 (m, 1 H) 3.75 (d, J=7.48 Hz, 1 H) 3.83 - 3.94 (m, 2 H). |
| 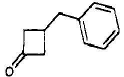 | ¹H NMR (400 MHz, CHLOROFORM-d) δ ppm 1.31 (d, 2 H), 1.59 (s, 1 H), 2.69 - 2.87 (m, 1 H), 2.92 (d, 1 H), 3.11 - 3.21 (m, 1 H), 3.23 - 3.43 (m, 2 H), 7.17 - 7.26 (m, 1 H), 7.28 - 7.40 (m, 4 H) |
| 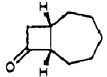 | ¹H NMR (400 MHz, CHLOROFORM-d) δ ppm 1.15 -1.36 (m, 3 H) 1.42 - 1.65 (m, 2 H) 1.81 (br. m., 2 H) 1.84 (m, 2 H) 1.93 (m, 1 H) 2.50 - 2.71 (m, 2 H) 3.15 (m, 1 H) 3.32 - 3.50 (m, 1 H) |
| 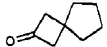 | ¹H NMR (500 MHz, CHLOROFORM-d) δ ppm 1.70 -1.76 (m, 4 H) 1.78 - 1.82 (m, 4 H) 2.93 (s, 4 H) |
| 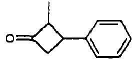 | ¹H NMR (400 MHz, CHLOROFORM-d) δ ppm 1.29 (d, J=7.06 Hz, 3 H) 3.15 (m, 1 H) 3.20 - 3.42 (m, 3 H) 7.25 (d, J=4.57 Hz, 1 H) 7.27 - 7.38 (m, 4 H) |
| 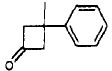 | ¹H NMR (400 MHz, CHLOROFORM-d) δ ppm 1.60 (s, 3 H) 3.06 - 3.19 (m, 2 H) 3.40 - 3.53 (m, 2 H) 7.16 (m, 1 H) 7.20 - 7.38 (m, 4 H) |

| | |
|---|---|
| * Refluxed 24 hrs. | |

### Preparation 3: 3-phenoxycyclobutanone

Step 1: To a 0 °C solution of 3-(benzyloxy)cyclobutanone (0.500g, 2.84 mmol) in THF (20 mL) was added LiAlH₄ (0.119g, 3.1 mmol). Following the addition, the reaction was allowed to warm to 25°C, stirred 3h, and quenched by addition of H₂O. The reaction was extracted with EtOAc and the organic layers combined, dried with MgSO₄, filtered, and concentrated to yield 0.500 g of a yellow oil.

Step 2: To a solution of the product of Step 1 (0.500g, 2.81 mmol) in DCM (40 mL) was added NEt₃ (0.980 mL, 7.03 mmol) followed by methanesulfonyl chloride (0.454 mL, 5.62 mmol). The reaction was stirred at 25°C for 30 minutes, then poured into H₂O and extracted with DCM. The organic layers were combined, dried with MgSO₄, filtered, and concentrated under reduced pressure to give 3-(benzyloxy)cyclobutyl methanesulfonate contaminated with minor impurities (0.79g). A portion of the crude material (0.25 g) was combined with phenol (0.092 g) and Cs₂CO₃ (0.434g) in DMF (2 mL) and heated overnight at 100 °C. The reaction was cooled to 25°C, diluted with H₂O, and extracted with EtOAc. The organic layers were combined, dried over MgSO₄, filtered, and concentrated. The crude product was purified by MPLC chromatography (gradient elution from 1-10% EtOAc in heptane) to give 0.115 g of a clear oil.

Step 3: The product of Step 2 (0.115 g, 0.452 mmol) and Pd black (0.049g, 0.452 mmol) were combined in a solution of formic acid (0.5 mL) in MeOH (10 mL) and stirred overnight. The catalyst was removed by filtration and the reaction concentrated under reduced pressure to afford 0.0823g of crude product.

Step 4: The product of Step 3 was combined with Dess Martin periodinane (0.211g) in DCM (5 mL) and stirred 3h at 25°C. The reaction was then poured into 1 M NaOH and extracted with DCM. The organic layers were combined, dried over MgSO₄, filtered, and concentrated under reduced pressure. The residue was dissolved in DCM and washed again with 1M NaOH. The combined organics were then dried over MgSO₄, filtered, and concentrated to give the title compound (0.0768 g) which was used without further purification. ¹H NMR (400 MHz, CDCl₃) 3.22-3.32 (m, 2H), 3.43-3.53 (m, 2H), 4.93-4.99 (m, 1H), 6.83-6.87 (m, 2H), 6.96-7.01 (m, 1H), 7.27-7.33 (m, 2H).

### Preparation 4: 3-(pyridin-2-yl)cyclobutanone

Step 1: To a cooled (0 °C) solution of *i*-PrMgCl in THF (2.0M, 6 mL) was added 2-bromopyridine (0,95 mL) drop-wise. Once the addition was complete, the reaction was allowed to warm to 25°C and stir for 3h. The reaction was then cooled to 0 °C whereupon a solid precipitated. Addition of THF (5 mL) and sonication provided a suspension to which 3-(benzyloxy)cyclobutanone (1.8g) was added drop-wise. After stirring 15 min, the reaction was quenched by addition of sat. aq. NH₄Cl and extracted with EtOAc. The organic phases were combined, dried (MgSO₄), filtered, and concentrated. Chromatography (gradient elution from 15-60% EtOAc in heptane) afforded an impure yellow oil that was chromatograhed again (gradient elution from 0 to 10% MeOH in CHCl₃). This operation provided 3-(benzyloxy)-1-(pyridin-2-yl)cyclobutanol as a light yellow oil of sufficient purity (0.4021 g) for use in the next step.

Step 2: To a cooled (-78 °C) solution of the product of Step 1 (0.40g) in DCM (5 mL) was added DAST (0.32 mL) drop-wise by syringe. After stirring 5 minutes at -78 °C, the reaction was allowed to warm to 0 °C and stir 75 minutes, whereupon it was quenched with 10 mL H₂O, diluted with EtOAc and the phases were separated. The organic phase was washed with sat. aq. NaHCO₃ and brine, then dried over MgSO₄, filtered, and concentrated. The crude product was purified by chromatography (gradient elution from 0-40% EtOAc in heptane) to afford 2-(3-(benzyloxy)-1-fluorocyclobutyl)pyridine as a faintly yellow oil (0.24g)( ca. 2:1 mixture of diastereomers).

Step 3: To a solution of the product of Step 2 (0.24g) in MeOH (20 mL) and formic acid (1 mL) was added Pd black (0.108g). The reaction was stirred vigorously under N₂. After ca. 1.5h, additional Pd black was added (0.13g) and the reaction stirred overnight. The reaction mixture was filtered through celite and concentrated. The residue was dissolved in EtOAc and washed with sat. aq. Na₂CO₃. The organic phase was dried over MgSO₄, filtered and concentrated to an oily residue. The residue was purified by chromatography (gradient elution from 0-20% MeOH in CHCl₃) to afford 3-(pyridin-2-yl)cyclobutanol in moderate purity (0.0648g).

Step 4: To a solution of the product of Step 3 (0.0648g) in DCM (4 mL) was added PS-IBX (0.46g, 1.2 mmol/g titer). The resulting mixture was sealed and stirred overnight at 25°C, then filtered and concentrated. The crude product was purified by MP chromatography (gradient elution from 50% EtOAc in heptane to 100% EtOAc) to provide the title compound (0.0118) as an oily residue. ¹H NMR (400 MHz, CDCl₃) 3.34-3.44 (m, 2H), 3.47-3.56 (m, 2H), 3.65-3.74 (m, 1 H), 7.13-7.17 (m, 1 H), 7.21-7.25 (m, 1H), 7.59-7.64 (m, 1H), 8.58 (d, 1H).

### Preparation 5: 3-(5-methyl-isothiazol-3-yl)-cyclobutanone

Step 1: A solution of 3,3-dimethoxy-cyclobutanecarboxylic acid N-methoxy-N-methyl-amide (0.25g, 1.23mmol) in anhydrous THF (10mL) was cooled to -78°C. Propynylmagnesium bromide (0.5M in THF, 4.92mL, 2.46mmol) was slowly added. Once the addition was complete, the reaction was allowed to warm to 25°C and stir overnight. The reaction was then poured into 1 N aq. HCl and extracted three times with EtOAc. The organic extracts were combined, dried over MgSO₄, filtered and concentrated to afford 1-(3,3-dimethoxy-cyclobutyl)-but-2-yn-1-one as an amorphous yellow residue (0.25g) which was used in the next reaction without purification.

Step 2: A suspension of the product of Step 1 (0.25g, 1.23mmol) in H₂O (0.5 mL)was cooled to 0°C. Hydroxylamine-O-sulfonic acid (0.155g, 1.23mmol) was added, and reaction stirred at 0°C for 30 minutes. Solid NaHCO₃ (0.104g, 1.23mmol) was added, followed by sodium hydrogen sulfide (1.4M in H₂O, 1.0mL, 1.35mmol). The reaction was allowed to warm to 25°C and stir overnight. The reaction was then diluted with H₂O and extracted three times with CHCl₃. The organic layers were combined, dried over MgSO₄, filtered and concentrated. The crude material was purified by chromatography (gradient elution of 10% to 50% EtOAc in heptane) to afford the title compound (0.01 g). ¹H NMR (400 MHz, CDCl₃) 2.55 (d, 3H), 3.37-3.51 (m, 4H), 3.69-3.78 (m, 1H), 6.83-6.85 (m, 1H).

### Preparation 6: 3-(3-methyl-1,2,4-oxadiazol-5-yl)cyclobutanone

Step 1: A solution of NaOH (228mg, 5.7mmol) in H₂O (5.7ml) was added to methyl 3,3-dimethoxycyclobutanecarboxylate (1g, 5.7mmol) in MeOH (11.4ml) and stirred for 30 minutes. The reaction mixture was subsequently diluted with diethyl ether (30ml), then neutralized and washed with 10% aqueous citric acid solution (1 x 20ml). The organic phase was separated, dried over sodium sulfate, filtered and concentrated to furnish 886mg of a colorless oil.

Step 2: To a solution of the product of Step 1 (320mg, 2mmol) in THF (10ml) was added N,N-diisopropylethylamine (0.34ml, 2mmol) and TFFH (528mg, 2mmol) followed by (Z)-*N*'-hydroxyacetamidine (148mg, 2mmol). A slight exotherm was noted and the reaction mixture was allowed to stir at 25°C overnight under N₂. The reaction mixture was then diluted with EtOAc (20ml) and washed with H₂O (1 x 10ml). The organic phase was dried over Na₂SO₄, filtered and concentrated to furnish 900mg of crude material. This material was taken up in EtOAc (30ml) and washed with 0.5N HCl (15ml) followed by sat. aq. NaHCO₃ (15ml). The organic phase was dried over Na₂SO₄, filtered and concentrated to furnish 150 mg of impure product. A further 100 mg of product was recovered from re-extracting the aqueous phase with DCM.

Step 3: To a solution of the product of Step 2 (250mg, 1.15mmol) in THF (11.5ml, 0.1M) was added tetrabutylammonium fluoride (1.15ml of a 1 M solution in THF) and heated to reflux for 2 hours. The reaction mixture was then diluted with EtOAc (30 ml) and washed with sat. aq. NaHCO₃ (1 x 25ml). The organic phase was dried over Na₂SO₄, filtered, passed through a pad of silica gel and concentrated. The resulting material was passed through a second pad of silica gel eluting with 1:1 EtOAc:heptanes to furnish 64mg of an oil.

Step 4: The product of Step 3 (44mg, 0.22mmol) was dissolved in acetone (0.9ml, 0.25M) and treated with catalytic iodine (6mg, 0.02mmol). The reaction mixture stirred at 25°C for 2hrs before being diluted with EtOAc (5ml) and washed with sat. aq. sodium thiosulfate solution (5ml). The organic phase was separated, dried over sodium sulfate, filtered and concentrated to yield the title compound (38mg) as an oil. ¹H NMR (400 MHz, CDCl₃) *δ* ppm 2.42 (s, 3 H), 3.60 (d, 4 H), 3.82 - 3.91 (m, 1 H), 4.22 (t, 1 H).

### Preparation 7: 3-hydroxy-3-phenylcyclobutanone

Step 1: A solution of 3-(benzyloxy)cyclobutanone (0.5g, 2.84mmol) in THF, (14ml, 0.2M) was cooled to -78°C under N₂. To this was added phenyl magnesium Grignard (1.36ml, 3.12mmol) drop-wise via syringe. The reaction mixture was allowed to warm to 25°C over 2 h before being quenched with H₂O (5-10 ml) and extracted with EtOAc (40ml) to furnish 463mg of product. The aqueous phase was then diluted with brine and re-extracted with EtOAc (40ml) to yield a further 170mg of product.

Step 2: Palladium black (38mg, 0.36mmol) was added to the product of Step 1 (460mg, 1.81mmol) in a 4.4% solution of formic acid in MeOH (36ml, 0.05M). The resulting mixture was allowed to stir at 25°C overnight. An additional portion of palladium black (140mg) was added and the reaction was allowed to stir for 4 days. The reaction was subsequently filtered and the catalyst washed with MeOH (20-30ml). Concentration of the filtrate and washings furnished 260mg of an off white solid.

Step 3: To a solution of the product of Step 2 (100mg, 0.61mmol) in DCM (6ml, 0.1M) was added PS-IBX (610mg, 1.2mmol/g, 0.73 mmol). The resulting mixture was allowed to stir at 25°C under N₂. After 2 hours, an additional portion of PS-IBX (610mg, 1.2mmol/g, 0.73 mmol) was added and the reaction was allowed to stir for another 2 hours. The reaction was then filtered and the resin washed with DCM. The filtrate and washings were concentrated to provide the title compound (88mg) which was used without further purification. ¹H NMR (400 MHz, CDCl₃) *δ* (ppm) 3.39 - 3.55 (m, 2 H), 3.55 - 3.69 (m, 2 H), 7.34 - 7.49 (m, 5 H), 7.53 (d, 1 H).

### Preparation 8: 3-(5-methyl- 1,3,4-oxadiazol-2-yl)cyclobutanone

Step 1: To a solution of methyl 3,3-dimethoxycyclobutanecarboylate (1g, 5.75mmol) in MeOH (11.5ml, 0.5M) was added hydrazine (0.36ml, 11.49mmol) and the resulting mixture heated to 65°C overnight under N₂. The reaction was then concentrated to furnish 1.0 g of a white solid.

Step 2: The product of Step 1 (228mg, 1.3mmol) was suspended in trimethylorthoacetate (0.84ml, 6.5ml) and heated to reflux under N₂ for 3 days. The reaction was then concentrated to furnish the product (229mg) as an oil.

Step 3: Iodine (8mg, 0.03mmol), was added to a solution of the product of Step 2 (60mg, 0.3mmol) in acetone (1.2ml) and the mixture allowed to stir for 1 hour at 25°C. The reaction mixture was then diluted with EtOAc (15ml) and washed with sat. aq. sodium thiosulfate solution (15ml). The organics were separated, dried over sodium sulfate, filtered and concentrated to furnish the title compound (56 mg), which was used without further purification.. ¹H NMR (400 MHz, CDCl₃) *δ* (ppm) 2.47 - 2.55 (m, 3 H), 3.19 (d, 3 H), 3.54 - 3.61 (m, 2 H).

### Preparation 9: 3-(3-methylisoxazol-5-yl)cyclobutanone

Step 1: To a solution of methyl 3,3-dimethoxycyclobutanecarboxylate (1.74 g, 10 mmol) in 2:1 MeOH:Water (30 mL) was added solid NaOH (2.5 g, 62 mmol) and the mixture stirred at 25°C overnight. The mixture was poured into 1:1 ether:citric acid in water (100 mL) and the layers separated. Organic phase was washed with water and brine, then was dried, filtered, and concentrated to give 1,0 g of a colorless solid.

Step 2: The product of Step 1 (1.0 g, 6.24 mmol), N,O-Dimethylhydroxylamine hydrochloride (0.91 g, 9.37 mmol), N-Hydroxybenzotriazole (1.27 g, 9.37 mmol) and 1-ethyl-3-(3-dimethylaminopropyl)carbodiimide HCl (1.8 g, 9.37 mmol), were combined in anhydrous DMF (12 mL). Diisopropylethylamine (2.42 g, 18.2 mmol) was added and the mixture stirred at 25°C overnight under an atmosphere of nitrogen. The resulting solution was diluted with water and extracted with 1:1 diethyl ether:ethyl acetate. The organic phase washed with water (3X), 10% citric acid in water, water, 1.0 N NaOH in water, brine and dried. The solvent was removed under reduced pressure to give 0.5 g of a coloriess oil which was used in the next step without further purification.

Step 3: To a solution of acetone oxime (0.216 g, 2.95 mmol) in THF (6 mL) at 0°C, was added nBuLi (2.36 mL of 2.5 M solution in Hexanes, 5.9 mmol) and the mixture was stirred for 30 minutes. The product of Step 2 (0.5 g, 2.95 mmol) was added as a solution in THF (2 mL) and the reaction stirred at 0°C for 1.5 h. The resulting mixture was poured into 10 mL of 4:1 THF:H₂0 containing 0.5 mL concentrated H₂SO₄ and heated at 65°C for 1 h whereupon the mixture was diluted with ice-cold water, neutralized with solid NaHCO₃, and extracted with ether (2X). The organic phase was washed with water, brine, then dried, filtered and concentrated to give the title compound 0.17 g as a pale yellow gum which was used without further purification. ¹H NMR (400 MHZ, CDCl₃) δ (ppm) 5.93 (s, 1 H), 3.68 (m, 1 H), 3.2-3.5 (m, 4H), 2.3 (s, 3H).

### Preparation 10: methyl 4-oxo-1-((trans)-3-phenylcyclobutyl)piperidine-2-carboxylate

To a solution of methyl 4-oxopiperidine-2-carboxylate (1eq) in THF (0.1M) and MeOH (0.4M) was added 3-cyclobutanone (1eq), 4A molecular sieves and AcOH (1.5eq). The reaction mixture stirred at 25°C for 1 h before the addition of MP-cyanoborohydride resin (1.2eq). The reaction mixture was then stirred at 25°C overnight, whereupon it was diluted with DCM (double volume), filtered and the resin washed with additional DCM, (5-10ml). The organics were washed with sat. aq. NaHCO₃ (equal volume) and dried over Na₂SO₄, filtered, and concentrated to dryness. The material thus obtained was purified via column chromatography, (Combiflash, gradient elution with 0-100% EtOAc in heptanes over 40 minutes) to yield the title compound (347mg) as an oil. ¹H NMR (400 MHz, CDCl₃) *δ* (ppm) 2.03 (q, 2 H), 2.44 (t, 2 H), 2.53 - 2.63 (m, 5 H), 2.83 - 2.95 (m, 2 H), 3.09 (s, 2 H), 3.12 - 3.23 (m, 1 H), 3.77 (s, 3 H), 7.17 - 7.26 (m, 2 H); MS ESI+ m/z (M+H)⁺ 288.2.

### Preparation 11: (3R,4R)-4-[3-Hydroxy-3-(6-methoxy-quinolin-4-yl)-propyl]-piperidine-3-carboxylic acid

Step 1: To a cooled (0 °C) solution of (3R,4R)-1-tert-butyl 3-methyl 4-(3-(6-methoxyquinolin-4-yl)-3-oxopropyl)piperidine-1,3-dicarboxylate (207g, 453.4 mmol) in MeOH (3.2L) (J. Org. Chem. 2006, 71, 9045-9050) was added NaBH₄ (18.9g, 498.8 mmol) portion-wise. After the addition was complete, the reaction was allowed to warm to 25°C and stir 90 minutes. The reaction was then concentrated under reduced pressure and the residue partitioned between ether and sat. aq, NH₄Cl solution. The layers were separated and the aqueous layer extracted with ether. The organic phases were then combined, washed with brine, dried over MgSO₄, and concentrated to dryness to afford a bright yellow solid (192.2g) as a mixture of diastereomers. The diastereomers (Diastereomer A and Diastereomer B) were separated via chiral chromatography using a ChiralPak AD (10 cm x 50 cm) column, eluting with 85:15 heptane:EtOH at a flow rate of 250 mL/min.

The isolated diastereomers were analyzed using a ChiralPakAD-H 5 µm column (mobile phase 80:20:0.2 heptane:EtOH:DEA, flow rate 1.5 mL/min). Under these conditions, Diastereomer A had a retention time of 6.996 min and Diastereomer B had a retention time of 7.672 min.

Diastereomers A and B as described above were individually derivatized with a chiral acid chloride to form the corresponding esters. Analysis of ¹H and ¹⁹F NMR spectra according to the method of Mosher (see, for example: J. Am Chem. Soc. 1973, 95, 512 and J. Org. Chem. 1973, 38, 2143) was used to assign the stereochemical configuration of the benzylic stereocenter bearing the hydroxyl group. This analysis supports the assignment of diastereomer A having the R configuration and diastereomer B having the S configuration. It is appreciated by one skilled in the art that this technique may also be used to ascertain the stereochemical configuration of other compounds and chemical intermediates in this invention.

Diastereomer B was further purified by chromatography (gradient elution from 20% to 50% EtOAc in heptane followed by 100% EtOAc) to provide the product (47.32 g), as a single diastereomer as a yellow solid. ¹H NMR (400MHz, CDCl₃): *δ* (ppm) 8.55(br d, 1 H); 7.91 (d, 1 H); 7.42(d, 1 H); 7.26(dd, 1 H); 7.11 (d, 1 H); 5.21 (dd, 1 H); 3.92-3.82(m, 4H); 3.73-3.62(m, 1 H); 3.52(s, 3H); 3.14(dd, 1H); 3.03-2.83(m, 1H); 2.51(s, 1H); 1.91-1.59(m, 6H); 1.44-1.32(m, 10H).

Step 2: Diastereomer B (3R,4R)-4-[3-(S)-Hydroxy-3-(6-methoxy-quinolin-4-yl)-propyl]-piperidine-1,3-dicarboxylic acid 1-tert-butyl ester 3-methyl ester (25.0g, 54.5mmol) and hydrochloric acid (6M aqueous solution, 1.663L, 9977mmol) were combined and heated at 75°C overnight. The reaction mixture was cooled to 25°C, and concentrated to near dryness. The residual material was dissolved in a minimal volume of water, the pH adjusted to approximately pH 7 by the addition of 6N aq. NaOH and re-concentrated. The material thus obtained was triturated with 1 L of 9:1 DCM/MeOH and filtered through a pad of Celite. The filtrate was concentrated to dryness and dried under vacuum to afford the title compound as a tan solid (19.69g). Elemental analysis of the solid indicated a potential mixture of salt forms: Found C 61.54, H 7.46, N 7.35, Cl 3.76, Na 0.30; LCMS M+ 1 = 345; ret. time (polar) = 0.35min ; ¹H NMR (400MHz, DMSO-d6): *δ* (ppm) 8.66(d, 1 H); 7.88(d, 1H); 7.52(d, 1 H); 7,36-7.31 (m, 2H); 5.23-5.17(m, 1H); 3.90(s, 3H); 3.18-3.05(m, 3H); 2.78-2.68(m, 2H); 2.08-1.95(m, 1 H); 1.76-1.65(m, 1 H); 1.63-1.43(m, 5H).

### Preparation 12: 3-fluoro-6-methoxyquinoline

Step 1: To a solution of dimethyl 2-fluoromalonate (2945 g, 19.62 mol) in methanol (40 L) at 25°C was added LiOH·H₂O (1893 g, 45.12 mol) in one portion. Following the addition, the reaction temperature rose to about 40-45 °C. The reaction mixture was then heated to about 40°C for approximately 16 h, after which the reaction was filtered to collect solids. The filtrate was concentrated to dryness to yield a solid residue. All solids were combined and dried in a vacuum oven at about 30-35°C to remove all traces of methanol before proceeding. The dried solids were dissolved in water (4.5L) and mixed with MTBE (22L). The mixture was cooled with the addition of ice and acidified to pH 1 with 12M HCl (3.5L), adding additional ice as needed to keep the reaction temperature below about 20°C. The layers were separated and the aqueous layer extracted with MTBE (4 x 4L). The organic extracts were then combined, dried with MgSO₄, fiitered and concentrated to afford an oily solid product which was transferred to a drying tray and dried in a vacuum oven at 30-35°C overnight. After drying, the product (1894 g) is a white powder.

Step 2: The product of Step 1 (1109 g, 9.09 mol) was combined with POCl₃ (7.0 L) and heated to about 85°C to dissolve all of the solids. Once the solids were dissolved, the reaction was cooled to 60°C in a water bath before p-anisidine (1119 g, 9.09 mol) was added portion-wise over 1 hour. With each addition of p-anisidine a short duration of rapid gas production and a small exotherm was noted. Once the addition is complete, the reaction is slowly heated and refluxed (about 100°C -105°C) for 2 hours. Very vigorous gas production occurs as the reaction temperature reaches 80°C and above. Reaction progress is monitored by quenching an aliquot with ice and basifying to about pH 9 with NH₄OH, adding additional ice as necessary to control the high exotherm. The resulting solids were filtered and analyzed by TLC (7:3 Hexane/EtOAc). Once the p-anisidine has been consumed, the excess POCl₃ is removed from the reaction via vacuum distillation. The reaction mixture is then cooled to 25°C before being poured into ice (35.0 Kg) with vigorous stirring. The resulting slurry is stirred for 30-40 minutes, adding additional ice as necessary to maintain the reaction temperature below 20°C. NH₄OH (∼30L) is then slowly added until the solution is at pH 9.5, adding more ice as necessary to maintain a temperature <20°C (about 45Kg of ice is needed). The mixture is then stirred for an additional 2 hours before being filtered. The collected solids are triturated with warm water (5L), collected by filtration and washed with additional water (1L). The wet solids (3.2Kg) were added to EtOAc (9L) and heated to dissolve. The solution was transferred to a 40L separatory funnel and the water layer (1.4L) separated. Decolorizing charcoal was added to the still warm organic layer and the mixture was filtered. The filtrate was concentrated to a volume of 3L to provide a slurry which was cooled to -20°C in a freezer. The resulting solids were collected via filtration, washed with cold EtOAc (2 x 250mL) and MTBE (2 x 250mL), and dried in a vacuum oven at 35-40°C to provide a light brown powder (808 g). ¹H NMR (400 mHz, CDCl₃) *δ* (ppm) 7.91 (d, J = 9.1 Hz, 1H), 7.38 (dd, J = 9.1, 2.9 Hz, 1H), 7.32 (d, J = 2.9 Hz, 1H), 3.98 (s, 3H). MS m/z 246.1 (M+H).

Step 3: To the product of Step 2 (808 g, 3.28 mol) in methanol (7L) and NH₃/MeOH (7L) was added a first portion of Raney Nickel (150 g) and the resulting mixture hydrogenated at 150 psi. A second portion of Raney Nickel (150 g) was added after 18 hours, a third portion of Raney Nickel (100 g) was added after 42 hours and a final portion (50 g) was added after 50 hours. Reaction progress was monitored by TLC (7:3 Hexane/EtOAc). After approximately 65 hours, dicolite (200 g) was added and the mixture filtered through 2 GF pads and a bed of dicolite, washing with additional methanol. The filtrate was concentrated to dryness to provide a dark oil which was warmed with MTBE (600 mL) until dissolved. Decolorizing charcoal was then added and the mixture hot filtered. The filtrate was slowly cooled with agitation to precipitate the product. The resulting thick mixture was cooled to -20°C before the resulting solids were collected by filtration. The solids were washed with cold (<-20°C) MTBE (2 x 100mL) and dried in a vacuum oven at 30-35°C to afford the title compound (489 g) as a tan solid (fine needles). ¹H NMR (400 mHz, CDCl₃) δ (ppm) 8.64 (d, J = 2.9 Hz, 1H), 7.98 (d, J = 9.1 Hz, 1H), 7.65 (dd, *J*=9.1, 2.9 Hz, 1H), 7.31 (dd, J = 9.1, 2.9 Hz, 1H), 7.01 (d, J = 2.9 Hz, 1 H), 3.92 (s, 3H), MS *m*/*z* 178.19 (M+H).

### Example 1: (3R,4R)-4-[3-Hydroxy-3-(6-methoxy-quinolin-4-yl)-propyl-1-(3-phenyl-cyclobutyl)-piperidine-3-carboxylic acid

Step 1: Methyl (3R,4R)-1-tert-butyl-4-(3-hydroxy-3-(6-methoxyquinolin-4-yl)propyl)piperidine-1,3-dicarboxylate (17.44g, 38.03 mmol) (prepared as in Step 1 of Preparation 11) was dissolved in HCl/dioxane (4M, 200 mL) and stirred 30 minutes at 25°C and then concentrated under reduced pressure. The resulting material was partitioned between 1N aq. NaOH and ether, and the aqueous layer extracted three times with ether. The combined organic layers were dried over MgSO4, filtered, and concentrated to afford methyl (3R,4R)-4-(3-hydroxy-3-(6-methoxyquinolin-4-yl)propyl)piperidine-3-carboxylic acid as a yellow solid (6.8g). Additional amounts of this material, as well as of the saponified product, could be isolated through additional extractive work-ups.

Step 2: Methyl (3R,4R)-4-(3-hydroxy-3-(6-methoxyquinolin-4-yl)propyl)piperidine-3-carboxylic acid (2.84g, 7.92 mmol) was dissolved in THF (25 mL), MeOH (25 mL) and H2O (12.5 mL), treated with LiOH (0.949 g, 39.6 mmol), and allowed to react overnight at 40°C. The reaction was then diluted with H2O and concentrated under reduced pressure to remove the organic solvents. After pH adjustment to about 3 and washing with ethyl acetate, the aqueous layer was concentrated and the residue azeotroped with benzene. The resulting mass was dried under vacuum and then purified via ion-exchange chromatography on an MCX column to afford (3R,4R)-4-[3-hydroxy-3-(6-methoxy-quinolin-4-yl)-propyl]-piperidine-3-carboxylic acid (0.930 g).

Step 3. (3R,4R)-4-[3-hydroxy-3-(6-methoxy-quinolin-4-yl)-propyl]-piperidine-3-carboxylic acid (0.825 g), 3-phenylcyclobutanone (0.700 g), AcOH (0.275 mL) and 4Å molecular sieves (about 25 mg) were combined in THF (20 mL) and MeOH (16 mL) and stirred for 2.5 h at 25°C. To this was added NaCNBH₃ (0.302 g) in one portion and the resulting mixture was allowed to stir at 25°C overnight. The reaction was diluted with H₂O, adjusted to pH 6-7 with 1 M aq. NaOH, and extracted with DCM. The organic extracts were combined, dried over MgSO₄, filtered, and concentrated. The crude product was purified by chromatography (gradient elution from 1 to 25% MeOH in CHCl₃) to afford the title compound as a yellow solid (0.6767 g). LCMS: 2.21 min, M+1 475 (polar).

The title compound of Example 1 (665mg) was subjected to chiral HPLC separation (Chiralcel OD-H column (3 cm x 25 cm), mobile phase 70:30 CO₂: MeOH, flow rate 65 mL/min) to afford diastereomers A, B and C based on their order of elution.

Example 2, Diastereomer A (219.7mg), a single enantiomer was the first relative eluting HPLC peak. ¹H NMR (400MHz, CDCl₃): *δ* (ppm) 8.68(d, 1H); 7.96(d, 1H); 7.57(d, 1H); 7.35-7.12(m, 7H); 5.37(br d, 1H); 3.95(s, 3H); 3.22-3.10(m, 2H); 3.04(d, 1H); 2.90(p, 1H); 2.74(s, 1H); 2.68-2.52(m, 2H); 2.32-2.22(m, 1H); 2.16-1.96(m, 4H); 1.74-1.46(m, 6H). In a separate synthesis, the method described in Step 3 of Example 1 was repeated except that (3R,4R)-4-[3-(S)-hydroxy-3-(6-methoxyquinolin-4-yl)propyl]piperidine-3-carboxylic acid (from Step 2 of Preparation 11 using diastereoromer B) was used instead of (3R,4R)-4-[3-hydroxy-3-(6-methoxyquinolin-4-yl)propyl]piperidine-3-carboxylic acid (from Step 2 of Example). The ¹H NMR spectrum of the resultant product corresponded to the ¹H NMR spectrum for the product of Example 2. The result indicates that diastereomer A of Example 2 (obtained by resolution of the product of Example 1) has an S configuration at the benzylic stereocenter bearing the hydroxyl group.

Example 3, Diastereomer B (226.9mg), a single enantiomer of unidentified absolute configuration, was the second relative eluting HPLC peak. ¹H NMR (400MHz, CDCl₃): δ (ppm) 8.69(d, 1 H); 7.98(d, 1 H); 7.54(d, 1H); 7.34-7.13(m, 7H); 5.27(dd, 1 H); 3.89(s, 3H); 3.26-3.02(m, 3H); 2.94(p, 1 H); 2.81(s, 1 H); 2.68-2.53(m, 2H); 2.18-1.45(m, 11H). In a separate synthesis, the method described in Step 3 of Example 1 was repeated except that (3R,4R)-4-[3-(R)-hydroxy-3-(6-methoxyquinolin-4-yl)propyl]piperidine-3-carboxylic acid (from Step 2 of Preparation 11 using diastereoromer A) was used instead of (3R,4R)-4-[3-hydroxy-3-(6-methoxyquinotin-4-yl)propyl]piperidine-3-carboxylic acid (from Step 2 of Example). The ¹H NMR spectrum of the resultant product corresponded to the ¹H NMR spectrum for the product of Example 3. The result indicates that diastereomer B of Example 3 (obtained by resolution of the product of Example 1) has an R configuration at the benzylic stereocenter bearing the hydroxyl group.

Example 4, Diastereomer C (65.3mg), a mixture of other diastereomers, was the last relative eluting peak.
Table 2 provides additional non-limiting compounds of general Formula I that were prepared in a manner analogous to that described in Example 1 using the appropriate starting materials. Unless otherwise noted, LCMS data was acquired using standard conditions.

**TABLE 2**

| Ex.# | NAME | Ret Time | MS (M+1) |
|---|---|---|---|
| 5 | ethyl (3R,4R)-4-[3-hydroxy-3-(6-methoxyquinolin-4-yl)propyl]-1-{[(*trans*)-2-phenylcyclopropyl] methyl}piperidine-3-carboxylate | 1.17 | 489 |
| 6 | (3R,4R)-4-[3-hydroxy-3-(6-methoxyquinolin-4-yl)propyl]-1-[3-(4-methylphenyl)cyclobutyl] piperidine-3-carboxylic acid | 1.56 | 489 |
| 7 | (3R,4R)-1-[3-(2-fluorophenyl)cyclobutyl]-4-[3-hydroxy-3-(6-methoxyquinolin-4-yl)propyl] piperidine-3-carboxylic acid | 1.39 | 493 |
| 8 | (3R,4R)-4-[3-hydroxy-3-(6-methoxyquinolin-4-yl)propyl]-1-(3-phenoxycyclobutyl)piperidine-3-carboxylic acid | 2.39 | 491* |
| 10.11^{†} | (3R,4R)-4-[3-methoxy-3-(6-methoxyquinolin-4-yl)propyl]-1-(3-phenylcyclobutyl)piperidine-3-carboxylic acid | 1.39 | 489.2 |
| 12, 13^{†} | (4R)-4-[3-hydroxy-3-(6-methoxyquinolin-4-yl)propyl]-3-methyl-1-(3-phenylcyclobutyl) piperidine-3-carboxylic acid | 2.2 2.1 | 489.3* |
| 14, 15, 16 ^{‡} | (3R,4R)-4-[3-(3-fluoro-6-methoxyquinolin-4-yl)-3-hydroxypropyl]-1-(3-phenylcyclobutyl)piperidine-3-carboxylic acid | 2.21 | 493.4 |
| 17, 18,19 ^{‡} | (3R,4R)-1-[3-(2,6-difluorophenyl)cyclobutyl]-4-[3-(3-fluoro-6-methoxyquinolin-4-yl)-3-hydroxypropyl] piperidine-3-carboxylic acid | 1.92 | 529.1 |
| 20 | (3R,4R)-1-[3-(benzyloxy)cyclobutyl]-4-[3-hydroxy-3-(6-methoxyquinolin-4-yl)propyl]piperidine-3-carboxylic acid | 1.3 | 505.7 |
| 21 | (3R,4R)-4-[3-hydroxy-3-(6-methoxyquinolin-4-yl)propyl]-1-(3-pyridin-2-ylcyclobutyl)piperidine-3-carboxylic acid | 0.7 | 476.4 |
| 22 | (3R,4R)-4-[3-hydroxy-3-(6-methoxyquinolin-4-yl)propyl]-1-[3-(5-methylisothiazol-3-yl)cyclobutyl] piperidine-3-carboxylic acid | 2.26 | 496* |
| 23 | 4-[3-(3-fluoro-6-methoxyquinolin-4-yl)propyl]-1-(3-phenylcyclobutyl)piperidine-4-carboxylic acid | 1.8 | 477.1 |
| 24 | 4-[3-(3-fluoro-6-methoxyquinolin-4-yl)-3-hydroxypropyl]-1-(3-phenylcyclobutyl)piperidine-4-carboxylic acid | 3.17 | 493* |
| 25^{b} | 4-[3-hydroxy-3-(6-methoxyquinolin-4-yl)propyl]-1-{[(*trans*)-2-phenylcyclopropyl]methyl}piperidine-3-carboxylic acid | 1.17 | 475 |
| 26^{b} | 1-{[(*trans*)-2-(2,5-difluorophenyl)cyclopropyl] methyl}-4-[3-hydroxy-3-(6-methoxyquinolin-4-yl)propyl] piperidine-3-carboxylic acid | 1.26 | 511 |
| 27^{b‡} | (3R,4R)-1-[3-(2,6-difluorophenyl)cyclobutyl]-4-[3-hydroxy-3-(6-methoxyquinolin-4-yl)propyl] piperidine-3-carboxylic acid | 2.08 | 511 |
| 28 ^{a,c} | (3R,4R)-1-[3-(4-chlorophenyl)cyclobutyl]-4-[3-hydroxy-3-(6-methoxyquinolin-4-yl)propyl] piperidine -3-carboxylic acid | 1.4-1.5 | 509.1 |
| 29 ^{a,d} | (3R,4R)-1-[3-(3-chlorophenyl)cyclobutyl]-4-[3-hydroxy-3-(6-methoxyquinolin-4-yl)propyl] piperidine-3-carboxylic acid | 1.6 | 509.1 |
| 30 ^{a,d} | (3R,4R)-4-[3-hydroxy-3-(6-methoxyquinolin-4-yl)propyl]-1-{3-[4-(trifluoromethyl)phenyl]cyclobutyl} piperidine-3-carboxylic acid | 1.7 | 543.1 |
| 31 ^{a,c} | (3R,4R)-4-[3-hydroxy-3-(6-methoxyquinolin-4-yl)propyl]-1-[3-(4-hydroxyphenyl)cyclobutyl] piperidine-3-carboxylic acid | 1.0 | 491.3 |
| 32 ^{a,d} | (3R,4R)-1-[3-(3,4-dimethoxyphenyl)cyclobutyl]-4-[3-hydroxy-3-(6-methoxyquinolin-4-yl)propyl] piperidine -3-carboxylic acid | 1.1 | 535.3 |
| 33 ^{a,d} | (3R,4R)-1-[3-(4-hydroxy-3-methoxyphenyl) cyclobutyl]-4-[3-hydroxy-3-(6-methoxyquinolin-4-yl)propyl]piperidine-3-carboxylic acid | 1.2 | 521.3 |
| 34 ^{a,c} | (3R,4R)-4-[3-hydroxy-3-(6-methoxyquinolin-4-yl)propyl]-1-[3-(2-methylphenyl)cyclobutyl] piperidine -3-carboxylic acid | 1.2-1.3 | 489.2 |
| 35^{c} | (3R,4R)-1-[3-(2,6-dimethylphenyl)cyclobutyl]-4-[3-hydroxy-3-(6-methoxyquinolin-4-yl)propyl] piperidine-3-carboxylic acid | 1.4-1.5 | 503.4 |
| 36^{c} | (3R,4R)-1-[3-(2,3-difluorophenyl)cyclobutyl]-4-[3-hydroxy-3-(6-methoxyquinolin-4-yl)propyl] piperidine -3-carboxylic acid | 1.4 | 511.4 |
| 37^{b,c} | (3R,4R)-1-[3-(2,4-difluorophenyl)cyclobutyl]-4-[(3S)-3-hydroxy-3-(6-methoxyquinolin-4-yl)propyl] piperidine-3-carboxylic acid | 1.4 | 511.4 |
| 38^{a,c} | (3R,4R)-1-[3-(2,5-difluorophenyl)cyclobutyl]-4-[3-hydroxy-3-(6-methoxyquinolin-4-yl)propyl] piperidine-3-carboxylic acid | 1.3-1.4 | 511.3 |
| 39^{c} | (3R,4R)-1-[3-(2,5-difluorophenyl)cyclobutyl]-4-[3-(3-fluoro-6-methoxyquinolin-4-yl)-3-hydroxypropyl] piperidine-3-carboxylic acid | 1.8 | 529.2 |
| 40^{b,c} | (3R,4R)-1-[3-(2,5-difluorophenyl)cyclobutyl]-4-[(3S)-3-hydroxy-3-(6-methoxyquinolin-4-yl)propyl] piperidine-3-carboxylic acid | 1.3 | 511.3 |
| 41^{b,c} | (3R,4R)-1-(3-biphenyl-4-ylcyclobutyl)-4-[(3S)-3-hydroxy-3-(6-methoxyquinolin-4-yl)propyl] piperidine-3-carboxylic acid | 2.0 | 551.4 |
| 42^{a,c} | (3R,4R)-4-[3-hydroxy-3-(6-methoxyquinolin-4-yl)propyl]-1-(3-methyl-3-phenylcyclobutyl)piperidine -3-carboxylic acid | 1.5 | 489.3 |
| 43^{c} | (3R,4R)-4-[3-hydroxy-3-(6-methoxyquinolin-4-yl)propyl]-1-(2-methyl-3-phenylcyclobutyl)piperidine -3-carboxylic acid | 1.3-1.4 | 489.3 |
| 44^{a,c} | (3R,4R)-1-(3-cyclohexylcyclobutyl)-4-[3-hydroxy-3-(6-methoxyquinolin-4-yl)propyl]piperidine-3-carboxylic acid | 1.4-1.5 | 481.2 |
| 45,46,47, 480^{a,c,‡} | (3R,4R)-1-(3-cyclopentylcyclobutyl)-4-[3-hydroxy-3-(6-methoxyquinolin-4-yl)propyl]piperidine-3-carboxylic acid | 1.5 | 467.3 |
| 49^{a,c} | (3R,4R)-1-[3-cyclopentylmethyl)cyclobutyl]-4-[3-hydroxy-3-(6-methoxyquinolin-4-yl)propyl] piperidine-3-carboxylic acid | 1.7 | 481.3 |
| 50^{c} | (3R,4R)-4-[3-hydroxy-3-(6-methoxyquinolin-4-yl)propyl]-1-[3-(tetrahydro-2H-pyran-4-yl)cyclobutyl] piperidine-3-carboxylic acid | .9 | 483.4 |
| 51^{c} | (3R,4R)-4-[3-hydroxy-3-(6-methoxyquinolin-4-yl)propyl]-1-[3-(tetrahydrofuran-3-ylmethyl) cyclobutyl]piperidine-3-carboxylic acid | .9 | 483.4 |
| 52^{c} | (3R,4R)-4-[3-hydroxy-3-(6-methoxyquinolin-4-yl)propyl]-1-[3-(tetrahydrofuran-2-ylmethyl) cyclobutyl]piperidine-3-carboxylic acid | .9 | 483.4 |
| 53^{c} | (3R,4R)-1-[(1,7)-bicyclo[5.2.0]non-8-yl]-4-[3-hydroxy-3-(6-methoxyquinolin-4-yl)propyl] piperidine-3-carboxylic acid | 1.3-1.4 | 467.4 |
| 54^{c} | (3R,4R)-1-[(1,6)-bicyclo[4.2.0]oct-7-yl]-4-[3-hydroxy-3-(6-methoxyquinolin-4-yl)propyl] piperidine-3-carboxylic acid | 1.1 | 453.4 |
| 55^{a,c} | (3R,4R)-4-[3-hydroxy-3-(6-methoxyquinolin-4-yl)propyl]-1-spiro[3.4]oct-2-ylpiperidine-3-carboxylic acid | 1.1 | 453.3 |
| 56^{c} | (3R,4R)-4-[3-hydroxy-3-(6-methoxyquinolin-4-yl)propyl]-1-spiro[3.5]non-2-ylpiperidine-3-carboxylic acid | 1.4 | 467.4 |
| 57^{b,c} | (3R,4R)-1-[3-(3-fluoro-2-methylphenyl)cyclobutyl]-4-[(3S)-3-hydroxy-3-(6-methoxyquinolin-4-yl)propyl] piperidine-3-carboxylic acid | 1.5 | 507.6 |
| 58^{c} | (3R,4R)-1-[3-(2,3-difluorophenyl)cyclobutyl]-4-[3-hydroxy-3-(6-methoxyquinolin-4-yl)propyl] piperidine-3-carboxylic acid | 1.4 | 511.4 |
| 59, 60^{c±} | (3R,4R)-1-[3-(2,5-difluorophenyl)cyclobutyl]-4-[3-(3-fluoro-6-methoxyquinolin-4-yl)-3-hydroxypropyl] piperidine-3-carboxylic acid | 1.8 | 529.2 |
| 61^{b,c} | (3R,4R)-1-[3-(2-fluoro-5-methylphenyl)cyclobutyl]-4-[(3S)-3-hydroxy-3-(6-methoxyquinolin-4-yl)propyl] piperidine-3-carboxylic acid | 1.4 | 507.2 |
| 62^{b,c} | (3R,4R)-1-{3-[2-fluoro-5-(trifluoromethyl)phenyl] cyclobutyl}-4-[(3S)-3-hydroxy-3-(6-methoxyquinolin -4-yl)propyl]piperidine-3-carboxylic acid | 1.7 | 561.2 |
| 63^{b,c} | (3R,4R)-1-[3-(2-chloro-6-fluorophenyl)cyclobutyl]-4-[(3S)-3-hydroxy-3-(6-methoxyquinolin-4-yl)propyl] piperidine-3-carboxylic acid | 1.5 | 527.2 |
| 64^{b,c} | (3R,4R)-1-[3-(3-chloro-2-fluorophenyl)cyclobutyl]-4-[(3S)-3-hydroxy-3-(6-methoxyquinolin-4-yl)propyl]piperidine-3-carboxylic acid | 1.4 | 527.2 |
| 64^{b,c} | (3R,4R)-1-[3-(3-chloro-2-fluorophenyl)cyclobutyl]-4-[(3S)-3-hydroxy-3-(6-methoxyquinolin-4-yl)propyl] piperidine-3-carboxylic acid | 1.4 | 527.2 |
| 65^{b,c} | (3R,4R)-1-[3-(4-chloro-2-fluorophenyl)cyclobutyl)-4-[(3S)-3-hydroxy-3-(6-methoxyquinolin-4-yl)propyl] piperidine-3-carboxylic acid | 1.7 | 527.2 |
| 66 ^{b,c} | (3R,4R)-1-[(2,3)-3-(2,6-difluorophenyl)-2-methyl cyclobutyl]-4-[(3S)-3-hydroxy-3-(6-methoxyquinolin -4-yl)propyl]piperidine-3-carboxylic acid | 1.4 | 525.3 |
| 67^{b,c} | (3R,4R)-1-[(2,3)-3-(2,6-difluorophenyl)-2-methyl cyclobutyl]-4-[(3S)-3-hydroxy-3-(6-methoxyquinolin -4-yl)propyl]piperidine-3-carboxylic acid | 1.4 | 525.3 |
| 68 ^{b,c} | (3R,4R)-1-[3-(3-chloro-2,6-difluorophenyl) cyclobutyl]-4-[(3S)-3-hydroxy-3-(6-methoxyquinolin -4-yl)propyl]piperidine-3-carboxylic acid | 1.4 | 545.2 |
| 69 ^{b,c} | (3R,4R)-4-[(3S)-3-hydroxy-3-(6-methoxyquinolin-4-yl)propyl]-1-[3-(2,4,6-trifluorophenyl)cyclobutyl] piperidine-3-carboxylic acid | 1.4 | 529.3 |
| 70 ^{b,c} | (3R,4R)-1-(3-biphenyl-4-ylcyclobutyl)-4-[(3S)-3-hydroxy-3-(6-methoxyquinolin-4-yl)propyl] piperidine-3-carboxylic acid | 2.0 | 551.4 |
| 71^{e} | methyl (3R,4R)-4-[3-hydroxy-3-(6-methoxyquinolin-4-yl)propyl]-1-{[(*trans*)-2-phenylcyclopropyl]methyl} piperidine-3-carboxylate | 1.56 | 489 |
| 72^{b} | methyl (3R,4R)-4-[3-hydroxy-3-(6-methoxyquinolin-4-yl)propyl]-1-{[(*trans*)-2-phenylcyclopropyl]methyl} piperidine-3-carboxylate | 1.61 | 489 |

| | | | |
|---|---|---|---|
| ^{a} Starting amine was a dichloride salt, 1.1 mmol of N,N isopropylethylamine was substituted for AcOH. ^{b} Prepared using the product of Step 2 of Preparation 11 (using diastereomer B). ^{c} Crude product mixture was purified via prep HPLC, gradient elution of 5-60% ACN: H₂O with 0.1% formic acid, over a range of between 9-11 minutes (Xterra 30 x 50 C18 column). ^{d} Crude product mixture was purified via prep HPLC, gradient elution ranging from between 0-55% of 0.1 % formic acid in ACN: H₂O wit 0.1 % formic acid. ^{e} Prepared using Diastereomer A of Preparation 11. * LC method (polar conditions) † Separation of diastereomers (benzylic alcohol center) via chromatography using a CHCl₃/MeOH elutent system. ‡ Diastereomers separated via chiral chromatography using the conditions described below. ± Partial separation of diastereomers (benzylic alcohol center) via MP chromatography eluting with 5:4:1 EtOAc/CHCl₃/MeOH. | | | |

Example 14 was subjected to chiral chromatography using the following conditions: Chiralpak AD (10cm X 50cm) with a mobile phase Heptane:EtOH (70/30) with 0.2% DEA and a flow rate of 500ml/min to afford the following separated diastereomers (Examples 15 and 16) as DEA salts. Each diasteromer was then worked up separately as follows. The salt was dissolved in DCM and extracted with 0.1 N HCl solution 4X. The aqueous layer was then neutralized to pH 6-7 with the addition of 1 N NaOH solution and was then extracted 3X with DCM. The organic layers were then combined, dried over MgSO₄, filtered, and concentrated to provide yellow foamy solids.

Example 15: (Diastereomer 1, 0.6212 g) ¹H NMR (CDCl₃, 400MHz) *δ* (ppm) 8.54 (d, 1 H), 7.94 (d, 1 H), 7.71 (d, 1 H), 7.15-7.31 (m, 6 H), 5.51 (q, 1 H), 3.95 (s, 3 H), 3.12-3.23 (m, 2 H), 3.07 (d, 1 H), 2.88-2.96 (m, 1 H), 2.75 (s, 1 H), 2.54-2.68 (m, 2 H), 1.97-2.16 (m, 6 H), 1.56-1.80 (m, 5 H).

Example 16: (Diastereomer 2, 0.6144 g) ¹H NMR (CDCl₃, 400MHz) *δ* (ppm) 8.54 (s, 1 H), 7.95 (d, 1 H), 7.83 (d, 1 H), 7.16-7.32 (m, 6 H), 5.48 (q, 1 H), 3.95 (s, 3 H), 3.10-3.26 (m, 2 H), 3.06 (d, 1 H), 2.87-2.97 (m, 1 H), 2.83 (s, 1 H), 2.46-2.68 (m, 3 H), 1.98-2.16 (m, 4 H), 1.56-1.92 (m, 5 H), 1.34-1.46 (m, 1 H).

Example 17 was subjected to chiral chromatography using the following conditions: Chiralpak OD-H (10 cm x 250cm) with a 70/30 CO₂/EtOH mobile phase and a flow rate of: 10.0 mL/min to afford the following separated diastereomers (Examples 18 and 19).

Example 18: (Diastereomer 1, 62.5 mg) had a retention time of 3.18 min. ¹H NMR (CDCl₃, 400MHz) *δ* (ppm) 8.48 (d, 1 H), 7.89 (d, 1 H), 7.72 (d, 1 H), 7.24 (dd, 1 H), 7.06-7.15 (m, 1 H), 6.75-6.84 (m, 2 H), 5.48 (q, 1 H), 3.92 (s, 3 H), 3.30-3.42 (m, 1 H), 3.16 (d, 1 H), 3.04 (d, 1 H), 2.86-2.96 (m, 1 H), 2.48-2.76 (m, 3 H), 2.28-2.39 (m, 2 H), 1.97-2.17 (m, 4 H), 1.50-1.80 (m, 5 H).

Example 19: (Diastereomer 2, 71.7 mg) had a retention time of 5.51 min. ¹H NMR (CDCl₃, 400MHz) *δ* (ppm) 8.53 (d, 1 H), 7.93 (d, 1 H), 7.83 (d, 1 H), 7.27 (dd, 1 H), 7.08-7.16 (m, 1 H), 6.76-6.84 (m, 2 H), 5.47 (q, 1 H), 3.94 (s, 3 H), 3.32-3.43 (m, 1 H), 3.20 (d, 1 H), 3.04 (d, 1 H), 2.86-2.96 (m, 1 H), 2.81 (s, 1 H), 2.59-2.70 (m, 2 H), 2.45-2.56 (m, 1 H), 2.35 (q, 2 H), 2.00-2.14 (m, 2 H), 1.81-1.91 (m, 1 H), 1.55-1.80 (m, 4 H), 1.33-1.43 (m, 1 H). Contaminated by a small amount of an apparent diastereomeric impurity.

Example 25 was prepared using the product of Step 2 of Preparation 11 (using diastereomer B) and (*trans*)-2-Phenyl-cyclopropanecarbaldehyde to afford a white solid (160mg). ¹H NMR (400MHz, CD₃OD): *δ* (ppm) 8.62(d, 1 H); 7.90(d, 1 H); 7.63(d, 1 H); 7.42(br s, 1 H); 7.38(dd, 1 H); 7.22(t, 2H); 7.15-7.07(m, 3H); 5.37(m, 1 H); 3.96(s, 3H); 3.73-3.61(m. 1 H); 3.48(br t, 1H); 3.19-3.10(m, 1 H); 3.09-2.99(m, 1 H); 2.97-2.84(m, 2H); 2.69(br s, 1H); 2.18(br t, 1 H); 2.06-1.94(m, 1 H); 1.93-1.61(m. 6H); 1.45-1.35(m, 1 H); 1.23-1.00(m. 2H).

Example 26 was prepared using the product of Step 2 of Preparation 11 (using diastereomer B) and (*trans*)-2-(2,5-Difluoro-phenyl)-cyclopropanecarbaldehyde to afford a white solid (270mg).¹H NMR (400MHz, CD₃OD): *δ* (ppm) 8.63(d, 1 H); 7.90 (d, 1 H); 7.63(d, 1 H); 7.42(br s, 1 H); 7.38(dd, 1 H); 7,08-6.99(m, 1 H); 6.94-6.86(m, 1 H); 6.84-6.77(m, 1 H); 5.37(m, 1 H); 3.96(s, 3H); 3.75-3.63(m, 1 H); 3.55-3.45(m, 1 H); 3.29-3.22(m, 1 H); 3.18-3.06(m, 1 H); 3.03-2.87(m, 2H), 2.70(s, 1 H); 2.24-2.12(m, 2H); 1.97-1.60(m, 6H); 1.59-1.45(m, 1 H); 1.25-1.07(m, 2H).

Example 27 was prepared using the product of Step 2 of Preparation 11 (using diastereomer B) and 3-(2,6-difluoro-phenyl)-cyclobutanone to afford a white solid (1,19g), as a ca. 9:1 *cis:trans* mixture of cyclobutyl diastereomers. A portion of this material (750 mg) was subjected to chiral chromatography using a Chiralcel OJ-H (10 cm x 250 cm) a 85 :15 (CO₂: MeOH) Mobile phase with a flow rate of 10 mL/min to afford the cis diastereomer as a white solid (418.8 mg) in 98% diastereomeric purity. ¹H NMR (500MHz, CDCl₃): *δ* (ppm) 8.63(d, 1 H); 7.94(d, 1H); 7.56(d, 1 H); 7.33-7.24(m, 2H); 7.10(p, 1 H); 6.78(p, 2H); 5.34(dd, 1 H); 3.93(s, 3H); 3.42-3.32(m, 1 H); 3.21(d, 1 H); 3.07(d, 1 H); 2.98(p, 1 H); 2.85-2.55(m, 3H); 2.39(p, 2H); 2.16(p, 3H); 1.75-1.52(m, 6H).

Example 37 is a > 95:5 mixture of alcohol diastereomers having an undetermined mixture of cis / trans isomers on the cyclobutane ring as a white solid foam (64.4 mg, 72%) after purification via preparatory HPLC; 5-50% CH₃CN: H₂O with 0.1% formic acid, 10 min on an Xterra 30 x 50 C18 column. The configuration at the alcohol stereocenter was assigned relative to known compounds. ¹H NMR (400 MHz, CD₃OD) *δ* (ppm) 1.77 - 1.94 (br. m, 6 H) 2.10 (br. s., 1 H) 2.25 (m, 1 H) 2.44 - 2.54 (m, 2 H) 2.74 (m, 2 H) 2.84 - 3.00 (br. s., 3 H) 3.32 - 3.49 (m, 2 H) 3.61 (m, 1 H) 3.94 (s, 3 H) 5.37 (t, *J*=4.15 Hz, 1 H) 6.83 - 6.96 (m, 2 H) 7.26 - 7.36 (m, 1 H) 7.36 - 7.46 (m, 2 H) 7.65 (d, *J*=4.57 Hz, 1 H) 7.91 (d, *J*=9.14 Hz, 1 H) 8.63 (d, *J*=4.57 Hz, 1 H).

Example 40 is a > 95:5 mixture of alcohol diastereomers having an undetermined mixture of cis / trans isomers on the cyclobutane ring as a white solid foam (37.1 mg, 41%) after purification via preparatory HPLC; 5-50% CH₃CN: H₂O with 0.1% formic acid, 10 min on an Xterra 30 x 50 C18 column. The configuration at the alcohol stereocenter was assigned relative to known compounds. ¹H NMR (400 MHz, CD₃OD) *δ* (ppm) 1.60 - 1.90 (m, 5 H) 1.94 (br. m, 1 H) 2.10 (br, m., 1 H) 2.26 (m, 1 H) 2.40 - 2.54 (m, 2 H) 2.70 -3.20 (br. m, 5 H) 3.34 - 3.51 (m, 2 H) 3.63 (m, 1 H) 3.95 (s, 3 H) 5.37 (t, *J*=4.57 Hz, 1 H) 6.91 - 7.10 (m, 3 H) 7.36 - 7.47 (m, 2 H) 7.66 (d, *J*=4.57 Hz, 1 H) 7.91 (d, *J*=9.97 Hz, 1 H) 8.64 (d, *J*=4.57 Hz, 1 H).

Example 41 is a > 95:5 mixture of alcohol diastereomers having an undetermined mixture of cis / trans isomers on the cyclobutane ring as a white solid foam (59.1 mg, 58%) after purification via preparatory HPLC; 5-60% CH₃CN: H₂O with 0.1% formic acid, 13 min on an Xterra 30 x 50 C18 column. The configuration at the alcohol stereocenter was assigned relative to known compounds. ¹H NMR (400 MHz, CD₃OD) *δ* (ppm) 1.69-1.93 (br. m., 7 H) 2.00-2.30 (m, 2 H) 2.31-2.51 (m, 1 H) 2.71-2.97 (m, 5 H) 3.16 (m, 1 H) 3.23 (m, 1 H) 3.46 (m, 1 H) 3.55 (m, 1 H) 3.94 (s, 3 H) 5.36 (t, *J*=7.89 Hz, 1 H) 7.26 (m, 3 H) 7.38-7.43 (m, 4 H) 7.52 (m, 4 H) 7.64 (d, *J*=4.57 Hz, 1 H) 7.91 (d, *J*=9.55 Hz, 1 H) 8.63 (d, *J*=4.57 Hz, 1 H).

Example 45 was purified via preparatory HPLC; 5-60% CH₃CN: H₂O with 0.1% formic acid, 9 min on an Xterra 30 x 50 C18 column, to provide the title compound as a white solid foam (354.3 mg, 59%) as a mixture of alcohol diasteromers (c.a. 1:1) having an undetermined mixture of cis / trans isomers on the cyclobutane ring. The mixture was subjected to chiral preparatory HPLC, (70/30 CO₂/MeOH to provide the following:

Example 46: (3R,4R)-1-(3-cyclopentylcyclobutyl)-4-[(3R)-3-hydroxy-3-(6-methoxyquinolin-4-yl)propyl]piperidine-3-carboxylic acid eluted as a single peak from 2.43 - 2.80 min as a 95:5 mixture of alcohol diastereomers having an undetermined mixture of cis / trans isomers on the cyclobutane ring as a solid white foam (25.6 mg 4.2%). For the major isomer, the configuration at the alcohol stereocenter was assigned relative to known compounds. ¹H NMR shows a mixture of two compounds. ¹H NMR (400 MHz, CD₃OD) *δ* (ppm) 1.11 (m, 2 H) 1.30 - 2.11 (br. m., 18 H) 2.35 - 2.45 (br. m., 3 H) 2.70 - 2.87 (m, 2 H) 3.30 - 3.46 (m, 2 H) 3.94 (s, 3 H) 5.35 (t, *J*=5.40 Hz, 1 H) 7.37 (d, *J*=3.32 Hz, 1 H) 7.39 (s, 1 H) 7.60 (d, *J*=4.57 Hz, 1 H) 7.91 (d, *J*=9.14 Hz, 1 H) 8.62 (d, *J*=4.57 Hz, 1 H).

Example 47: (3R,4R)-1-(3-cyclopentylcyclobutyl)4-[(3*S*)-3-hydroxy-3-(6-methoxyquinolin-4-yl)propyl]piperidine-3-carboxylic acid eluted as a single peak from 3.13 - 4.69 min as a 95:5 mixture of alcohol diasteromers and an undetermined mixture of cis / trans isomers on the cyclobutane ring as a solid white foam (44.3 mg 7.3%). ¹H NMR shows a single diasteromer. The configuration at the alcohol stereocenter was assigned relative to known compounds. ¹H NMR (400 MHz, CD₃OD *δ* (ppm) 1.09 (br. s., 2 H) 1.37 - 1.90 (br. m., 16 H) 1.92 - 2.18 (br. m., 2 H) 2.38 (br. s., 2 H) 2.70 (br. s., 3 H) 3.34 -3.43 (br. s., 2 H) 3.85 - 3.99 (s, 3 H) 5.36 (br. m., 1 H) 7.36 - 7.39 (br. m., 2 H) 7.63 (d, *J*=4.15 Hz, 1 H) 7.90 (d, *J*=9.14 Hz, 1 H) 8.62 (d, *J*=4.15 Hz, 1 H).

Example 48: (3R,4R)-1-(3-cyclopentylcyclobutyl)-4-[(3S)-3-hydroxy-3-(6-methoxyquinolin-4-yl)propyl]piperidine-3-carboxylic acid eluted as a single peak from 4.42 - 5.40 min as a 95:5 mixture of alcohol diasteromers having an undetermined mixture of cis / trans isomers on the cyclobutane ring as a solid white foam (57.0 mg 9.4%). For the major isomer, the configuration at the alcohol stereocenter was assigned relative to known compounds. ¹H NMR (400 MHz, CD₃OD) *δ* (ppm) 1.12 (m, 2 H) 1.44 - 2.11 (br. m., 18 H) 2.38 (br. m., 2 H) 2.70 (br. m., 3 H) 3.29 (br. m., 1 H) 3.43 (m, 2 1) 3.93 (s, 3 H) 5.35 (t, *J*=5.40 Hz, 1 H) 7.35 - 7.43 (m, 2 H) 7.60 (d, *J*=4.15 Hz, 1 H) 7.91 (d, *J*=9.14 Hz, 1 H) 8.63 (d, *J*=4.15 Hz, 1 H).

Example 56 is a > 95:5 mixture of alcohol diastereomers having an undetermined mixture of cis / trans isomers on the cyclobutane ring as a white solid foam (92.3 mg, 84.6%) after purification via preparatory HPLC; 5-50% CH₃CN: H₂O with 0.1 % formic acid, 9 min on an Xterra 30 x 50 C18 column. ¹H NMR (400 MHz, CD₃OD) *δ* (ppm) 1.64 - 2.02 (m, 6 H) 2.06 (br. s., 3 H) 2.08 (s, 3 H) 2.41 (m, 1 H) 2.73 (m, 2 H) 2.84 (br. s., 3 H) 3.31 - 3.43 (m, 2 H) 3.59 (m 1 H) 3.93 (s, 3 H) 5.37 (m, 1 H) 6.87 (t, *J*=8.93 Hz, 1 H) 6.97 (d, *J*=7.89 Hz, 1 H) 7.09 - 7.19 (m, 1 H) 7.34 - 7.44 (m, 2 H) 7.65 (d, *J*=4.57 Hz, 1 H) 7.90 (d, *J*=9.14 Hz, 1 H) 8.63 (d, *J*=4.57 Hz, 1 H).

Example 59, Diastereomer A (4.5 mg), was obtained (after prep HPLC and silica chromatography) as a white solid, in greater than 85% d.e. (diastereomeric excess). ¹H NMR (400 MHz, CD₃OD) *δ* (ppm) 1.53 (br. m., 1 H) 1.78 (br. m., 1 H) 1.85 (m, 2 H) 2.15 - 2.32 (m, 1 H) 2.21 (m, 2 H) 2.40 (br. m., 1 H) 2.54 (m, 1 H) 2.66 (br. s., 1 H) 2.79 (m, 4 H) 3.46 (m, 2 H) 3.61 (m, 1 H) 3.78 (m, 1 H) 3.94 (s, 3 H) 5.48 (m, 1 H) 6.93 - 7.14 (m, 3 H) 7.33 (dd, *J*=9.14, 2.49 Hz, 1 H) 7.89 (d, *J*=9.14 Hz, 1 H) 7.96 (d, *J*=2.49 Hz, 1 H) 8.52 (d, *J*=1.66 Hz, 1 H).

Example 60, Diastereomer B (4.9 mg), was obtained (after prep HPLC and silica chromatography) as a white solid in greater than 90% d.e. (diasteromeric excess). ¹H NMR (400 MHz, CD₃OD *δ*(ppm) 1.12 - 1.35 (m, 2 H) 1.74 - 1.91 (m, 4 H) 1.94 - 2.14 (m, 1 H) 2.17 - 2.35 (m, 1 H) 2.39 (m,2 H) 2.74 (br. m., 5 H) 3.34 - 3.58 (m, 3 H) 3.94 (s, 3 H) 5.50 (t, *J*=7.27 Hz, 1 H) 6.92 - 7.13 (m, 3 H) 7.33 (dd, *J*=9.14, 2.49 Hz, 1 H) 7.90 (d, *J*=9.14 Hz, 1 H) 8.00 (d, *J*=2.49 Hz, 1 H) 8.53 (d, *J*=1.66 Hz, 1 H).

Example 61 is a > 95:5 mixture of alcohol diastereomers having an undetermined mixture of cis / trans isomers on the cyclobutane ring obtained as a white solid foam (66.5 mg, 85%). ¹H NMR (400 MHz, CD₃OD) *δ* (ppm) 1.64 - 2.10 (br., m, 6 H) 2.26 (s, 3 H) 2.39 - 2.59 (m, 1 H) 2.45 (m, 1 H) 2.72 (m, 2 H) 2.86 (br. m., 4 H) 3.38 (m, 2 H) 3.63 (m, 2 H) 3.94 (s, 3 H) 5.37 (t, *J*=4.15 Hz, 1 H) 6.86 (d, *J*=9.97 Hz, 1 H) 6.97 (d, *J*=4.98 Hz, 1 H) 7.06 (d, *J*=7.06 Hz, 1 H) 7.35 - 7.50 (m, 2 H) 7.67 (d, *J*=4.57 Hz, 1 H) 7.90 (d, *J*=9.55 Hz, 1 H) 8.64 (d, *J*=4.57 Hz, 1 H).

Example 62 is a > 95:5 mixture of alcohol diasteromers having an undetermined mixture of cis / trans isomers on the cyclobutane ring obtained as a white solid foam (47.9 mg, 55%). ¹H NMR (400 MHz, CD₃OD) *δ* (ppm) 1.77 - 1.99 (br. m.,6 H) 2.11 (br, s., 1 H) 2.30 (m, 1 H) 2.54 (m, 2 H) 2.82 - 3.04 (br. m., 5 H) 3.34 - 3.40 (m, 1 H) 3.52 (m, 1 H) 3.62 - 3.72 (m, 1 H) 3.94 (s, 3 H) 5.37 (m, 1 H) 7.23 (t, *J*=9.14 Hz, 1 H) 7.35 - 7.43 (m, 2 H) 7.55 - 7.70 (m, 3 H) 7.91 (d, *J*=9.14 Hz, 1 H) 8.64 (d, *J*=4.57 Hz, 1 H).

Example 63 is a > 95:5 mixture of alcohol diasteromers having an undetermined mixture of cis / trans isomers on the cyclobutane ring obtained as a white solid foam (71.8 mg, 75.5%). ¹H NMR (400 MHz, CD₃OD) *δ* (ppm) 1.59 - 2.16 (br. m., 7 H) 2.58 (m, 1 H) 2.71 (m, 2 H) 2.86 (br. s., 5 H) 3.33 (br. s., 1 H) 3.59 (m, 2 H) 3.94 (s, 3 H) 5.37 (m, 1 H) 6.97 - 7.06 (m, 1 H) 7.14 - 7.23 (m, 2 H) 7.35 - 7.43 (m, 2 H) 7.67 (d, *J*=4.57 Hz, 1 H) 7.91 (d, *J*=9.55 Hz, 1 H) 8.64 (d, *J*=4.57 Hz, 1 H).

Example 64 is a > 95:5 mixture of alcohol diasteromers having an undetermined mixture of cis / trans isomers on the cyclobutane ring obtained as a white solid foam (61.9 mg, 76%). ¹H NMR (400 MHz, CD₃OD) *δ* (ppm) 1.60 - 2.20 (br. m., 7 H) 2.10 (br. s., 1 H) 2.26 (m, 1 H) 2.49 (m, 1 H) 2.77 (m, 2 H) 2.88 (br. m., 3 H) 3.33 - 3.55 (m, 2 H) 3.60 - 3.72 (br., m, 2 H) 3.94 (s, 3 H) 5.38 (t, *J*=4.57 Hz, 1 H) 7.11 (d, *J*=7.89 Hz, 1 H) 7.21 (t, *J*=6.85 Hz, 1 H) 7.27 (m, 1 H) 7.39 (d, *J*=4.57 Hz, 2 H) 7.68 (d, *J*=4.98 Hz, 1 H) 7.91 (d, *J*=9.55 Hz, 1 H) 8.65 (d, *J*=4.57 Hz, 1 H).

Example 65 is a > 95:5 mixture of alcohol diasteromers having an undetermined mixture of cis / trans isomers on the cyclobutane ring obtained as a white solid foam (37 mg, 53%). ¹H NMR (400 MHz, CD₃OD) *δ* (ppm) 1.57 - 1.90 (br. m., 7 H) 2.26 (br. m., 1 H) 2.41 (m, 1 H) 2.76 (br. m., 5 H) 3.38 - 3.63 (m, 4 H) 3.96 (s, 3 H) 5.37 (br, m., 1 H) 7.12 (d, *J*=9.97 Hz, 1 H) 7.18 (d, *J*=8.31 Hz, 1 H) 7.32 - 7.41 (br. m., 3 H) 7.64 (d, *J*=4.57 Hz, 1 H) 7.91 (d, *J*=9.14 Hz, 1 H) 8.64 (d, *J*=4.15 Hz, 1 H).

Example 66 is a > 95:5 mixture of alcohol diasteromers having an undetermined mixture of cis / trans isomers on the cyclobutane ring obtained as a white solid foam (51.5 mg, 63%). ¹H NMR (400 MHz, CD₃OD) *δ* (ppm) 1.01 (m, 2 H) 1.25 (m, 1 H) 1.60 - 1.84 (br. m., 6 H) 2.17 (br. s., 1 H) 2.70 - 2.94 (m, 4 H) 3.05 - 3.22 (m, 2 H) 3.41 - 3.68 (br. m., 4 H) 3.91 - 4.04 (m, 3 H) 5.39 (br. m., 1 H) 6.91 (t, J=8.31 Hz, 2 H) 7.25 (m, 1 H) 7.36 - 7.51 (m, 2 H) 7.69 (br. s., 1 H) 7.92 (d, *J*=9.14 Hz, 1 H) 8.65 (d, *J*=3.74 Hz, 1 H).

Example 67 is a > 95:5 mixture of alcohol diasteromers having an undetermined mixture of cis / trans isomers on the cyclobutane ring obtained as a white solid foam (53.9 mg, 66%). ¹H NMR (400 MHz, CD₃OD) *δ* (ppm) 1.29 (br. m., 3 H) 1.60 - 2.12 (br. m., 7 H) 2.41 (m, 1 H) 2.55 (m, 1 H) 2.72 (m, 1 H) 2.80 - 3.20 (br. m., 5 H) 3.31 - 3.52 (m, 2 H) 3.95 (s, 3 H) 5.38 (br. m., 1 H) 6.90 (t, *J*=8:31 Hz, 2 H) 7.24 (m, 1 H) 7.36 - 7.50 (m, 2 H) 7.67 (d, *J*=4.15 Hz, 1 H) 7.91 (d, *J*=9.14 Hz, 1 H) 8.65 (d, *J*=3.32 Hz, 1 H).

Example 68 is a > 95:5 mixture of alcohol diasteromers having an undetermined mixture of cis / trans isomers on the cyclobutane ring obtained as a white solid foam (46.1 mg, 55%). ¹H NMR (400 MHz, CD₃OD) *δ* (ppm) 1.60 - 2.11 (br. m., 7 H) 2.53 (m, 1 H) 2.67 (m, 2 H) 2.84 (br. m., 5 H) 3.33 - 3.55 (m, 2 H) 3.61 (m, 1 H) 3.94 (s, 3 H) 5.37 (m, 1 H) 6.93 (t, *J*=8.72 Hz, 1 H) 7.29 - 7.44 (m, 3 H) 7.66 (d, *J*=4.57 Hz, 1 H) 7.91 (d, *J*=9.55 Hz, 1 H) 8.64 (d, *J*=4.57 Hz, 1 H).

Example 69 is a > 95:5 mixture of alcohol diasteromers having an undetermined mixture of cis / trans isomers on the cyclobutane ring obtained as a white solid foam (44.4 mg, 54%). ¹H NMR (400 MHz, CD₃OD) *δ* (ppm) 1.71 (d, *J*=10.80 Hz, 4 H) 1.85 (br. s., 2 H) 1.99 (d, *J*=18.69 Hz, 1 H) 2.12 (br. s., 1 H) 2.50 (q, *J*=9.97 Hz, 1 H) 2.64 (d, *J*=6.65 Hz, 1 H) 2.83 (d, *J*=6.23 Hz, 5 H) 3.29 - 3.36 (m, 1 H) 3.36 - 3.52 (m, 1 H) 3.43 (d, *J*=8.31 Hz, 1 H) 3.61 (d, *J*=7.48 Hz, 1 H) 3.95 (s, 4 H) 5.37 (d, *J*=4.15 Hz, 1 H) 6.80 (t, *J*=8.93 Hz, 2 H) 7.36 - 7.48 (m, 2 H) 7.67 (d, *J*=4.15 Hz, 1 H) 7.91 (d, *J*=9.97 Hz, 1 H) 8.64 (d, *J*=4.57 Hz, 1 H).

Example 70 is a > 95:5 mixture of alcohol diasteromers having an undetermined mixture of cis / trans isomers on the cyclobutane ring obtained as a white solid foam (59.1 mg, 58%). ¹H NMR (400 MHz, CD₃OD) *δ* (ppm) 1.69-1.93 (br. m., 7 H) 2.00-2.30 (m, 2 H) 2.31-2.51 (m, 1 H) 2.71-2.97 (m, 5 H) 3.16 (m, 1 H) 3.23 (m, 1 H) 3.46 (m, 1 H) 3.55 (m, 1 H) 3.94 (s, 3 H) 5.36 (t, *J*=7.89 Hz, 1 H) 7.26 (m, 3 H) 7.38-7.43 (m, 4 H) 7.52 (m, 4 H) 7.64 (d, *J*=4.57 Hz, 1 H) 7.91 (d, *J*=9.55 Hz, 1 H) 8.63 (d, *J*=4.57 Hz, 1 H).

### Example 73: methyl (3R,4R)-1-(5-fluoro-2,3-dihydro-1H-inden-1-yl)-4-[3-hydroxy-3-(6-methoxyquinolin-4-yl)propyl]piperidine-3-carboxylate

Step 1: (3R,4R)-methyl 4-(3-(6-methoxyquinolin-4-yl)-3-oxopropyl)piperidine-3-carboxylate (2HCl) and 5-fluoro-2,3-dihydro-1H-inden-1-yl methanesulfonate (2 equiv.) were combined in THF (15 mL) and DMF (5 mL). To this was added TEA (2.8 mL, 22.09 mmol) and powdered K₂CO₃ (610 mg, 4.419 mmol). The reaction vessel was then sealed and heated at 50°-65° C for 5 days, whereupon the reaction was filtered. The crude material was purified on silica gel (gradient elution of 0% to 75% EtoAc / Hexanes over 55 min) provided 260 mg of product.

Step 2: To a solution of the product of Step 1 (260 mg, 0.53 mmol) in MeOH (5 mL) was added NaBH₄ (24 mg, 0.63 mmol). The resulting mixture was stirred for 2.5 h at 25°C. The reaction mixture was subsequently quenched by the addition of H₂O (3.0 mL) and stirred overnight (16 hrs). The mixture was then concentrated and purified by silica gel chromatography (gradient elution of 55% to 100% EtoAc : Hexanes over 55 min) to provide the title compound as a mixture of four diastereomers as a white solid foam (153 mg, 59%). LCMS: ret. time 1.1 min, [M+H]⁺ 493.0.

### Example 74: (3R,4R)-1-(5-fluoro-2,3-dihydro-1 H-inden-1-yl)-4-(3-hydroxy-3-(6-methoxyquinolin-4-yl)propyl)piperidine-3-carboxylic acid

To a solution of the title compound of Example 73 (264 mg, 0.5359 mmol) in THF (3.0 mL) and H₂O (1.0 mL) was added LiOH (32 mg, 1.33 mmol), and the resulting mixture stirred at 25°C. Upon completion by TLC, the reaction mixture was neutralized (ca. pH 7) with 1M HCl, concentrated and purified via chromatography eluting with DCM: MeOH : NH₄OH (40 : 4 : 0.05) to afford the title compound as a mixture of four diasteromers as a white solid foam (124.2 mg, 48%) LCMS (ESI): 1.1 min, [M+H)⁺ 479.4.

The title compound of Example 74 was subjected to chiral prep HPLC, using a 2.1 x 250 AD-H column (70/30 Heptane/EtOH) to afford Example 75, (3R,4R)-1-(5-fluoro-2,3-dihydro-1H-inden-1-yl)-4-((S)-3-hydroxy-3-(6-methoxyquinolin-4-yl)propyl)piperidine-3-carboxylic acid as a single diastereomer, eluting at 7.197 - 7.857 min, as a white solid (18.3 mg, 7.1%). Stereochemistry of the alcohol was assigned by ¹H NMR. ¹H NMR (400 MHz, CD₃OD δ (ppm) 1.24 (br. m., 2 H) 1.55 - 1.80 (br. m., 6 H) 1.97 (br. m., 2 H) 2.34 (br. s., 2 H) 2.63 (br. s., 1 H) 2.73 (br. m., 1 H) 2.89 (br. m., 2 H) 3.08 (br. m., 1 H) 3.95 (br. s., 3 H) 5.32 (br. m., 1 H) 7.03 (br. s., 2 H) 7.43 (br. s., 3 H) 7.64 (br. s., 1 H) 7.91 (s, 1 H) 8.62 (br. s., 1 H).
Table 3 provides additional non-limiting Examples of Formula I that were prepared in a manner analogous to that described in Examples 74 or 75 using the appropriate starting materials. Unless otherwise noted, the LCMS data was acquired using standard conditions.

**TABLE 3**

| Ex.# | NAME | Ret Time | MS (M+1) |
|---|---|---|---|
| 76 | (3R,4R)-methyl 4-(3-hydroxy-3-(6-methoxyquinolin-4-yl) propyl)-1-(1,2,3,4-tetrahydronaphthalen-1-yl)piperidine-3-carboxylate | 1.1 | 489.5 |
| 77 | (3R,4R)-4-(3-hydroxy-3-(6-methoxyquinolin-4-yl)propyl)-1-(1,2,3,4-tetrahydronaphthalen-1-yl)piperidine-3-carboxylic acid | 1.2 | 475.5 |
| 78 | (3R,4R)-methyl 1-(2,3-dihydro-1H-inden-2-yl)-4-(3-hydroxy-3-(6-methoxyquinolin-4-yl)propvl)piperidine-3-carboxylate | 1.1 | 475.5 |
| 79 | (3R,4R)-1-(2,3-dihydro-1H-inden-2-yl)-4-(3-hydroxy-3-(6-methoxyquinolin-4-yl)propyl)piperidine-3-carboxylic acid | 1.15 | 461.5 |
| 80 | (3R,4R)-1-(5-chloro-2,3-dihydro-1H-inden-2-yl)-4-(3-hydroxy -3-(6-methoxyquinolin-4-yl)propyl)piperidine-3-carboxylic acid | 1.45 | 495.2 |
| 81 | (3R,4R)-1-(4-bromo-2,3-dihydro-1H-inden-2-yl)-4-(3-hydroxy -3-(6-methoxyquinolin-4-yl)propyl)piperidine-3-carboxylic acid | 1.4 | 541.3 |
| 82 | (3R,4R)-methyl 1-((2,3-dihydro-1H-inden-2-yl)methyl)-4-(3-hydroxy-3-(6-methoxyquinolin-4-yl)propyl)piperidine-3-carboxylate | 1.3 | 489.2 |
| 83 | (3R,4R)-1-((2,3-dihydro-1H-inden-2-yl)methyl)-4-(3-hydroxy-3-(6-methoxyquinolin-4-yl)propyl)piperidine-3-carboxylic acid | 1.2 | 475.2 |
| 84 | (3R,4R)-1-((2-hydroxy-2,3-dihydro-1H-inden-2-yl)methyl)-4-(3-hydroxy-3-(6-methoxyquinolin-4-yl)propyl)piperidine-3-carboxylic acid | 1.0 | 491.5 |

### Example 85: (3R,4R)-4-[3-hydroxy-3-(6-methoxyquinolin-4-yl)propyi]-1-{[(trans)-2-phenylcyclopropyl]methyl}piperidine-3-carboxylic acid

Example 5 (0.074g, 0.151mmol) and LiOH (0.018g, 0.757mmol) were combined in 2 mL THF, 2 mL MeOH and 1 mL H₂O. The resulting mixture was heated at 40°C overnight. The reaction was then diluted with H₂O, and the pH adjusted to pH 3 with 1N aq. HCl. The aqueous layer was washed once with EtOAc, and then the aqueous layer was concentrated to dryness. The crude material was purified via cation exchange chromatography washing with MeOH and eluting with 0.25M NH₄OH in MeOH to afford the title compound as a yellow solid (0.048g).
Table 4 provides additional non-limiting compounds of Formula I that were prepared in a manner analogous to that described in Example 85 using the appropriate starting materials. Unless otherwise noted, LCMS data was acquired using standard conditions.

**TABLE 4**

| Ex.# | NAME | Ret Time | MS (M+1) |
|---|---|---|---|
| 86 | 3-[3-(3-chloro-6-methoxyquinolin-4-yl)propyl] -1-(3-phenylcyclobutyl)pyrrolidine-3-carboxylic acid | 1.95 | 479 |
| 87 | 3-[3-(3-chloro-6-methoxyquinolin-4-yl)propyl]-1-[3-(2,6-difluorophenyl)cyclobutyl] pyrrolidine-3-carboxylic acid | 2.04 | 515 |
| 88 | (3R,4R)-1-[3-(4-fluorophenyl)cyclobutyl]-4-[3-hydroxy-3-(6-methoxyquinolin-4-yl)propyl] piperidine-3-carboxylic acid | 1.2-1.3 | 493.1 |
| 93 | 4-[3-(6-methoxyquinolin-4-yl)propyl]-1-(3-phenylcyclobutyl)piperidine-4-carboxylic acid | 1.1 | 459.3 |

### Examples 89 and 90: (3R,4R)-1-[3-(2-fluorophenyl)cyclobutyl]-4-[3-hydroxy-3-(6-methoxyquinolin-4-yl)propyl]piperidine-3-carboxylic acid

Examples 89 and 90 were prepared in a manner analogous to that described in Example 85 and were isolated from a mixture of alcohol diasteromers (c.a. 1:1) having an undetermined mixture of cis / trans isomers on the cyclobutane ring via chromatography. The mixture (0.544g in 1.5 mL of DMAC) was loaded onto a 40 g silica gel Redisep column pre-equilibrated in 1:8:10 MeOH:CHCl₃:EtOAc, eluting with 4 L of 1:4:5 MeOH:CHCl₃:EtOAc. Fractions were analyzed by 1 H NMR to assess the separation of the diastereomers. Concentration of the appropriate fractions afforded the following:

Example 89, (3R,4R)-1-[3-(2-fluorophenyl)cyclobutyl]-4-[(3S)-3-hydroxy-3-(6-methoxyquinolin-4-yl)propyl]piperidine-3-carboxylic acid (125 mg, 96% pure), as a white solid. ¹H NMR (400 MHz, CD₃OD) *δ*(ppm) 1.71 (br s., 4 H) 1.84 (br. s., 4 H) 2.27 (br. s., 1 H) 2.30 (br q, 1 H), 2.40 (m, 1 H), 2.7-2.85, (m, ca/ 6H), 3.48 (m, ca. 4H) 3.97 (s, 3 H) 5.38 (m, 1 H), 7.02 (t, J= 8 Hz, 1 H), 7.15 (t, J = 7.5 Hz, 1 H), 7.23 (m, 1 H) 7.32 (t, *J*=7.48 Hz, 1 H), 7.39 (dd, *J*=9.24, 2.49 Hz, 1 H) 7.42 (br s, 1 H), 7.64 (d, J= 4.5 Hz, 1 H), 7.91 (d, J= 9.1 Hz, 1 H), 8.64 (d, J=4.6Hz, 1 H).

Example 90, (3R,4R)-1-[3-(2-fluorophenyl)cyclobutyl]-4-[(3R)-3-hydroxy-3-(6-methoxyquinolin-4-yl)propyl]piperidine-3-carboxylic acid (222 mg, 95% pure) as a white solid. ¹H NMR (400 MHz, CD₃OD) *δ* (ppm) 1.45 (br s, 1 H), 1.80 (br s., ca. 4 H) 1.9-2.05 (m, 4 H) 2.25 (br q, 1 H), 2.39 (br m, 1 H), 2.7 (s, 1 H), 2.7-2.85, (m, ca. 4H), 3.48 (m, ca. 2H) 3.55 (br s, 1 H) 3.97 (s, 3 H) 5.36 (m, 1 H), 7.03 (t, ca 9 Hz, 1 H), 7.15 (t, J = 7.5 Hz, 1 H), 7.23 (m, 1 H) 7.32 (t, *J*=7.48 Hz, 1 H), 7.39 (dd, *J*=9.24, 2.49 Hz, 1 H) 7.42 (br s, 1 H), 7.60 (d, J= 4.5 Hz, 1 H_), 7.91 (d, J= 9.2 Hz, 1 H), 8.63 (d, J= 4.5Hz, 1 H).

**Examples 91 and 92**: (3R,4R)-1-[3-(3-fluorophenyl)cyclobutyl]-4-[3-hydroxy-3-(6-methoxyquinolin-4-yl)propyl]piperidine-3-carboxylic acid were prepared in a manner analogous to that described in Example 85 and were isolated from a mixture of alcohol diasteromers (c.a. 1:1) having an undetermined mixture of cis / trans isomers on the cyclobutane ring via chromatography. The mixture (0.89 g in 3 mL of DMAC) was loaded onto a 40 g silica gel Redisep column pre-equilibrated in 1:8:10 MeOH:CHCl₃:EtOAc, eluting first with 3 L of 1:4:5 MeOH:CHCl₃:EtOAc, then with 1 L of 1.3:4:5 MeOH:CHCl₃:EtOAc, and finally 500 mL of 3:4:5 MeOH:CHCl₃:EtOAc. Analysis of pertinent fractions was carried out by multiple elutions on TLC in 1:4:5 MeOH:CHCl₃:EtOAc and purity assessed by 1H NMR. Concentration of the relevant fractions afforded the following:

Example 91, (3R,4R)-1-[3-(3-fluorophenyl)cyclobutyl]-4-[(3R)-3-hydroxy-3-(6-methoxyquinolin-4-yl)propyl]piperidine-3-carboxylic acid (173 mg, 97% pure) as a white solid. ¹H NMR (400 MHz, CD₃OD) *δ* (ppm) 1.7 (br s, 4H), ca. 1.8 (br s, 2H) 2.17 (br m, 1 H) 2.36 (brq, J=9.97 Hz, 1 H), 2.5 (m, ca. 0.5 H), 2.71 (br. s., ca. 4 H) 3.24 (m, 1 H), , 3.4 (m, 1 H), ca. 3.52 (m, 1 H), 3.95 (s, 3 H) 5.36 (t, *J*=7.06 Hz, 1 H) 6.91 (td, J=8.31 Hz, J'=2 Hz, 1 H) 7.0 (m, 2 H), 7.29 (m, 1 H) 7.37 (dd, J=9.1 Hz, J'=2.5 Hz, 1 H) ca. 7.40 (br s, 1 H) 7.63 (d, *J*=4.57 Hz, 1 H) 7.90 (d, *J*=9.14 Hz, 1 H) 8.62 (d, *J*=4.57 Hz, 1 H).

Example 92, (3R,4R)-1-[3-(3-fluorophenyl)cyclobutyl]-4-[(3S)-3-hydroxy-3-(6-methoxyquinolin-4-yl)propyl]piperidine-3-carboxylic acid (260 mg, 98% pure) as a white solid. 1 H NMR (400 MHz, CD₃OD) *δ* (ppm) 1.45 (br. s., 1 H), 1.8 (br s, 4H), ca. 1.95 (m, 2H) 2.17 (brq, *J*=10.39 Hz, 1 H) 2.39 (brq, *J*=9.97 Hz, 1 H) 2.71 (br. s., 4 H) 3.24 (m, 1 H), ca. 3.5 (m, 3 H), 3.93 (s, 3 H) 5.36 (t, *J*=7.06 Hz, 1 H) 6.91 (t, *J*=8.31 Hz, 1 H) 7.03 (brt, *J*=8.31 Hz, 2 H) 7.25 - 7.34 (m, 1 H) ca. 7.40 (m, 2 H) 7.60 (d, *J*=4.57 Hz, 1 H) 7.91 (d, *J*=9.14 Hz, 1 H) 8.62 (d, *J*=4.57 Hz, 1 H).

### Examples 94, 95 and 96: (3R,4R)-4-[3-hydroxy-3-(6-methoxyquinolin-4-yl)propyl]-1-(3-hydroxy-3-phenylcyclobutyl)piperidine-3-carboxcylic acid

(3R,4R)-4-(3-hydroxy-3-(6-methoxyquinolin-4-yl)propyl)piperidine-3-carboxylic acid (158mg, 0.38mmol) and 3-hydroxy-3-phenylcyclobutanone (88mg, (70% pure, 0.38mmol) were combined in MeOH and allowed to stir at 25°C for 75 minutes before the addition of MP-cyanoborohydride resin (182mg, 0.42mmol). The resulting reaction mixture was stirred for 3 days at 25°C, whereupon the reaction was filtered and concentrated. The residue was taken up in DMSO (1ml) and purified via preparatory HPLC (gradient elution of 0-35% B over 9 minutes, where A=0.1 % formic acid in H₂O, B= 0.1% formic acid in acetonitrile) to afford the title compound, isolated as three enriched diastereomeric mixtures of undetermined stereochemistry. Examples 94, 95 and 96 had retention times of 3.5 min, 3.93 min, and 4.55 min, respectively.

Example 94: ¹H NMR (400 MHz, CD₃OD) *δ* (ppm) 1.36 - 1.49 (m, 1 H), 1.76 - 1.96 (m, 3 H), 1.98 - 2.11 (m, 2 H), 2.33 - 2.42 (m, 1 H), 2.57 - 2.72 (m, 7 H), 3.11 - 3.20 (m, 1 H), 3.20 - 3.28 (m, 1 H), 3.34 - 3.49 (m, 2 H), 3.91 - 4.02 (m, 4 H), 4.07 - 4.11 (m, 1 H), 5.36 - 5.44 (m, 1 H), 7.24 - 7.32 (m, 1 H), 7.34 - 7.46 (m, 6 H), 7.64 (t, 1 H), 7.92 - 7.98 (m, 1 H), 8.21 (br. s., 3 H), 8.66 (d, 1 H).

Example 95: ¹H NMR (400 MHz, CD₃OD *δ* (ppm) 1.76 (dd, 3 H), 1.86 (br. s., 2 H), 1.95 - 2.09 (m, 2 H), 2.58 - 2.72 (m, 4 H), 2.75 - 2.83 (m, 2 H), 2.89 - 3.10 (m, 2 H), 3.89 - 4.01 (m, 4 H), 4.06 - 4.11 (m, 1 H), 5.40 (d, 1 H), 7.28 (d, 1 H), 7.34 - 7.52 (m, 6 H), 7.66 (dd, 1 H), 7.94 (dd, 1 H), 8.16 (s, 4 H), 8.67 (d, 1 H).

Example 96: ¹H NMR (400 MHz, CD₃OD) *δ* (ppm) 1.40-52 (br. s 1H), 1.73 (br. s., 2 H), 1.87 (br. s., 3 H), 2.04 (s, 1 H), 2.25-38 (br. s, 1H)2.51 - 2.72 (m, 3 H), 2.79 (br. s., 3 H), 2.90 - 3.08 (m, 3 H), 3.47 (d, 1 H), 3.53 - 3.73 (m, 1 H), 3.98 (d, 3 H), 5.35 - 5.43 (m, 1 H), 7.27 - 7.35 (m, 1 H), 7.36 - 7.46 (m, 4 H), 7.53 (d, 2 H), 7.65 (dd, 1 H), 7.94 (dd, 1 H), 8.18 (s, 2 H), 8.66 (d, 1 H) MS ES+ m/z (M+H)⁺491.3

Table 5 provides additional non-limiting compounds of Formula I that were prepared in a manner analogous to that described in Examples 94-96 using the appropriate starting materials. Unless otherwise noted, LCMS data was acquired using standard conditions.

**TABLE 5**

| Ex.# | NAME | Ret. Time | MS (M+1) |
|---|---|---|---|
| 97^{a} | (3R,4R)-1-[3-(4-chlorophenyl)cyclopentyl]-4-[3-hydroxy-3-(6-methoxyquinolin-4-yl)propyl] piperidine-3-carboxylic acid | 1.74 | 523 |
| 98 | 4-[3-(3-fluoro-6-methoxyquinolin-4-yl) propyl]-1-[3-(3-methyl-1,2,4-oxadiazol-5-yl)cyclobutyl]piperidine-4-carboxylic acid | 1.56 | 483.3 |
| 99^{a} | methyl (3R,4R)-4-[3-hydroxy-3-(6-methoxy quinolin-4-yl)propyl]-1-[3-(5-methyl-1,3,4-oxadiazol-2-yl)cyclobutyl]piperidine-3-carboxylate | .7 | 495.3 |
| 100 | 4-{[2-(3-chloro-6-methoxyquinolin-4-yl)ethyl]amino}-1-(3-phenylcyclobutyl) piperidine-4-carboxylic acid | 1.6 | 494.3 |
| 101 | (2,4)-4-{[2-(3-chloro-6-methoxyquinolin-4-yl)ethyl]amino}-1-(3-phenylcyclobutyl) piperidine-2-carboxylic acid | 1.4 | 494.3 |
| 102 | (3R,4R)-4-(3-hydroxy-3-(6-methoxyquinolin-4-yl)propyl]-1-[3-(3-methyl-1,2,4-oxadiazol-5-yl)cyclobutyl]piperidine-3-carboxylic acid | .67 | 481.3 |
| 103 | (3R,4R)-1-(3-benzylcyclobutyl)-4-[3-hydroxy-3-(6-methoxyquinolin-4-yl)propyl]piperidine-3-carboxylic acid | 1.46 | 489.4 |
| 104 | (3R,4R)-4-(3-hydroxy-3-(6-methoxyquinolin-4-yl)propyl]-1-[3-(pyridin-2-yloxy)cyclobutyl] piperidine-3-carboxylic acid | .53 | 492.3 |
| 105 | methyl (3R,4R)-4-[3-hydroxy-3-(6-methoxy quinolin-4-yl)propyl]-1-[3-(3-methylisoxazol-5-yl)cyclobutyl]piperidine-3-carboxylate | 1.92 | 494.3 |

| | | | |
|---|---|---|---|
| ^{a} Reaction was heated in a microwave for 5-10 minutes at between 80-100° C. | | | |

### Example 106: 4-(3-(3-fluoro-6-methoxy-1,5-naphthyridin-4-yl)propyl)-1-(3-phenylcyclobutyl)piperidine-4-carboxylic acid

Step 1: Ethyl 4-(3-(3-fluoro-6-methoxy-1,5-naphthyridin-4-yl)propyl)piperidine-4-carboxylate (0.2032 g, 0.54 mmol) was dissolved in DMF (1.2 mL) and combined with 3-phenylcyclobutanone (0.1393 g, 0.95 mmol), MP-cyanoborohydride resin (0.4247 g of 2.55 mmol/g resin, 1.1 mmol) and glacial AcOH (0.3 mL) in a microwave vial. The vial was capped and the reaction heated to 80° C for 10 min in a microwave. The crude reaction mixture was poured onto a cation exchange (MCX) column and eluted with MeOH followed by 0.25 M NH₄OH solution in MeOH to provide 0.244 g of product as an oil.

Step 2: To a solution of the product of Step 1 (0.2167 g, 0.43 mmol) in MeOH (1.5 mL) and THF (1.5 mL) was added water (1.5 mL) and NaOH pellets (0.3148 g, 7.87 mmol). The resulting mixture was then hated to 100° C for 4 hours, whereupon the reaction mixture was cooled to 25°C and the pH adjusted to pH 7-8 using a pH 7 phosphate buffer. The aqueous layer was extracted with EtOAc (3 x 50 mL), and the organic layers combined, dried with MgSO₄, filtered, and concentrated to an oil. The crude product was purified by chromatography using a CHCl₃/MeOH as eluent to provide the title compound as a white solid (0.0133 g). LCMS: ret. time 2.23; M+1 478.2.

**Examples 116- 120** were prepared according to the following procedure:
Step 1: To a solution of methyl-(3R, 4R)-4-(3-hydroxy-3-(6-methoxyquinofin-4-yl)propyl)piperidine-3-carboxylate (0.22 mmol) and the appropriate aldehyde (0.15 mmol) in THF (4 mL) was added acetic acid (0.7 ml), and MP-cyanoborohydride (0.58 mmol , 0.25 g Loading Factor: 2.30mmol/g). The reaction was capped and the mixture shaken at 25°C for 16 hours. The reaction was then filtered and the filtrate concentrated to provide a crude product that was used without purification.
Step 2: The product of Step 1 was dissolved in DMSO (1 mL) and 5.0 M KOH (0.1 mL) and allowed to stir overnight at 25°C. The reaction was then filtered through a 5 micron syringe filter and the filtrate purified by RP preparatory HPLC using a HPLC using a 30x50 mm Xterra column (Waters) with an 8 minute elution gradient of 5-40% of solvent A: solvent B (where solvent A is H₂O containing 0.1% formic acid and solvent B is ACN containing 0.1% Formic acid) to give the corresponding acids as colorless foams.

The following non-limiting Examples in Table 6 were prepared in a manner analogous to that described in Example 106 or according to the general procedure outlined above using the appropriate starting materials. Unless otherwise noted, LCMS data was acquired using standard conditions.

**TABLE 6**

| Example | NAME | Ret Time | MS (M+1) |
|---|---|---|---|
| 107 | 4-[3-(6-methoxyquinolin-4-yl)propyl]-1-{[(1,2)-2-phenylcyclopropyl]methyl}piperidine-4-carboxylic acid | 1.7* | 459.5 |
| 108 | 4-[3-(3-chloro-6-methoxyquinolin-4-yl)propyl]-1-(3-phenylcyclobutyl)piperidine-4-carboxylic acid | 2.47 | 493.2 |
| 109 | 4-[3-(3-chloro-6-methoxyquinolin-4-yl)propyl]-3-hydroxy-1-(3-phenylcyclobutyl)piperidine-4-carboxylic acid | 1.87 | 510.6 |
| 110 | (3R,4R)-4-[3-hydroxy-3-(6-methoxyquinolin-4-yl)propyl]-1-[3-(5-methyl-1,3,4-oxadiazol-2-yl)cyclobutyl]piperidine-3-carboxylic acid | .44 | 481.3 |
| 111 | 4-[3-(6-methoxyquinazolin-4-yl)propyl]-1-(3-phenylcyclobutyl)piperidine-4-carboxylic acid | 1.9 | 460.4 |
| 112 | (4)-4-{[2-(3-chloro-6-methoxyquinolin-4-yl)ethyl]amino}-1-(3-phenylcyclobutyl)-proline | 2.12 | 479.9 |
| 113 | 3-{[2-(3-chloro-6-methoxyquinolin-4-yl)ethyl]amino}-1-(cis-3-phenylcyclobutyl)pyrrolidine-3-carboxylic acid | 2.7 | 494.3 |
| 114 | 3-{[2-(3-chloro-6-methoxyquinolin-4-yl)ethyl]amino}-1-(trans-3-phenylcyclobutyl)pyrrolidine-3-carboxylic acid | 2.5 | 494.3 |
| 115 | (3R,4R)-4-[3-hydroxy-3-(6-methoxyquinolin-4-yl)propyl]-1-[3-(3-methylisoxazol-5-yl)cyclobutyl]piperidine-3-carboxylic acid | 1.3 | 480.3 |
| 116 | (3R,4R)-4-[3-hydroxy-3-(6-methoxyquinolin-4-yl)propyl]-1-[(2-isopropyl-5,6,7,8-tetrahydroquinazolin-6-yl)methyllpiperidine-3-carboxylic acid | 1.7 | 533 |
| 117 | (3R,4R)-1'-{[2-(cyclopropylmethyl)-5,6,7,8-tetrahydroquinazolin-6-yl]methyl}-4-[3-hydroxy-3-(6-methoxyquinolin-4-yl)propyl]piperidine-3-carboxylic acid | 1.7 | 545 |
| 118 | (3R,4R)-4-[3-hydroxy-3-(6-methoxyquinolin-4-yl)propyl]-1-[(2-phenyl-5,6,7,8-tetrahydroquinazolin-6-yl)methyl]piperidine-3-carboxylic acid | 2.3 | 567 |
| 119 | (3R,4R)-4-[3-hydroxy-3-(6-methoxyquinolin-4-yl)propyl]-1-[(2-pyrazin-2-yl-5,6,7,8-tetrahydroquinazolin-6-yl)methyl]piperidine-3-carboxylic acid | 1.6 | 569 |
| 120 | (3R,4R)-4-[3-hydroxy-3-(6-methoxyquinolin-4-yl)propyl)-1-[(2-piperidin-1-yl-5,6,7,8-tetrahydroquinazolin-6-vl)methyl]piperidine-3-carboxylic acid | 2.0 | 574 |
| 121 | 4-[3-(7-methoxyisoquinolin-1-yl)propyl]-1-(3-phenylcyclobutyl)piperidine-4-carboxylic acid | 1.24 | 459.4 |
| 122 | 4-{[(2R)-2-hydroxy-2-(6-methoxyquinolin-4-yl)ethyl]amino}-1-(trans-3-phenylcyclobutyl)piperidine-3-carboxylic acid | .86 | 476.3 |
| 123 | 4-{[(2R)-2-hydroxy-2-(6-methoxyquinolin-4-yl)ethyl]amino}-1-(cis-3-phenylcyclobutyl)piperidine-3-carboxylic acid | .93 | 476.3 |

| | | | |
|---|---|---|---|
| * LCMS (polar conditions) | | | |

### Example 124: methyl (3R,4R)-4-[3-hydroxy-3-(6-methoxyquinolin-4-yl)propyl)-1-(1-pyridin-2-ylazetidin-3-yl)piperidine-3-carboxylate

Step 1: Methyl (3R,4R)-4-[3-Hydroxy-3-(6-methoxy-quinolin-4-yl)-propyl]-piperidine-3-carboxylate (0.2g, 0.558mmol) and 3-oxo-azetidine-1-carboxylic acid tert-butyl ester (0.0115g, 0.670mmol) were combined in THF (6 mL) and MeOH (1.5 mL) followed by addition of glacial AcOH (48µL, 0.837mmol) and 4A mol. sieves. The reaction was stirred 3 hours at 25°C, before the addition of NaCNBH₃ (0.042g, 0.670mmol). The reaction was stirred overnight, poured into sat. aq. NaHCO₃ and extracted three times with DCM. The organic extracts were combined, dried over MgSO₄, filtered, and concentrated. The crude material was purified by chromatography (gradient elution using 1% to 10% MeOH in CHCl₃) to afford a viscous yellow oil (0.151g):

Step 2: To a solution of the product of Step 1 (0.151 g, 0.294mmol) in MeOH (3 mL) and DCM (3 mL) was added a solution of HCl in ether (2M, 0.735mL, 1.47mmol). After 2 hours of stirring at 25°C, additional HCl in dioxane (2M, 2mL, 4.0mmol) was added. The reaction was allowed to stir overnight at 25°C, then concentrated to dryness to afford a white solid (0.122g).

Step 3: The product of Step 3 (0.116g, 0.258mmol) was combined with 2-chloropyridine (0.037mL, 0.387mmol) and K₂HPO₄ (0.225g, 1.29mmol) in DMSO (2 mL) and heated at 90°C overnight. Another 2.5 eq. of 2-chloropyridine and 2.5 eq. K₂HPO₄ in DMSO (2 mL) were added and the reaction was allowed to stir at 100°C for 3 days. The reaction was then diluted with H₂O and the pH adjusted to pH 5 with 1 N aq. HCl. The aqueous layer was extracted with EtOAc (3 x). The pH of the aqueous layer was then adjusted to pH 7 with 1 N aq. HCl and extracted with EtOAc (3 x). All organic extracts were then combined, washed with H₂O (3 x), dried over MgSO₄, filtered, and concentrated. The crude material was purified by chromatography (gradient elution using 1% to 10% MeOH in CHCl₃) to afford the title compound as a white solid (0.0283g). LCMS: ret. time 1.35, M+1 491.

### Example 125: (3R,4R)-4-[3-hydroxy-3-(6-methoxyquinolin-4-yl)propyl]-1-(1-pyridin-2-ylazetidin-3-yl)piperidine-3-carboxylic acid

The title compound of Example 124 (0.023g, 0.047mmol) and LiOH (0.006g, 0.24mmol) were combined in MeOH (1 mL), THF (1 mL), and H₂O (0.5 mL) and heated overnight at 40°C. The reaction was diluted with H₂O, adjusted to pH 3 by addition of 1 N aq. HCl, and extracted with EtOAc (3 x). The aqueous layer was then concentrated to dryness. The resulting residue was purified on a cation exchange column (MCX) washing first with MeOH and then eluting with 0.25M NH₄OH in MeOH to afford the title compound as a white solid (0.017g), LCMS: ret. time 0.44, M+1 477.

**Example 126**: 4-(3-(3-chloro-6-methoxyquinolin-4-yl)propy)-1-(1-(pyridin-2-yl)azetidin-3-yl)piperidine4-carboxylic acid was prepared in a manner similar to Example 124 using ethyl 4-(3-(3-chloro-6-methoxyquinolin-4-yl)propyl)piperidine-4-carboxyiate (0.2091 g, 0.53 mmol) as the starting amine. To provide the title compound, ethyl 4-(3-(3-chloro-6-methoxyquinolin-4-yl)propyl)-1-(1-(pyridin-2-yl)azetidin-3-yl)piperidine-4-carboxylate (0.170 g, 0.325 mmol) was dissolved in MeOH (1.2 mL) and THF (1.2 mL). To this was added water (1.2 mL) and NaOH pellets (0.22 g, 5.5 mmol). The reaction was sealed and heated to 100° C for 4 hours before being allowed to cool to 25°C overnight. The reaction was then diluted with H₂O, the pH adjusted to ca. 4 with 1 N aq. HCl and extracted three times with EtOAc. The organic layers were then combined, dried with MgSO₄, filtered, and concentrated. The crude product was loaded onto silica gel and purified by chromatography using a CHCl₃: MeOH eluent system to provide the title compound as a white solid (0.020 g). LCMS: ret. time 1.26; MS+ 495.4.

### Example 127: (3R,4R)-4-(3-hydroxy-3-(6-methoxyquinolin-4-yl)propyl)-1-(1-(pyridine-2-ylmethyl)azetidin-3-yl)piperdine-3-carboxylic acid

Step 1: (3R,4R)-1-Azetidin-3-yl-4-[3-hydroxy-3-(6-methoxy-quinolin-4-yl}-propyl]-piperidine-3-carboxylic acid (2 HCl) (97mg, 0.2mmol) was combined with 2.5eq triethylamine (1eq) in THF (0.1M) and MeOH (0.4M) before the addition of 2-pyridyl carboxaldehyde (22ul, 0.22mmol), 4A molecular sieves and AcOH (1.5eq). The reaction stirred at 25°C for 1 h, after which MP-cyanoborohydride resin (1.2eq) was added and the mixture was allowed to stir overnight. The reaction was then diluted with DCM (double volume), filtered and the resin washed with additional DCM (5-10ml). The organics were then washed with sat. aq. NaHCO₃ (equal volume) and dried over Na₂SO₄, filtered and concentrated to dryness to yield 97mg of a foam (approx. 80% pure) that was used in the subsequent step without further purification. MS ESI+ m/z (M+H)⁺ 505.3

Step 2: To a solution of the product of Step 1 (1eq) in THF (0.05M) was added a freshly prepared solution of LiOH in H₂O (2.5eq in 0.2M). The reaction mixture is allowed to stir at 25°C until ester is consumed. Reaction acidified with 1 N aq. HCl (2.5-3.5eq.) and concentrated under reduced pressure. The residue dissolved in DMSO, (1ml/100mg), filtered and purified via reverse phase HPLC (gradient elution using 0-40%B where A: 0.1% formic acid in H₂O and B:0.1% formic acid in acetonitrile over 8 minutes) to yield the title compound (33 mg) as a formate salt. ¹H NMR (400 MHz, CD₃OD) *δ* (ppm) 1.57 (br. s., 1 H), 1.64 - 1.85 (m, 5 H), 1.85 - 2.09 (m, 2 H), 2.32 (br. s., 1 H), 2.47 (d, 1 H), 2.66 (s, 1 H), 2.74 (br. s., 2 H), 3.37 - 3.49 (m, 1 H), 3.84 (t, 1 H), 3.91 - 3.99 (m, 4 H), 4.12 (t, 2 H), 4.37 (s, 2 H), 5.38 (tt, 1 H), 7.36 - 7.45 (m, 4 H), 7.64 (dd, 1 H), 7.85 (t, 1 H), 7.91 - 7.97 (m, 1 H), 8.22 (s, 2 H), 8.57 (d, 1 H), 8.66 (d, 1 H) MS ES+ m/z (M+H)⁺ 491.3.

The following non-limiting Examples in Table 7 were prepared in a manner analogous to that described in Example 127 using the appropriate starting materials. Unless otherwise noted, LCMS data was acquired using standard conditions.

**TABLE 7**

| Ex.# | NAME | MS (M+1) | Ret. Time |
|---|---|---|---|
| 128 | (3R,4R)-4-[3-hydroxy-3-(6-methoxyquinolin-4-yl) propyl]-1-[1-(tetrahydro-2H-pyran-4-yl)azetidin-3-yl] piperidine-3-carboxylic acid | 484 | 0.3 |
| 129 | (3R,4R)-4-(3-hydroxy-3-(6-methoxyquinolin-4-yl)propyl]-1-[1-(tetrahydrofuran-3-yl)azetidin-3-yl] piperidine-3-carboxylic acid | 470 | 0.2 |
| 130 | (3R,4R)-1-(1-cyclobutylazetidin-3-yl)-4-[3-hydroxy-3-(6-methoxyquinolin-4-yl)propyl]piperidine-3-carboxylic acid | 468 | 2.0 |
| 131 | (3R,4R)-1-(1-cydohexylazetidin-3-yl)-4-[3-hydroxy-3-(6-methoxyquinolin-4-yl)propyl]piperidine-3-carboxylic acid | 482 | 1.9 |
| 132 | (3R,4R)-1-(1-cyclopentylazetidin-3-yl)-4-[3-hydroxy-3-(6-methoxyquinolin-4-yl)propyl]piperidine-3-carboxylic acid | 468 | 1.8 |

### Example 133: (3R,4R)-4-(3-fluoro-3-(6-methoxyquinolin-4-yl)propyl)-1-(3-phenylcyclobutyl)piperidine-3-carboxylic acid

Step 1: Example 3 (0.10 g, 0.211 mmol), anhydrous toluene (2.25 mL) and anhydrous MeOH (2.25 mL) were combined and cooled to 0 °C. To this was added TMS-diazomethane (2.0 M in ether, 0.32 mL, 0.64 mmol) drop-wise over 4 minutes. The resulting mixture was stirred for an additional 2 minutes at 0 °C before being allowed to warm to 25°C and stir for 1 h. The reaction mixture was concentrated to give 0.108 g of crude product which was used without purification. Ret. time: 1.27 min. MS+ 489.2

Step 2: A solution of the product of Step 1 (0.108 g, 0.221 mmol) in DCM (2.25 mL) was cooled to -78 ° C. DAST (0.045 mL, 0.34 mmol) was added and the reaction mixture warmed to 0 ° C and allowed to stir for 1 h under N₂. The reaction was diluted with H₂O and extracted with DCM. The organic layer was extracted with sat. aq. NaHCO₃ and brine, then dried over MgSO₄, filtered, and concentrated to an oil. The oil was loaded onto silica gel and purified by chromatography using a CHCl₃: MeOH eluent system to give 0.0588 g of a yellow oil. Ret. time: 1.69 min. MS+ 491.2

Step 3: To a solution of the product of Step 2 (0.0588 g, 0.12 mmol) in THF (1 mL), MeOH (1 mL), and H₂O (0.5 mL) was added LiOH (0.0150 g, 0.62 mmol) and the reaction mixture heated at 40 ° C overnight. The reaction was then diluted with H₂O and the pH adjusted to 6-7 range with addition of 1 N HCl solution. The mixture was concentrated to remove most of the organic solvents then extracted with DCM three times. The organic layers were combined, dried with MgSO₄, filtered, and concentrated to give an oil. The oil was loaded onto silica gel and purified by chromatography using a CHCl₃: MeOH eluent system to give the title compound (0.0339 g) as a glassy solid. Ret. time: 1.48 min. MS+ 477.3

**Example 134** was prepared in an analogous manner to that described in Example 133 using the title compound of Example 2 as the starting material. LCMS ret. time 1.48; MS+ 477.2.

### Example 135: (3R,4R)-4-[3-(6-Methoxy-quinolin-4-yl)-3-oxo-propyl]-1-(3-phenyl-cyclobutyl)-piperidine-3-carboxylic acid

The title compound of Example 1 (500mg, 1.05mmol) and Dess-Martin periodinane (601 mg, 1.42mmol) were combined in anhydrous DCM (25 mL). The reaction was stirred at 25°C for 90 minutes before being concentrated. chromatography (gradient elution from 1% to 25% MeOH in CHCl₃) afforded the title compound as an off-white solid (321.6mg).LCMS ret. time 1.78, M+1 473.

### Example 136: (3R,4R)-4-[3-(6-Methoxy-quinolin-4-yl)-3-methylaminopropyl]-1-(3-phenyl-cyclobutyl)-piperidine-3-carboxylic acid

Example 135 (50mg, 0.106mmol), methylamine (33% solution in EtOH, 68µL, 0.53mmol), MP-cyanoborohydride resin (2.43mmol/g, 57mg, 0.138mmol), and glacial AcOH (0.1 mL) were combined in THF (1.5 mL) and heated in a microwave at 100°C for 2 hours. The mixture was then diluted with DCM, filtered, concentrated onto silica gel and purified via chromatography (gradient elution from 1% MeOH in CHCl₃ to 100% MeOH). The product thus obtained was dissolved in MeOH (1 mL), the solution acidified to pH 3 with glacial AcOH, and loaded onto a cation exchange column, washing with MeOH and eluting with 0.25M NH₄OH in MeOH to afford the title compound as a white solid (27.0mg). LCMS ret. time 1.48, M+1 488.

Using the appropriate starting materials, the following non-limiting Examples (137-140) were prepared in a manner analogous to Example 136.

### Example 137: (3R,4R)-4-[3-Azetidin-1-yl-3-(6-methoxy-quinolin-4-yl)-propyl]-1-(3-phenylcyclobutyl)piperidine-3-carboxylic acid. LCMS ret. time 1.43, M+1 514.

### Example 138: (3R,4R)-4-[3-Amino-3-(6-methoxy-quinolin-4-yl)-propyl]-1-(3-phenyl-cyclobutyl)-piperidine-3-carboxylic acid. LCMS ret. time 1.22, M+1 474.

### Example 139: (3R,4R)-4-[3-(6-methoxyquinolin-4-yl)-3-morpholin-4-ylpropyl]-1-(3-phenylcyclobutyl)piperidine-3-carboxylic acid. LCMS ret. time 1.26, M+1 544.

### Example 140: (3R,4R)-4-[3-(dimethylamino)-3-(6-methoxyquinolin-4-yl)propyl]-1-(3-phenylcyclobutyl)piperidine-3-carboxylic acid. LCMS ret. time 1.30, M+1 502.

### Example 141: 4-(3-(3-chloro-6-methoxyquinolin-4-yl)-3-hydroxypropyl)-1-(3-phenylcyclobutyl)piperidine-4-carboxylic acid

Step 1: Pure oxygen was bubbled through a solution of 1-tert-butyl 4-ethyl 4-(3-(3-chloro-6-methoxyquinolin-4-yl)propyl)piperidine-1,4-dicarboxylate (0.5378 g, 1.1 mmol) in *t*-BuOH (12 mL) and DMSO (40 mL) for 5 minutes at 25°C before the addition of a solution of potassium t-butoxide (0.301 g, 2.68 mmol) in *t*-BuOH (3 mL). The reaction mixture was oxygenated for 1 h, whereupon additional potassium t-butoxide (2.45 eq.) was added. Oxygenation was continued for 90 minutes before the reaction was capped and allowed to stir overnight at 25°C. Ice-cold H₂O (60 mL) was then added, followed by AcOH (0.7 mL) and the solution was extracted with DCM (3 x 20 ml). The organic layers were then combined and extracted with H₂O (4 x 10 mL), dried over MgSO₄, filtered, and concentrated to a yellow oil. The oil was dissolved in EtOAc, extracted with H₂O (1 x 10 ml), dried over MgSO₄, filtered, and concentrated to afford 0.330 g of product as a yellow oil.

Step 2: The product of Step 1 (0.330 g, 0.69 mmol) was combined with HCl in dioxane (4 M, 5 mL, 20 mmol) and was allowed to stir at 25°C for 1 h under N₂ before being concentrated to dryness. The residue was then dissolved in MeOH and poured onto a cation exchange (MCX) column, washing first with MeOH and then eluting the product with 0.25 M NH₄OH in MeOH to afford the deprotected product (0.21 g).

Step 3: To a solution of the product of Step 2 (0.0502 g, 0.13 mmol) in DMF (1 mL) was added 3-phenylcyclobutanone (0.0415 g, 0.28 mmol), MP-cyanoborohydride resin (0.099 g of 2.55 mmol/g resin, 0.25 mmol), and glacial AcOH (0.07 mL). The reaction was capped and heated to 80° C for ten minutes in a microwave. The crude reaction mixture was then poured onto a cation exchange (MCX) column which was washed with MeOH followed by 0.25 M NH₄OH solution in MeOH. Concentration gave impure product that was loaded onto silica gel and purified by chromatography using a CHCl₃: MeOH solvent system to provide the title compound (0.019 g) as a solid. LCMS ret. time 1.96; MS+ 509.5.

### Example 142: (3R,4R)-4-[3-hydroxy-3-(6-hydroxyquinolin-4-yl)propyl]-1-(3-phenylcyclobutyl)piperidine-3-carboxylic acid

In each of four separate tubes was placed 100 mg of Example 3 and 6 mL of 48% HBr after which the tubes were sealed. Tube 1 was left at 25°C while tubes 2, 3 and 4 were heated at 60°C, 70°C, and 80°C, respectively, overnight. The contents of Tube 4 were discarded. Tubes 1, 2 and 3 were heated at 70°C for 3 days, then heated at 100°C overnight. Tubes 1, 2 and 3 were cooled to 25°C, combined, and the pH adjusted to ca. pH 7 using 6 N NaOH and 1 N HCl. The crude reaction mixture was then filtered, yielding 160 mg of a brown solid. Purification via HPLC (30 mm column; gradient elution using an acetonitrile (0.1% formic acid) water (0.15 formic acid) solvent system) afforded the title compound (6.2 mg) as a solid. LCMS: ret. time 1.0; (M+ 1) 461.

### Example 143: (3R,4R)-4-{3-[6-(difluoromethoxy)quinolin-4-yl]-3-hydroxypropyl}-1-(3-phenylcyclobutyl)piperidine-3-carboxylic acid

A solution of the title compound of Example 142 (170 mg, 0.37 mmol) in 2.5 mL of dioxane and 2.2 mL (2.2 mmol, 6 eq.) of 1 N NaOH was heated to 60 °C while bubbling chlorodifluoromethane through the reaction mixture for 2 hours. During this time, the pH was maintained at or above pH 10 with the periodic addition of 1 N NaOH. The reaction was then cooled to 25°C and the pH was adjusted to 7 using 1N HCl. The crude reaction mixture was then concentrated and chromatographed (gradient elution from 89/10/1 to 84/15/1 using CHCl₃ / MeOH/ NH₄OH) to afford the title compound (15 mg) as a solid. ¹⁹F NMR (CD₃OD): - 84.3 (dd); ¹H NMR (CD₃OD): 8.79 (d, 1H, J = 4.6 Hz), 8.06 (d, 1H, J = 9.1 Hz), 7.86 (s, 1H), 7.67 (d, 1H, J = 4.6 Hz), 7.57 (dd, 1 H), 7.20-7.32 (m, 4H), 7.17 (m, 1H), 7.06 (t, 1H, J = 73.8 Hz), 5.32 (m, 1H), 1.2-3.6 (m, 18H); MS (m/z): 511 (M⁺+1, 100).

### Example 144: (3R,4R)-4-[3-Hydroxy-3-(6-methoxy-quinolin-4-yl)-propyl]-1-(3-phenyl-cyclobutyl)-piperidine-3-carboxylic acid amide

The title compound of Example 1 (0.070g, 0.148mmol) was combined with bromotripyrrolidinophosphonium hexafluorophosphate (0.09g, 0.193mmol); hydroxybenzotriazole (0.026g, 0.193mmol) and triethylamine (62µL, 0.444mmol) in 2.5 mL DMF. To this was added NH₄Cl (0.032g, 0.592mmol) and the mixture was stirred at 25°C overnight. The reaction was then concentrated and the resulting residue dissolved in CHCl₃ with a couple drops of MeOH added for solubility. This solution was poured into H₂O, and the aqueous layer extracted with CHCl₃ (3X). The organic layers were combined, dried over MgSO₄, filtered and concentrated to dryness. The crude material was purified first by chromatography (gradient elution from 1% to 25% MeOH in CHCl₃). Material was then further purified by cation exchange (MCX) column washing with MeOH and eluting product with 0.25M NH₄OH in MeOH to afford the title compound as a white solid (0.027g). LCMS: ret. time 1.30, M+1 474.

### Example 145: (3R,4R)-4-[3-Hydroxy-3-(6-methoxy-quinolin-4-yl)-propyl]-1-(3-phenyl-cyclobutyl)-piperidine-3-carboxylic acid methylamide

Prepared according to the procedure of Example 144 using methyl amine hydrochloride. The title compound (0.025 g) was isolated as a white solid. LCMS: ret. time 1.48, M+1 488.

### Example 146: 4-(2-(3-fluoro-6-methoxyquinolin-4-yl)-2-hydroxyethoxy)-1-(3-phenylcyclobutyl)piperidine-4-carboxylic acid

Step 1: To a cooled (-78 °C) solution of diisopropylamine (0.27 mL) in THF (5 mL) was added n-BuLi (0.77 mL, 2.5M in hexanes) drop-wise by syringe over several minutes. The reaction was stirred for 25 min after which 3-fluoro-6-methoxyquinoline (0.34 g) in THF (1 mL) was added drop-wise by cannula. The reaction was stirred for 4h at -78 °C before 1-tert-butyl 4-methyl 4-(2-oxoethoxy)piperidine-1,4-dicarboxylate (0.48 g) in THF (1 mL) was added drop-wise by cannula. The resulting reaction mixture was stirred 5 min at -78 °C, then allowed to warm to ca. 0 °C before being quenched by the addition of 5 mL sat. aq. NH₄Cl. The reaction was diluted with EtOAc and phases separated. The aqueous layer was extracted with EtOAc, and the combined organic layers were dried over MgSO₄, filtered, and concentrated under reduced pressure to give a yellow solid. Purification by chromatography (gradient elution from 5% EtOAc in heptane to 100% EtOAc) afforded the product as an off-white solid (0.15 g).

Step 2: The product of Step 1 (150mg, 0.313mmol) and LiOH (38mg, 1.57mmol) were combined in THF (2 mL), MeOH (2 mL), and H₂O (1 mL) and heated at 40°C overnight, then concentrated to dryness. The residue was suspended in H₂O, pH adjusted to approximately pH 4 with 1N aq. HCl, and extracted with CHCl₃ (3 x). The organic extracts were combined, dried over MgSO₄, filtered and concentrated to afford the product as an off-white solid (141.1 mg).

Step 3: The product of Step 2 (141mg, 0.305mmol) and HCl (4M solution in dioxane, 2mL, 8mmol) were combined and stirred at 25°C for 2 h before being concentrated to dryness. The residue was dissolved in 2-3mLs of H₂O and the pH adjusted to approximately pH 7 by the addition of 1 N aq. NaOH. The mixture was again concentrated to dryness and the resulting solid triturated with 9:1 CHCl₃:MeOH and filtered through celite, washing with 9:1 CHCl₃:MeCH. The filtrate was discarded and the celite washed with 4:1 CHCl₃:MeOH followed by 1:1 CHCl₃:MeOH. The filtrate was concentrated to afford the product as a white solid (0.0838 g).

Step 4: To a solution of the product of Step 3 (0.0268 g, 0.074 mmol) in DMF (1 mL) was added 3-phenylcyclobutanone (0.026 g, 0.178 mmol), glacial AcOH (0.040 mL) and MP-cyanoborohydride resin (0.065 g, 2.55 mmol/g, 0.166 mmol). The reaction was heated in a microwave for 60 minutes at 60° C before being poured onto a cation exchange (MCX) column. The column was washed with MeOH followed by 0.25 M NH₄OH solution in MeOH. Concentration of the appropriate fractions provided impure material that was concentrated onto silica gel. Chromatography using a CHCl₃: MeOH eluent system afforded the title compound as a white solid (0.0112 g). Ret. time: 1.82 min. MS+ 495.1

### Examples 147 and 148: 4-[(R)-2-(3-Chloro-6-methoxy-quinolin-4-yl)-2-hydroxy-ethylamino]-1-(3-phenyl-cyclobutyl)-azepane-4-carboxylic acid

Step 1: 3-Chloro-6-methoxy-4-(R)-oxiranyl-quinoline (73mg, 0.311mmol) and 4-amino-1-(3-phenyl-cyclobutyl)-azepane-4-carboxylic acid methyl ester (94mg, 0.311 mmol) were combined in *t*-BuOH (0.2 mL) and heated at 85°C overnight. The reaction mixture was diluted with DCM and concentrated onto silica gel. chromatography (gradient elution from 1% to 10% MeOH in CHCl₃) afforded the product as a yellow solid (48.1 mg).

Step 2: The product of Step 1 (48.1mg. 0.089mmol) and LiOH (11mg. 0.447mmol) were combined in MeOH (1 mL), THF (1 mL), and H₂O (0.5 mL), and the reaction heated at 40°C overnight. The pH was adjusted to between pH 6-7 with 1 N aq. HCl, and the reaction mixture concentrated onto silica gel. chromatography (gradient elution from 0.5% MeOH in CHCl₃ to 100% MeOH) afforded the title compound as separated diastereomers of unknown configuration as translucent glasses. Each diastereomer was further purified on a cation exchange column, washing with MeOH and eluting with 0.25M NH₄OH in MeOH to provide the following:

### Example 147 (Diastereomer A) was first relative eluting product, 8.1 mg. LCMS: Ret. time: 1.43 min. MS+ 524.

Example 148 (Diastereomer B) was second relative eluting product, 9.3mg, LCMS: Ret. time: 1.39 min. MS+ 524.

### Examples 149 and 150 4-[(R)-2-(3-Fluoro-6-methoxy-quinolin-4-yl)-2-hydroxy-ethylamino]-1-(3-phenyl-cyclobutyl)-azepane-4-carboxylic acid

The title compounds were prepared in an analogous manner to that described above for Examples 147 and 148 to afford the following as translucent glasses.

Example 149 (Diastereomer A) was first relative eluting product, 5.9mg. LCMS: Ret. time: 1.30 min. MS+ 508.

Example 150 (Diastereomer B) was the subsequently eluting product, (10.8mg). LCMS: Ret. time: 1.17 min. MS+ 508.

Using the appropriate starting materials, the additional non-limiting Examples in Table 8 were prepared according to the following general procedure:
To a solution of methyl 4-{[2-(3-chloro-6-methoxyquinolin-4-yl)ethyl]amino}azepane-4-carboxylate (0.11 mmol) in 1.0 mL NMP was added 30 mg STAB. This mixture was added to a vial containing 0.10 mmole of aldehyde or ketone and the reaction shaken at 25°C for 16 h before the addition of 200 *µ*L of 5 N LiOH. The resulting mixture was shaken at 25°C overnight, neutralized with 1 mmol TFA and purified via RP HPLC using an Xterra 30 x 50 mm column (C8, 5 micron) and a H₂O-ACN-NH₄OH mobile phase to furnish the title compound.

Unless otherwise noted, the LCMS data was acquired using standard conditions.

**TABLE 8**

| Example | NAME | MS (M+1) | Ret. Time |
|---|---|---|---|
| 151 | 4-{[2-(3-chloro-6-methoxyquinolin-4-yl)ethyl]amino}-1-[(2-phenylcyclopropyl) ethyl]zepane-4-carboxylic acid | 508.2 | 1.69 |
| 152 | 4-{[2-(3-chloro-6-methoxyquinolin-4-yl)ethyl] mino}-1-(3-phenylcyclobutyl) azepane-4-carboxylic acid | 509.2 | 1.67 |
| 153 | 4-{[2-(3-chloro-6-methoxyquinolin-4-yl)ethyl] amino}-1-[3-(3-methylphenyl) cyclobutyl] azepane-4-carboxylic acid | 522.2 | 1.76 |
| 154 | 4-{[2-(3-chloro-6-methoxyquinolin-4-yl)ethyl] amino}-1-[3-(2,6-difluorophenyl) cyclobutyl] azepane-4-carboxylic acid | 544.2 | 1.69 |
| 155 | 4-{[2-(3-chloro-6-methoxyquinolin-4-yl)ethyl] amino}-1-[3-(3-fluorophenyl) cyclobutyl] azepane-4-carboxylic acid | 526.2 | 1.68 |
| 156 | 4-{[2-(3-chloro-6-methoxyquinolin-4-yl) ethyl] amino}-1-{[2-(2,5-difluorophenyl) cyclo propyl]methyl}azepane-4-carboxylic acid | 544.2 | 1.72 |
| 157 | 4-{[2-(3-chloro-6-methoxyquinolin-4-yl) ethyl] 4-{[2-(3-chloro-6-methoxyquinolin-4-yl) amino}-1-{3-[3-(trifluoromethyl) phenyl]cyclobutyl}azepane-4-carboxylic acid | 576.2 | 1.87 |
| 158 | 4-{[2-(3-chloro-6-methoxyquinolin-4-yl) ethyl] amino}-1-[(2-methyl-5,6,7,8-tetra hydro quinazolin-6-yl)methyl]azepane-4-carboxylic acid | 538.2 | 1.28 |

### Example 159: 4-{[(2R)-2-hydroxy-2-(6-methoxy-1,5-naphthyridin-4-yl)ethyl]amino}-1-(3-phenylcyclobutyl)azepane-4-carboxylic acid

4-((R)-2-hydroxy-2-(6-methoxy-1,5-naphthyridin-4-yl)ethylamino)azepane-4-carboxylic acid (0.11 g), 3-phenylcyclobutanone (0.061 g), and MP-cyanoborohydride (0.16 g, 2.43 mmol/g) were combined in DMF (0.8 mL) and AcOH (0.2 mL) and the mixture heated 15 minutes in a microwave at 100 ° C. The crude reaction mixture was then poured onto a cation exchange column (SCX) and eluted with MeOH followed by 0.25M NH₄OH in MeOH to give a yellow oil. Further purification by chromatography (gradient elution from 100% EtoAc to 100% EtOH, followed by elution with 100% MeOH) afforded two principal products, the title compound and Example 160 methyl 4-((R)-2-hydroxy-2-(6-methoxy-1,5-naphthyridin-4-yl)ethylamino)-1-(3-phenylcyclobutyl)azepane-4-carboxylate.

Example 160 (0.011 g) eluted first, presumably obtained via esterification of the acid during the ion-exchange chromatography. LCMS M+1 = 505.2, ret time = 2.5 min (polar elution)

Subsequent elution of Example 159 afforded 0.070 g of a yellow solid. LCMS M+1 = 491,2, ret time = 1.6 min (polar elution).

### Example 161: 1-[3-(2-fluorophenyl)cyclobutyl]-4-{[(2R)-2-hydroxy-2-(6-methoxy-1,5-naphthyridin-4-yl)ethyl]amino}azepane-4-carboxylic acid

Step 1: (R)-2-methoxy-8-(oxiran-2-yl)-1,5-naphthyridine (500 mg) and racemic 4-amino-1-BOC-azepine-4-carboxylic acid methyl ester (670 mg) were combined in 2 mL of t-BuOH and heated in a sealed tube at 80°C for 4 days. The reaction was concentrated to dryness and purified via preparatory HPLC; 5-55% CH₃CN: H₂0, 11 min on an Xterra 30 x 50 C18 column to afford 400 mg of product. LCMS (ESI): [M+H]⁺ 509.1 ret time 1.4-1.5 min.

Step 2: The product of Step 1 was combined with 4M HCl in dioxane (10 mL) at 25°C, whereupon a pink precipitate appeared and ca. 0.5 mL of water was added. The precipitate dissolved and the resulting solution was stirred at 25°C for 3 days. The reaction was concentrated and then dried under high vacuum to give a brown solid (380 mg). [M+H]⁺ 361.1878 Found 361.3.

Step 3: The product of Step 2 (67 mg) was combined with 3-(2-fluorophenyl)cyclobutanone, 5 equivalents of diisopropylethylamine, 4 Å molecular sieves in 4 mL of MeOH. The reaction was allowed to stir for 1 h after which of Na(OAc)₃BH (1.5 equiv) was added. The reaction was then allowed to stir overnight at 25°C. Purification via preparatory HPLC; 10-40% CH₃CN: H₂0 with 0.1% formic acid, retention time 3.4 min on an Xterra 30 x 50 C18 column afforded the title compound (12 mg) as a solid. MS (ESI): 1.1-1.4 min, [M+H]⁺ 509.2.

### Example 162: (3R,4R)-1-[3-(3-ethyl-1,2,4-oxadiazol-5-yl)cyclobutyl]-4-[3-hydroxy-3-(6-methoxyquinolin-4-yl)propyl]piperidine-3-carboxylic acid

Step 1: N,N-Diisopropylethylamine (0.178ml, 1 mmol) and TFFH (264mg, 1mmol) were added to 3-oxocyclobutanecarboxylic acid (114mg, 1mmol) in THF (10ml) and stirred at 25°C for 2 h, whereupon 1 mmol of *N*'-hydroxyproprionamidine was added. The mixture was then allowed to stir at 25°C overnight and was used in the subsequent step without isolation or purification.

Step 2: To 1.5ml of the crude reaction mixture of Step 1 (0.15mmol) was added (3R,4R)-4-[3-hydroxy-3-(6-methoxy-quinolin-4-y)-propyl]-piperidine-3-carboxylic acid (36mg, 0.1mmol) in MeOH (0.4M), 4Å molecular sieves and AcOH (2.5eq). The resulting reaction mixture stirred at 25°C for 1 hour before the addition of MP-cyanoborohydride resin (1.2eq). The reaction was allowed to stir overnight, whereupon it was diluted with DCM (double volume) and neutralized to ca. pH 7 via the addition of MP-carbonate. The reaction was then filtered and the resins washed with additional DCM (5-10ml). The organics were then washed with sat. aq. NaHCO₃ (equal volume), dried over Na₂SO₄, filtered and concentrated to dryness.

Step 3: To a solution of the crude material from Step 2 in DCM (6ml) was added resin bound fluorine (70mg, 2eq). The resulting mixture stirred overnight at 25°C, whereupon the resin was removed via filtration and the filtrate concentrated to dryness. The crude material thus obtained was taken up in DMSO, (1ml/100mg) and purified via prep HPLC (gradient elution of 5-45%B where A:0.1% TFA in H₂O and B:0.1% TFA in acetonitrile, over 8 mins) to furnish the TFA salt of the title compound as a mixture of diastereomers. The title compound had a retention time of 4.03-4.18 minutes from prep HPLC. LCMS: ret. time 0.87; MS ESI+ m/z (M+H)⁺ 495.3

Table 9 lists additional non-limiting Examples that were prepared in a manner analogous to that described in Example 162 using the appropriate starting materials. Unless otherwise noted, LCMS data was acquired using standard conditions.

**TABLE 9**

| Ex.# | Name | MS (M+1) | Ret Time |
|---|---|---|---|
| 163 | (3 R,4R)-1-[3-(3-cyclobutyl-1,2,4-oxad iazol-5-yl) cyclobutyl]-4-[3-hydroxy-3-(6-methoxyquinolin-4-yl) propyl]piperidine-3-carboxylic acid | 521.4 | 1.21 |
| 164 | (3R,4R)-1-[3-(3-tert-butyl-1,2,4-oxadiazol-5-yl) cyclobutyl]-4-[3-hydroxy-3-(6-methoxyquinolin-4-yl) propyl]piperidine-3-carboxylic acid | 523.4 | 1.26 |
| 165 | (3R,4R)-1-[3-(3-cyclopropyl-1,2,4-oxadiazol-5-yl) cyclobutyl]-4-[3-hydroxy-3-(6-methoxyquinolin-4-yl) propyl]piperidine-3-carboxylic acid | 507.3 | 1.02 |
| 166 | (3R,4R)-4-[3-hydroxy-3-(6-methoxyquinolin-4-yl) propyl]-1-[3-(3-isopropyl-1,2,4-oxadiazol-5-yl) cyclobutyl]piperidine-3-carboxylic acid | 509.3 | 1.04 |
| 167 | (3R,4R)-4-[3-hydroxy-3-(6-methoxyquinolin-4-yl) propyl]-1-[3-(3-propyl-1,2,4-oxadiazol-5-yl) cyclobutyl]piperidine-3-carboxylic acid | 509.4 | 1.03 |
| 168 | (3R,4R)-1-{3-[3-(2-ethoxyethyl)-1,2,4-oxadiazol-5-yl]cyclobutyl}-4-[3-hydroxy-3-(6-methoxyquinolin-4-yl)propyl]piperidine-3-carboxylic acid | 539.4 | .91 |
| 169 | (3R,4R)-4-[3-hydroxy-3-(6-methoxyquinolin-4-yl) propyl]-1-{3-[3-(methoxymethyl)-1,2,4-oxadiazol-5-yl]cyclobutyl}piperidine-3-carboxylic acid | 256.3^{†} | .86 |
| 170 | (3R,4R)-1-{3-[3-(cyclopropylmethyl)-1,2,4-oxadiazol -5-yl]cyclobutyl}-4-[3-hydroxy-3-(6-methoxyquinolin-4-yl)propyl]piperidine-3-carboxylic acid | 521.4 | 1.1 |

| | | | |
|---|---|---|---|
| ^{†} (M/2H+) | | | |

### Example 171: (3R,4R)-4-[3-Hydroxy-3-(6-methoxy-quinolin-4-yl)-propyl]-1-[3-(5-methyl-isoxazol-3-yl)-cyclobutyl]-piperidine-3-carboxylic add

Step 1: A solution of 3,3-dimethoxy-cyclobutanecarboxylic acid N-methoxy-N-methyl-amide (1.65g, 8.12mmol) in 60mL anhydrous THF was cooled to -78°C. To this was added 1-propynylmagnesium bromide (0.5M solution in THF, 4.92mL, 32.5mL, 16.24mmol). Upon completion of addition, the reaction was slowly allowed to warm to 25°C and stir overnight. The reaction was poured into 1 N aq. HCl, and extracted with EtOAc (3X). The organic extracts were combined, dried over MgSO₄, filtered and concentrated to afford a brown oil that was used without purification (1.35g).

Step 2: The product of Step 1 (250mg, 1.37mmol) and hydroxylamine hydrochloride (191 mg, 2.74mmol) were combined in 5mL EtOH. The mixture was heated in a microwave at 80°C for 60 minutes and then concentrated to dryness. The resulting residue was dissolved in DCM and washed with H₂O, then dried over MgSO₄, filtered, and concentrated onto silica gel. The material thus obtained was purified by chromatography (gradient elution from 5% EtOAc in heptane to 100% EtOAc) to afford the product (37.9mg).

Step 3: (3R,4R)-4-(3-hydroxy-3-(6-methoxyquinolin-4-yl)propyl)piperidine-3-carboxylic acid (0.138 g), the product of Step 2 (0.0379 g), and AcOH (0.029 mL) were combined in THF (2.6 mL) and MeOH (2 mL). The resulting solution was stirred for 5 h in the presence of a small quantity of 4Å molecular sieves before the addition of NaCNBH₃ (0.032 g). The reaction stirred at 25°C overnight and was then concentrated onto silica gel and purified by chromatography (gradient elution from 1% MeOH in CHCl₃ to 100% MeOH) to afford the product (0.0899 g).

Step 4: The product of Step 3 65mg, 0.126mmol) and N,N-diisopropylethylamine (0.066mL, 0.379mmol) were combined in 3.5mL THF. The mixture was heated in a microwave for three hours at 175°C. Additional THF (1 mL) and N,N-diisopropylethylamine (0.030mL) were added and the reaction heated for another 2 hours at 175°C. The solvent was removed by rotary evaporation and the residue partitioned between CHCl₃ and H₂O. The aqueous layer was extracted twice more with CHCl₃, and the combined organic extracts were dried over MgSO₄, filtered, and concentrated. The crude material was purified by chromatography (gradient elution from 1% to 35% MeOH in CHCl₃) to afford the title compound as a white solid (13.7mg). LCMS: ret time .85 min, [M+1] 480.

Table 10 provides additional non-limiting examples that were prepared according to one or more of the procedures described above using appropriate starting materials. Unless otherwise noted, the compounds were prepared as mixtures of diastereomers and LCMS data was acquired under standard conditions.

**TABLE 10**

| Ex.# | NAME | MS (M+1) | Ret Time |
|---|---|---|---|
| 172 | (3R,4R)-1-[3-(2-fluoro-5-methoxyphenyl)cyclobutyl]-4-[(3S)-3-hydroxy-3-(6-methoxyquinolin-4-yl)propyl] piperidine-3-carboxylic acid | 523.5 | 1.4 |
| 173 | 3-[3-(3-fluoro-6-methoxyquinolin-4-yl)propyl]-1-(3-phenylcyclobutyl)pyrrolidine-3-carboxylic acid | 463 | 1.87 |
| 174 | 1-[3-(2,6-difluorophenyl)cyclobutyl)-3-[3-(3-fluoro-6-methoxyquinolin-4-yl)propyl]pyrrolidine-3-carboxylic acid | 499 | 1.91 |
| 175 | 3-[3-(3-chloro-6-methoxyquinolin-4-yl)-3-hydroxy propyl]-1-(3-phenylcyclobutyl)pyrrolidine-3-carboxylic acid | 495.1 | 1.9 |
| 176 | 3-[3-(3-chloro-6-methoxyquinolin-4-yl)-3-hydroxypropyl]-1-[3-(2,6-difluorophenyl)cyclobutyl] pyrrolidine-3-carboxylic acid | 531 | 1.9 |
| 177 | (3R,4R)-4-[3-hydroxy-3-(6-methoxyquinolin-4-yl)propyl]-1-[(3-phenylcyclobutyl)methyl]piperidine-3-carboxylic acid | 489.1 | 1.65 |
| 177 | (3R,4R)-4-[3-hydroxy-3-(6-methoxyquinolin-4-yl) propyl]-1-[(3-phenylcyclobutyl)methyl]piperidine-3-carboxylic acid | 489.1 | 1.65 |
| 179 | (3R,4R)-1-(3-(2,6-difluorophenyl)cyclobutyl]-4-(3-hydroxy-3-(6-methoxy-2-methylquinolin-4-yl)propyl] piperidine-3-carboxylic acid | 525.3 | 1.4 |
| 180, 181^{†} | 1-[3-(2,6-difluorophenyl)cyclobutyl]-3-[3-(3-fluoro-6-methoxyquinolin-4-yl)propyl]pyrrolidine-3-carboxylic acid | 499 | 2.04 |
| 182 | (3R,4R)-1-[3-(2,6-difluorophenyl)cyclobutyl]-4-[3-(3-fluoro-6-methoxy-1,5-naphthyridin-4-yl)-3-hydroxy propyl]piperidine-3-carboxylic acid | 530 | 1.91 |
| 183 | (3R,4R)-1-[3-(2,5-difluorophenyl)cyclopentyl]-4-[3-hydroxy-3-(6-methoxyquinolin-4-yl)propyl]piperidine-3-carboxylic acid | 525 | 1.52 |
| 184 | 3-[3-(3-chloro-6-methoxyquinolin-4-yl)-3-hydroxypropyl]-1-[3-(2,5-difluorophenyl)cyclobutyl] pyrrolidine-3-carboxylic acid | 531 | 1.9 |
| 185 | (3R,4R)-4-[3-hydroxy-3-(6-methoxyquinolin-4-yl) propyl]-1-(1-phenylpyrrolidin-3-yl)piperidine-3-carboxylic acid | 490 | 1.35 |
| 186 | (3R,4R)-4-[3-(3-chloro-6-methoxyquinolin-4-yl)-3-hydroxypropyl]-1-[3-(2,5-difluorophenyl)cyclobutyl] piperidine-3-carboxylic acid | 545.2 | 1.95 |
| 187 | (3R,4R)-4-[3-(3-chloro-6-methoxyquinolin-4-yl)-3-hydroxypropyl]-1-(3-phenylcyclobutyl)piperidine-3-carboxylic acid | 509.2 | 1.91 |
| 188 | (3R,4R)-4-[3-(3-chloro-6-methoxyquinolin-4-yl)-3-hydroxypropyl]-1-[3-(2,6-difluorophenyl)cyclobutyl] piperidine-3-carboxylic acid | 545.2 | 1.95 |
| 189, 190^{†} | (3R,4R)-1-[3-(2,6-difluorophenyl)cyclobutyl]-4-(3-(3-fluoro-6-methoxy-1,5-naphthyridin-4-yl)-3-hydroxy propyl]piperidine-3-carboxylic acid | 530 | 1.78 |
| 191 | (3R,4R)-4-[(3S)-3-hydroxy-3-(6-methoxyquinolin-4-yl)propyl]-1-[3-(2,4,5-trifluorophenyl)cyclobutyl] piperidine-3-carboxylic acid | 529.1 | 1.5 |
| 192 | (3R,4R)-1-[3-(3-fluorobenzyl)cyclobutyl]-4-[3-hydroxy -3-(6-methoxyquinolin-4-yl)propyl]piperidine-3-carboxylic acid | 507.2 | 1.52 |
| 193 | (3R,4R)-1-{3-[2-fluoro-3-(trifluoromethyl)phenyl] cyclobutyl}-4-[(3S)-3-hydroxy-3-(6-methoxyquinolin-4-yl)propyllpiperidine-3-carboxylic acid | 561.1 | 2.0 |
| 194 | (3R,4R)-1-{3-[2-fluoro-4-(trifluoromethyl)phenyl] cyclobutyl}-4-((3S}-3-hydroxy-3-(6-methoxyquinolin-4-yl)propyl]piperidine-3-carboxylic acid | 561.1 | 1.8 |
| 195 | (3R,4R)-4-[(3S)-3-hydroxy-3-(6-methoxyquinolin-4-yl)propyl]-1-[3-(2,3,6-trifluorophenyl)cyclobutyl] piperidine-3-carboxylic acid | 529.2 | 1.4 |
| 196 | (3R,4R)-1-[3-(4-bromo-2-fluorophenyl)cyclobutyl]-4-[(3S)-3-hydroxy-3-(6-methoxyquinolin-4-yl)propyl] piperidine-3-carboxylic acid | 573.0 | 1.7 |
| 197 | (3R,4R)-4-[(3S)-3-hydroxy-3-(6-methoxyquinolin-4-yl)propyl]-1-[3-(2,3,4-trifluorophenyl)cyclobutyl] piperidine-3-carboxylic acid | 529.1 | 1.5 |
| 198 | 3-[3-(3-chloro-6-methoxyquinolin-4-yl)propyl]-1-{[(1R, 2R)-2-(2,5-difluorophenyl)cyclopropyl]methyl} pyrrolidine-3-carboxylic acid | 515 | 1.95 |
| 199 | 3-[3-(3-chloro-6-methoxyquinolin-4-yl)propyl]-1-[3-(2,5-difluorophenyl)cyclopentyl]pyrrolidine-3-carboxylic acid | 529 | 2.04 |
| 200 | (3R,4R)-1-[3-(2,6-difluorophenyl)-2,2-dimethylcyclo butyl]-4-[(3S)-3-hydroxy-3-(6-methoxyquinolin-4-yl) propyl]piperidine-3-carboxylic acid | 539.3 | 1.6 |
| 201 | (3R,4R)-1-[3-(2,6-difluorophenyl)cyclobutyl]-4-[3-(3-fluoro-6-hydroxy-1,5-naphthyridin-4-yl)-3-hydroxy propyl]piperidine-3-carboxylic acid | 516 | 1.48 |
| 202 | (3R,4R)-1-[3-(2,5-difluorobenzyl)cyclobutyl]-4-[3-hydroxy-3-(6-methoxyquinolin-4-yl)propyl]piperidine-3-carboxylic acid | 525.2 | 1.59 |
| 203, 204^{†} | (3R,4R)-4-(3-(3-chloro-6-methoxyquinolin-4-yl)-3-hydroxypropyl]-1-[3-(2,5-difluorophenyl)cyclobutyl] piperidine-3-carboxylic acid | 545.4 | 2.04 |
| 205 | (3R,4R)-1-[3-(2,5-difluorophenyl)cyclobutyl]-4-[3-(3-fluoro-6-methoxy-1,5-naphthyridin-4-yl)-3-hydroxypropyl]piperidine-3-carboxylic acid | 530 | 1.87 |
| 206 | (3R,4R)-1-[3-(2,5-difluorophenyl)cyclobutyl]-4-[3-(3-fluoro-6-hydroxy-1,5-naphthyridin-4-yl)-3-hydroxypropyl]piperidine-3-carboxylic acid | 516 | 1.48 |
| 207 | 3-[3-(3-chloro-6-methoxyquinolin-4-yl)propyl]-1-[3-(2,5-difluorophenyl)cyclobutyl] pyrrolidine-3-carboxylic acid | 515 | 2.17 |
| 208 | (3R,4R)-1-[3-(2-fluoro-3-methoxyphenyl) cyclobutyl]-4-[(3S)-3-hydroxy-3-(6-methoxyquinolin-4-yl)propyl] piperidine-3-carboxylic acid | 523.2 | 1.4-1.5 |
| 209, 210^{†} | (3R,4R)-4-[3-(3chloro-6-methoxyquinolin-4-yl)-3-hydroxypropyl]-1-[3-(2,5-difluorophenyl)cyclobutyl] piperidine-3-carboxylic acid | 545.4 | 1.95 |
| 211, 212^{†} | (3R,4R)-4-[3-(3-chloro-6-methoxyquinolin-4-yl)-3-hydroxypropyl]-1-(3-phenylcyclobutyl)piperidine-3-carboxylic acid | 509.4 | 1.91 |
| 213, 214^{†} | (3R,4R)-4-[3-(3-chloro-6-methoxyquinolin-4-yl)-3-hydroxypropyl]-1-[3-(2,6-difluorophenyl)cyclobutyl) piperidine-3-carboxylic acid | 545.4 | 1.96 |
| 215 | (3R,4R)-1-[trans-3-(2,6-difluorophenyl)cyclobutyl]-4-[3-(3-fluoro-6-methoxyquinolin-4-yl)-3-hydroxypropyl] piperidine-3-carboxylic acid | 529 | 1.78 |
| 216 | (3R,4R)-1-[3-(3-cyano-4-fluorophenyl)cyclobutyl]-4-[(3S)-3-hydroxy-3-(6-methoxyquinolin-4-yl)propyl] piperidine-3-carboxylic acid | 518.2 | 1.2 |
| 217 | (3R,4R)-1-[3-(2,5-difluorophenyl)cyclobutyl]-4-[3-(3-fluoro-6-methoxy-1,5-naphthyridin-4-yl)-3-hydroxy propyl]piperidine-3-carboxylic acid | 530 | 1.82 |
| 218 | (3R,4R)-1-[3-(2,5-difluorophenyl)cyclobutyl]-4-[3-(3-fluoro-6-methoxy-1,5-naphthyridin-4-yl)-3-hydroxy propyl]piperidine-3-carboxylic acid | 530 | 1.87 |
| 219 | (3R,4R)-1-[3-(2,5-difluorophenyl)cyclobutyl]-4-[3-(3-fluoro-6-methoxy-1,5-naphthyridin-4-yl)-3-hydroxy propyl]piperidine-3-carboxylic acid | 511 | 1.5 |
| 220 | (3R,4R)-1-[3-(2-fluoro-4-methoxyphenyl)cyclobutyl]-4-[(3S)-3-hydroxy-3-(6-methoxyquinolin-4-yl)propyl] piperidine-3-carboxylic acid | 523.2 | 1.5 |
| 221 | (3R,4R)-1-[3-(3,5-difluorophenyl)cyclobutyl]-4-[(3S)-3-(3-fluoro-6-methoxyquinolin-4-yl)-3-hydroxypropyl] piperidine-3-carboxylic acid | 529 | 2.0 |
| 222 | (3R,4R)-1-[3-(3,5-difluorophenyl)cyclobutyl]-4-[3-(3-fluoro-6-methoxy-1,5-naphthyridin-4-yl)-3-hydroxy propyl]piperidine-3-carboxylic acid | 530 | 1.9 |
| 223 | (3R,4R)-1-[3-(3-cyanophenyl)cyclobutyl]-4-[(3S)-3-hydroxy-3-(6-methoxyquinolin-4-yl)propyl]piperidine-3-carboxylic acid | 500.2 | 1.2 |

| | | | |
|---|---|---|---|
| † prepared as a mixture and subsequently separated via chiral chromatography. | | | |

### Example 224: (S)-1-(6-methoxyquinolin-4-yl)-3-((3R,4R)-1-(3-phenylcyclobutyl)-3-(2H-tetrazol-5-yl)piperidin-4-yl)propan-1-ol

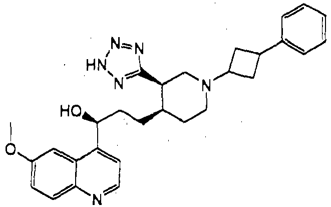

Step 1. A solution of (3R,4R)-4-((S)-3-hydroxy-3-(6-methoxyquinolin-4-yl)propyl)-1-(3-phenylcyclobutyl)piperidine-3-carboxylic acid (674.0 mg, 1.42 mmol) in acetonitrile (16.0 mL) was treated with *t*-butoxycarbonyl anhydride (403.0 mg, 1.85 mmol) in acetonitrile (2.0 mL), ammonium bicarbonate (135.0 mg, 1.70 mmol), followed by drop-wise addition of pyridine (0.069 mL, 0.85 mmol) at 25°C under N₂. The reaction flask was capped but allowed to vent. The reaction mixture was stirred for 17 hours and added treated with H₂O (0.5 mL). The solvent was removed under reduced pressure, and the resultant residue was washed with H₂O (3 x 0.25 mL) and concentrated under reduced pressure. The resultant pale yellow sticky oil was purified on 40 g ISCO silica gel with a gradient elution of MeOH/CH₂Cl₂ (0-20% in 50 min) to provide (3R,4R)-4-((S)-3-hydroxy-3-(6-methoxyquinolin-4-yl)propyl)-1-(3-phenylcyclobutyl)piperidine-3-carboxamide as light yellow sticky solid. Yield: 166.0 mg, 24.7%. LCMS (EI): 1.3 min, Exact Mass calcd for C₂₉H₃₅N₃O₃ [M+H]⁺, 474.268. Found 474.3

Step 2. A solution of the product of Step 1 (76.4 mg, 0.16 mmol) in anhydrous CH₂Cl₂ (3.0 mL) was treated with ethyl (carboxysulfamoyl)triethyl ammonium hydroxide inner salt (49.8 mg, 0.21 mmol) in 3 portions over 30 minutes and stirred for additional 5 minutes. The mixture was treated with water (5.0 mL) and the organic phase was collected. The aqueous phase was extracted with dichloromethane (2 x 30 mL), and the combined organic phases were dried over MgSO₄, filtered, and concentrated. The resultant reside was purified on 40 g silica gel with a gradient MeOH/CH₂Cl₂ (0-10 in 40 min) to provide (3R,4R)-4-((S)-3-hydroxy-3-(6-methoxyquinolin-4-yl)propyl)-1-(3-phenylcyclobutyl)piperidine-3-carbonitrile as white solid. Yield: 49.0 mg, 67.0%. ¹H NMR (400 MHz, CHLOROFORM-*d*) δ ppm 1.46 (br. s., 1 H) 1.54 - 1.76 (m, 5 H) 1.81 - 1.97 (m, 5 H) 2.44 - 2.46 (m, 2 H) 2.69 (m, 1 H) 2.80 (d, *J*=2.49 Hz, 1 H) 2.87 (d, *J*=12.05 Hz, 1 H) 2.95 - 3.15 (m, 2 H) 3.91 (s, 3 H) 5.28 (m, 1 H) 7.10 - 7.33 (m, 7 H) 7.48 (d, *J*=4.57 Hz, 1 H) 7.94 (d, *J*=9.14 Hz, 1 H) 8.59 (d, *J*=4.57 Hz, 1 H) LCMS (EI): 1.4 min, Exact Mass calcd for C₂₉H₃₃N₃O₂ [M+H]⁺, 456.257. Found 456.3

Step 3. A mixture of the product of Step 2 (30.0 mg, 0.066 mmol) in isopropanol:H₂O (1:2, 6.0 mL) and CH₂Cl₂ (2.0 mL) was treated with sodium azide (85.8 mg, 1.320 mmol) followed by ZnBr₂ (149.0 mg, 0.660 mmol) at 25°C under nitrogen atmosphere. The solution was refluxed for 2 days, cooled to 25°C, and the pH was adjusted to 6-7 by the addition of 5 N HCl. The solvent was evaporated, and the resultant residue was purified on a Shimadzu PR HPLC on Waters XTerra 19 x 50 C8 (gradient ACN (0.1% HCO₂H)/H₂O (0.1 % HCO₂H), 5-40%) in 10 min to provide the title compound as white solid. Yield: 17.0 mg, 51.7%. ¹H NMR (400 MHz, METHANOL-*d*₄) *δ* ppm 1.37 (m, 2 H) 1.71 - 1.85 (m, 3 H) 2.07 - 2.24 (m, 3 H) 2.53 (m, 3 H) 2.82 (m, 3 H) 3.65 (m, 4 H) 3.93 (s, 3 H) 5.31 (br. s., 1 H) 7.20 - 7.34 (m, 7 H) 7.59 (br. s., 1 H) 7.91 (d, *J*=9.14 Hz, 1 H) 8.68 (d, *J*=4.57 Hz, 1 H) LCMS (EI): 1.3 min, Exact Mass calcd for C₂₉H₃₄N₆O₂ [M+H]⁺, 499.274. Found 499.3

### Example 225: 3R,4R)-1-(1,7b-dihydrobenzo[b]cyclobuta[d]-thiophene-2(2aH)-yl)-4-((S)-3-hydroxy-3-(6-methoxyquinolin-4-yl)propyl)piperidine-3-carboxylic acid

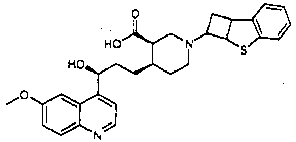

Step 1, A 25mL flame-dried flask was charged with a solution of sodium (3R,4R)-4-((S)-3-hydroxy-3-(6-methoxyquinolin-4-yl)propyl)piperidine-3-carboxylate (155 mg, 0.423 mmol) 1.3 equivalents of 1,7b-dihydrobenzo[b]cyclobuta[d]thiophene-2(2aH)-one (0.549 mmol), and a catalytic amount of 4Å molecular sieves (c.a. 25 mg) in THF (4 mL) and CH₃OH (1 mL) was stirred at 25°C for 2 hours. Sodium cyanoborohydride (29.2 mg, 0.465 mmol) was added, and the reaction mixture was stirred for 18 hours. Ethanol amine (5 equivalents, 0.13 mL, 2.125 mmol) was added, and the mixture was stirred at 25°C for 2.5 hours. The mixture was filtered through a pad of Celite, washed with CH₃OH (2 × 10 mL), and diluted with 50 mL CH₂Cl₂. The mixture was then treated with 10 mL of NaOH/KH₂PO4 buffer solution and the phases separated. The organic fraction was collected, and the aqueous fraction was extracted with CH₂Cl₂ (2 x 25 mL). The combined organic phases were washed with brine (2 x 15 mL), dried over MgSO₄, and concentrated under reduced pressure. The resultant residue was purified via preparative HPLC (5-55% CH₃CN: H₂0, 15 min on an Xterra 30 x 50 C18 column) to provide the title compound as a > 95:5 mixture of alcohol diasteromers (major illustrated) and an unassigned 10:1 mixture of diasteromers on the cyclobutane ring as a yellow foam. Yield: 6.9 mg, 4.0%. ¹H NMR (400 MHz, METHANOL-*d*₄) *δ* ppm 1.58 - 1.82 (m, 6 H) 2.12 - 2.20 (m, 1 H) 2.21 - 2.40 (m, 1 H) 2.62 (m, 1 H) 2.70 - 2.86 (m, 3 H) 3.18 (m, 1 H) 3.76 - 3.80 (m, 1 H) 3.81 - 3.90 (m, 1 H) 3.95 - 3.97 (m, 3 H) 4.03 - 4.10 (m, 1 H) 4.70 (m, 1 H) 5,34 (m, 1H) 7.10 - 7.20 (m, 1 H) 7.22 - 7.33 (m, 3 H) 7.33 - 7.45 (m, 2 H) 7.63 (d, *J*=4.57 Hz, 1 H) 7.91 (m, 1 H) 8.63 (d, *J*=4.15 Hz, 1 H). LCMS (EI): 1.0 min, Exact mass calcd for C₂₉H₃₃N₂O₄S [M+H]⁺, 505.2161. Found 505.3

### Example 226: (3R,4R)-1-(3-(3-cyanophenyl)cyclobutyl)-4-((S)-3-hydroxy-3-(6-methoxyquinolin-4-yl)propyl)piperidine-3-carboxylic acid

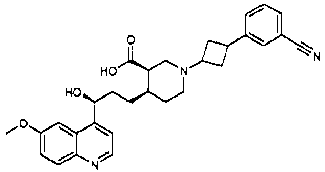

The title compound was prepared following the method of Example 226 and purified via preparative HPLC (HPLC; 5-50% CH₃CN: H₂0, 10 min on an Xterra 30 x 50 C18 column) to provide Example 227 as a > 95:5 mixture of alcohol diasteromers (major illustrated) and an undetermined mixture of cis /trans isomers on the cyclobutane ring as a white solid foam. Yield: 60.4 mg, 73%. ¹H NMR (400 MHz, METHANOL-*d*₄) δ ppm 1.60 - 1.80 (m, 4 H) 1,81 - 2.10 (m, 3H) 2.11 - 2.20 (m, 1H) 2.21 - 2.30 (m, 1 H) 2.40 - 2.60 (m, 1 H) 2.70 - 2.80 (m, 3H) 2.81 - 3.10 (m, 2H) 3.30 - 3.50 (m, 2 H) 3.56 - 3.72 (m, 1 H) 3.96 (s, 3 H) 5.38 (m, 1 H) 7.37 - 7.44 (m, 2 H) 7.49 (m, 1 H) 7.57 (d*, J*=7.48 Hz, 2 H) 7.62 - 7.70 (m, 2 H) 7.92 (d, *J*=9.97 Hz, 1 H) 8.65 (d, *J*=4.98 Hz, 1 H). LCMS (EI): 1.2 min, Exact mass calcd for C₃₀H₃₄N₃O₄ [M+H]⁺, 500.2549. Found 500.2

### Example 227: 3R,4R)-4-((S)-3-(3-fluoro-6-methoxyquinolin-4-yl)-3-hydroxypropyl)-1-(5-oxo-1-phenylpyrrolidin-3-yl)piperidine-3-carboxylic acid

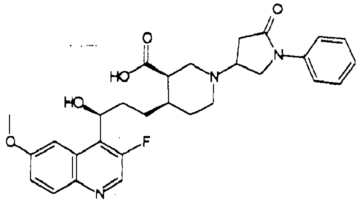

A mixture of (3R,4R)-4-((S)-3-(3-fluoro-6-methoxyquinolin-4-yl)-3-hydroxypropyl)piperidine-3-carboxylic acid (51.5mg, 0.142mmol, 1.0eq.), 1-phenylpyrrolidine-2,4-dione (50.0mg, 0.285mmol, 2.0eq.) and acetic acid (16.3µL, 0.285mmol, 2.0eq) in 2mL anhydrous tetrahydrofuran and 1mL anhydrous methanol was treated with a small spatula of 4Å molecular sieves. The mixture was then stirred at 25°C for 2.5 hours. Solid sodium cyanoborohydride (17.9mg, 0.285mmol, 2.0eq.) was added, and the reaction was stirred for 18 hours. The reaction mixture was then diluted with about 3mL of water, and the pH was adjusted to approximately neutral with a solution of 1N aqueous sodium hydroxide. The organic layer was collected, and the aqueous layer was extracted three times with chloroform. The combined organic extracts were dried over magnesium sulfate, filtered, and concentrated onto silica gel. The resultant residue was purified twice by silica gel column chromatography. The first purification used chloroform/methanol (1-50% gradient elution) and the second purification used chloroform/methanol (5-10% gradient elution) to provide the title compound as a tan solid. Yield: 17.9mg. The purity of the compound (¹HNMR) was 80%. M+1 = 522. Retention time = 1.52 min

### Example 228: (3R,4R)-1-(3-(2,5-difuorophenyl)cyclobutyl)-4-((S)-3-hydroxy-3-(6-methoxyquinolin-4-yl)propyl)-N-(methylsulfonyl)piperidine-3-carboxamide.

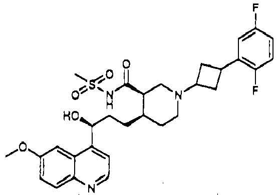

A 25 mL oven-dried round bottom flask was charged with cinchona acid (184 mg), (3R,4R)-1-[3-(2,5-difluorophenyl)cyclobutyl]-4-[(3S)-3-hydroxy-3-(6-methoxyquinolin-4-yl)propyl]piperidine-3-carboxylic acid (title compound of Example 40) (0.360 mmol), 0.85 mL EDCI (0.473 mmol), DMAP (11.2 mg, 0.091 mmol) an CH₂Cl₂ (4.0 mL). The reaction mixture was stirred for 15- 20 minutes and treated with methane sulfonamide (124 mg, 1.27 mmol). The mixture was stirred for 18 hours at 25°C, quenched with water (0.4 mL), and concentrated. The resultant residue was purified via preparatory HPLC (5-50% CH₃CN: H₂0, 10 min on an Xterra 30 x 50 C18 column) to provide the title compound (66 mg) as a white solid containing 10-15% impurity by ¹H and ¹⁹F NMR. This crude product was further purified on a 20 x 20, 2000 micron preparatory thin layer chromatography (tlc) plate and chromatographed eluting with 10% CH₃OH / CH₂Cl₂. The fraction containing product was scraped, filtered, washed with 10% CH₃OH (100 mL), and concentrated to provide the title compound as a > 95:5 mixture of alcohol diasteromers (major illustrated) and an undetermined mixture of cis / trans isomers on the cyclobutane ring as a white solid. Yield: 50.3 mg, 24%. ¹H NMR (400 MHz, METHANOL-d₄) δ ppm 1.67 (br. m., 3 H) 1.79 (m, 2 H) 1.94 (m, 1 H) 2.14 - 2.37 (m, 2 H) 2.45 (m, 1 H) 2.65 - 2.78 (m, 5 H) 2.98 (br. s., 3 H) 3.38 - 3.60 (m, 4 H) 3.99 (s, 3 H) 5.37 (m, 1 H) 6.89 - 6.98 (m, 1 H) 7.02 (m, 1 H) 7.16 (br. m., 1 H) 7.37 (dd, *J*=9.14, 2.49 Hz, 1 H) 7.44 (br. m., 1 H) 7.64 (d, *J*=4.57 Hz, 1 H) 7.89 (d, *J*=9.14 Hz, 1 H) 8.63 (d, *J*=4.57 Hz, 1 H). LCMS (EI): 1.4 min, Exact mass calcd for C₃₀H₃₆F₂N₃O₅S [M+H]⁺, 588.2344. Found 588.3

### Example 229: (3R,4R)-1-[3-(2,5-Difluoro-phenylsulfanyl)-cyclobutyl]-4-[(S)-3-(3-fluoro-6-methoxy-quinolin-4-yl)-3-hydroxy-propyl]-piperidine-3-carboxylic acid

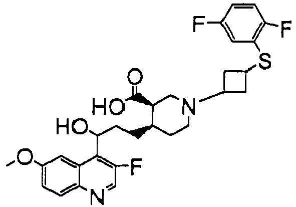

Step 1. Preparation of 3-(2,5-difluorophenylthio)cyclobutanone **(FF).** Compound **FF** was synthesized according to the procedure described below and depicted in Scheme 9.

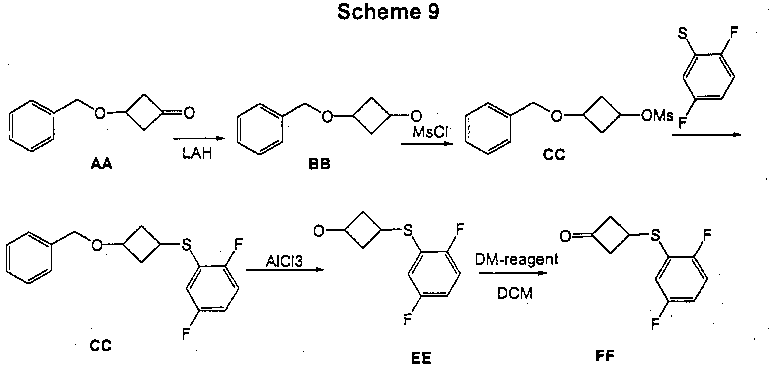

Preparation of 3-(benzyloxy)cyclobutanol **(BB):** A solution of 3-(benzyloxy)cyclobutanone **(AA)** (2 g, 11.34 m mol) in THF (40 ml) at 0 ºC was added drop-wise to a stirred suspension of LAH (474 mg, 12.48 m mol) in THF (40 ml) and stirred at 25°C for 2 hours. The reaction mixture was quenched with water and filtered through a bed of celite. The filtrate was then concentrated to provide **BB** (1.6 g, 79 %). ¹H NMR (400 MHz, CDCl₃): δ 7.35-7.25 (m, 5H), 4.40 (s, 2H), 3.90 (m, 1 H), 3.61 (m, 1 H), 2.74-2.67 (m, 2H), 1.95-1.89 (m, 2H).

Preparation of 3-(benzyloxy)cyclobutyl sulfochloridate **(CC):** A solution of compound **BB** (1.7 g, 9.53 m mol) in DCM (100 ml) was treated with triethylamine (3.34 ml, 23.84 m mol) followed by MeSO₂Cl (MsCl) (1.47 ml, 19.07 m mol) and stirred at 25°C for 30 minutes. The reaction mixture was poured into water and extracted with DCM. The organic layer was dried over Na₂SO₄, filtered, and concentrated to provide crude **CC.** Yield: 3.5 g. ¹H NMR (400 MHz, CDCl₃): δ 7.36-7.27 (m, 5H), 4.67-4.60 (m, 1H), 4.42 (s, 2H), 3.76-3.69 (m, 1 H), 2.97 (s, 3H), 2.85-2.78 (m, 2H), 2.35-2.28 (m, 2H).

Preparation of (3-(benzyloxy)cyclobutyl)(2,5-difluorophenyl)sulfane **(DD):** A solution of compound **CC** (3.5 g, 13.65 m mol), 2,5-difluorophenol (1.99 g, 13.65 m mol) and Cs₂CO₃ (6.67 g, 20.48 m mol) in DMF (100 ml) was heated at 100 ºC for 12 hours. The reaction mixture was diluted with water and extracted with EtOAc. The organic fraction was dried over Na₂SO₄ and concentrated. The resultant residue was then purified by 230-400-mesh column using pentane as eluting solvent to provide **DD.** Yield: 500 mg, 12 %. ¹H NMR (400 MHz, CDCl₃): δ 7.36-7.24 (m, 5H), 7.00-6.94 (m, 1H), 6.84-6.79 (m, 2H), 4.41 (s, 2H), 4.39-4.34 (m, 1H), 3.90-3.83 (m, 1 H), 2.61-2.54 (m, 2H), 2.34-2.30 (m, 2H),

Preparation of 3-(2,5-difluorophenylthio)cyclobutanol **(EE):** A solution of compound **DD** (500 mg, 1.63 m mol) in DCM (20 ml) was treated with N,N-dimethyl aniline (2.37 mg, 19.60 m mol) and AlCl₃ (2.17 g, 16.33 m mol) and stirred at 25°C for 3 hours. The reaction mixture was then quenched with 1 N HCl, and the organic phase was collected. The aqueous layer was extracted with EtOAc, and the combined organic phases were washed with 5% NaHCO₃ solution and brine solution. The organic phase was dried over Na₂SO₄ and concentrated. The resultant residue was then purified by column chromatography (using 100-200 mesh silica gel, 15 % EtOAc in hexane) to provide **EE.** Yield: 260 mg, 73 %. ¹H NMR (400 MHz, CDCl₃): 8 7.00-6.90 (m, 1 H), 6.84-6.80 (m, 2H), 4.69-4.61 (m, 1 H), 3.88-3.82 (m, 1 H), 2.52-2.35 (m, 4H).

Preparation of Compound **FF:** A stirred solution of **EE** (260 mg, 1.2 m mol) in DCM (20 ml) was treated with Dess Martin reagent (562 mg, 1.32 m mol) and stirred for 2 hours at 25°C. The reaction mixture was poured into 1 N NaOH solution and extracted with DCM. The organic layer was further washed with 1 N NaOH solution and concentrated to provide **FF.** Yield: 196 mg, 76 %. ¹H NMR (400 MHz, CDCl₃): δ 7.06-6.88 (m, 3H), 4.03-3.96 (m, 1H), 3.61-3.53 (m, 2H), 3.14-3.06 (m, 2H).

Step 2. Preparation of (3R,4R)-1-[3-(2,5-Difluoro-phenylsulfanyl)-cyclobutyl]-4-[(S)-3-(3-fluoro-6-methoxy-quinolin-4-yl)-3-hydroxy-propyl]-piperidine-3-carboxylic acid.

Activated 4 Ǻ molecular sieves were added to an oven dried flask followed. The flask was then charged with a solution of (3R,4R)-4-[(S)-3-(3-Fluoro-6-methoxy-quinolin-4-yl)-3-hydroxy-propyl]-piperidine-3-carboxylic acid (50mg, 0.14mmol) in a mixture of tetrahydrofuran: methanol (4mL:2mL). Compound **FF** (29.6mg, 0.138mmol) and acetic acid (0.016ml, 0.276mmol) were added, and the reaction mixture was stirred under nitrogen atmosphere at 25°C for 3 hours. Sodium cyanoborohydride (9.6mg, 0.15mmol) in 0.5mL of tetrahydrofuran was added, and the reaction mixture was stirred for 16 hours. The mixture was then filtered through celite, washed with methanol, and concentrated. The resultant residue was purified by reverse phase HPLC (Shimadzu 30X50mm Xterra column, 0.1% trifluoroacetic acid modified 15-65%acetonitrile in water. 10min gradient) and the eluents containing produce were collected and concentrated to provide the trifluoroacetate salt of the title compound as a white solid. Yield: 58.5mg, 63%. (LC/MS ret. time:1.93min M+1=561). ¹HNMR (CD₃OD, 500MHz) *δ* 8.58 (s, 1 H), 8.01-8.02 (m, 1 H), 7.94-7.96 (m, 1 H), 7.39-7.40 (m, 1 H), 7.13-7.19 (m, 2 H), 7.01-7.06(m, 1 H), 5.51-5.55 (m, 1 H), 4.91 (s, 3 H), 3.63-3.77(m, 3 H), 3.43-3.46 (m, 1 H), 2.81-3.04 (m, 5 H), 2.57-2.63 (m, 1 H), 1.68-2.26 (m, 8 H), 1.31-1.47 (m, 2 H).

### Biological Methodologies

In some embodiments, compounds of Formula I exhibit a broad spectrum of antibacterial activity and/or are effective against a variety of infectious strains of interest, including resistant strains. The ability of the compounds of Formula I or their pharmaceutically acceptable salts thereof to generally demonstrate their effectiveness for treating disorders or conditions characterized by microbial infections is shown by the following conventional in vitro assay tests described below.

Activity against bacterial and protozoa pathogens can be demonstrated by a compound's ability to inhibit growth of defined strains of pathogens at the particular dose(s) tested. The assays described herein include a panel of bacterial isolates of *Staphylococcus aureus*. Bacterial pathogens that comprise the various screening panels are shown in Table 11. Assay 1 comprises the strains noted in Columns A through E, and Assay 2 comprises the strains noted in Columns F through H. The assays are performed in microtiter trays according to Methods for Dilution Antimicrobial Susceptibility Tests for Bacteria that Grow Aerobically; Approved Standard-7^{th} edition (M7-A7) and interpreted according to the Performance Standards for Antimicrobial Susceptibility Testing; 16^{th} Informational Supplement (M100-S16) published by Clinical Laboratory Standards Institute (CLSI). The antibacterial activity is presented in the form of a minimum inhibitory concentration (MIC) value in µg/ml format. The MIC value represents the lowest concentration of drug measured which prevented macroscopically visible growth under the conditions and specific doses tested. The compounds were initially dissolved in DMSO as 30 mM stocks and diluted accordingly to adjust to a concentration of 10 mg/ml or the compounds were dissolved in DMSO at a concentration of 10 mg/ml. For assessment of activity in the presence of human serum, *S*. *aureus* 1146 and *S*. *aureus* 1031 were inoculated into Mueller-Hinton Broth with 50% pooled inactivated human serum. In some cases, compounds were tested more than once in a particular assay. Where noted, the MIC value shown in Table 12 represents the geometric mean of the data from multiple runs.

**TABLE 11**

| Strain designation | Column No. | +/- serum |
|---|---|---|
| Staph aureus 1095 MRSA | A | - |
| Staph aureus 1146 | B | - |
| Staph aureus 1146 | C | |
| Staph aureus 2811 | D | - |
| Staph aureus 2812 | E | - |
| Staph aureus 1031 | F | - |
| Staph aureus 1031 | G | + |
| Staph aureus 2810 | H | - |

Table 12 provides the in vitro assay data obtained for various Examples described above and/or listed in Tables 2 through 9. It should be understood that the data in columns A-H represents MIC data in µg/ml and the notation "<=" means 'less than or equal to'.

**TABLE 12**

| **Ex #** | **A** | **B** | **C** | **D** | **E** | **F** | **G** | **H** |
|---|---|---|---|---|---|---|---|---|
| 1* | <= .0992 | <= .0717 | 0.3969 | <= .0787 | <= .0992 | <= .0884 | | 0.125 |
| 2* | 0.25 | <= .0625 | 0.4629 | <= .0884 | 0.1575 | 0.125 | 0.5 | <=.0 625 |
| 3* | 0.25 | <= .1768 | 1.7818 | <= .25 | 0.25 | 0.5 | 4 | 0.5 |
| 4 | 2 | 1 | 4 | 1 | 1 | 0.5 | 4 | 0.5 |
| 5 | 2 | 4 | 32 | 8 | 4 | 4 | 64 | 8 |
| 6 | 2 | 1 | 16 | 2 | 1 | | | |
| 7 | 0.25 | 0.125 | 1 | 0.125 | 0.125 | | | |
| 8 | 0.25 | 0.25 | 8 | 0.125 | 0.25 | | | |
| 10 | 1 | 0.5 | 16 | 0.5 | 0.5 | | | |
| 11 | 1 | 2 | >64 | 2 | 2 | | | |
| 12 | 4 | 2 | 8 | 2 | 2 | | | |
| 13 | 4 | 4 | 32 | 2 | 2 | | | |
| 14* | <= .0947 | 0.1575 | <= .3789 | <= .0947 | <= .0947 | | | |
| 15* | 0.0884 | 0.0156 | 0.25 | 0.0625 | 0.03125 | | | |
| 16* | 0.125 | <= .0884 | 1 | 0.125 | 0.125 | | | |
| 17 | <= .0625 | <= .0625 | 0.5 | <= .0625 | <= .0625 | | | |
| 18 | <= .0625 | <= .0625 | 0.25 | <= .0625 | <= .0625 | | | |
| 19 | <= .0625 | <= .0625 | 0.5 | <= .0625 | <= .0625 | | | |
| 20 | | | | | | 16 | 64 | 16 |
| 21 | 16 | 4 | 16 | 4 | 4 | | | |
| 22 | 4 | 1 | 8 | 1 | 2 | | | |
| 23 | <= .0625 | <= .0625 | >64 | <= .0625 | <= .0625 | | | |
| 24 | 16 | 4 | >64 | 4 | 8 | | | |
| 25 | 2 | 2 | 4 | 1 | 1 | | | |
| 26 | 1 | 0.25 | 4 | 0.25 | 0.25 | | | |
| 27* | <= .0625 | <= .125 | 0.3535 | <= .0625 | <= .0625 | | | |
| 29 | 0.5 | 0.25 | 4 | 0.25 | 0.25 | | | |
| 30 | 4 | 8 | >64 | 4 | 8 | | | |
| 31 | 16 | 32 | >64 | 32 | 32 | | | |
| 32^{‡} | 64 | >64 | >64 | 64 | 64 | | | |
| 33 | >64 | >64 | >64 | >64 | >64 | | | |
| 34 | 1 | 1 | 16 | 0.5 | 0.5 | | | |
| 35 | 4 | 1 | 64 | 1 | 1 | | | |
| 36 | 0.125 | <= .0625 | 2 | <= .0625 | <= .0625 | | | |
| 37 | 0.25 | <= .0625 | 1 | <= .0625 | 0.25 | | | |
| 38 | 0.25 | 0.125 | 4 | 0.125 | 0.125 | | | |
| 39 | <= .125 | <= .0625 | 1 | 0.25 | <= .0625 | | | |
| 40* | 0.125 | <= .0625 | 1.2599 | <=.0625 | <= .0625 | | | |
| 41^{‡} | >64 | >64 | >64 | >64 | >64 | | | |
| 42 | >64 | >64 | 32 | >64 | 32 | | | |
| 43 | 0.25 | <= .0625 | 0.5 | <=.0625 | <=.0625 | | | |
| 44 | 0.5 | 0.25 | 2 | 0.125 | 0.125 | | | |
| 45* | <=.125 | <= .0884 | 0.3535 | <= .0884 | <= .0884 | | | |
| 46 | 1 | 1 | 8 | 1 | 2 | | | |
| 47 | 0.125 | <= .0625 | 0.5 | <=.0625 | 0.125 | | | |
| 48 | 0.25 | 0.125 | 1 | 0.125 | 0.125 | | | |
| 49 | 0.125 | <= .0625 | 0.25 | <=.0625 | <=.0625 | | | |
| 50 | 32 | 32 | 32 | 64 | 64 | | | |
| 51 | 32 | 16 | 16 | 16 | 8 | | | |
| 52 | >64 | 16 | 16 | 8 | 32 | | | |
| 53 | 16 | >64 | >64 | 32 | 16 | | | |
| 54^{‡} | >64 | 64 | >64 | >64 | >64 | | | |
| 55 | 4 | 1 | 32 | 2 | 2 | | | |
| 56 | 2 | 0.5 | 2 | 0.5 | 0.5 | | | |
| 57 | 0.5 | 0.25 | 8 | 0.25 | 0.25 | | | |
| 58 | 0.125 | <=.0625 | 2 | <=.0625 | <=.0625 | | | |
| 59 | <=.0625 | <=.0625 | <=.0625 | <=.0625 | <=.0625 | | | |
| 60 | 0.125 | <=.0625 | 4 | <=.0625 | <=.0625 | | | |
| 61 | 0.125 | <=.0625 | 2 | <=.0625 | <=.0625 | | | |
| 62 | 4 | 2 | 64 | 1 | 2 | | | |
| 63 | 0.125 | <=.0625 | 2 | <=.0625 | <=.0625 | | | |
| 64 | <=.0625 | <=.0625 | 8 | <=.0625 | <=.0625 | | | |
| 65 | 1 | 0.25 | 16 | 0.25 | 1 | | | |
| 66 | 1 | 0.125 | 4 | 0.125 | 0.25 | | | |
| 67 | 0.125 | <=.0625 | 2 | <=.0625 | 0.125 | | | |
| 68 | 0.25 | <=.0625 | 4 | <=.0625 | <=.0625 | | | |
| 69 | 0.5 | 0.125 | 2 | 0.125 | 0.25 | | | |
| 70^{‡} | >64 | >64 | >64 | >64 | >64 | | | |
| 73^{†} | | | | | | >64 | >64 | >64 |
| 74 | | | | | | >64 | >64 | >64 |
| 75 | | | | | | >64 | >64 | >64 |
| 76^{†} | | | | | | >64 | >64 | >64 |
| 77 | | | | | | >64 | >64 | >64 |
| 78^{†} | | | | | | >64 | >64 | >64 |
| 79 | | | | | | 8 | 64 | 8 |
| 80 | | | | | | 1 | >64 | 1 |
| 81 | | | | | | 4 | >64 | 4 |
| 82 | | | | | | 8 | 32 | 16 |
| 83 | | | | | | 8 | 64 | 8 |
| 84 | | | | | | >64 | >64 | >64 |
| 85* | 4 | 1.4142 | 5.6568 | 1.4142 | 2 | 2 | 4 | 2 |
| 86 | <=.0625 | <=.0625 | 8 | <=.0625 | <=.0625 | | | |
| 87 | <=.0625 | <=.0625 | 8 | <=.0625 | <=.0625 | | | |
| 88 | 1 | 0.5 | 4 | 0.25 | 0.25 | | | |
| 89* | <0.0625 | <0.0625 | 0.5 | <0.0625 | <0.0625 | | | |
| 90* | <0.0625 | <0.0625 | 1.0 | <0.0625 | <0.0625 | | | |
| 91 | 0.5 | 0.25 | 8 | 0.25 | 0.25 | | | |
| 92 | 0.125 | <=.0625 | 0.5 | <=.0625 | <=.0625 | | | |
| 93 | 2 | 2 | >64 | 2 | 1 | | | |
| 94 | >64 | 32 | >64 | >64 | >64 | | | |
| 95 | >64 | 16 | >64 | >64 | >64 | | | |
| 96 | >64 | 8 | >64 | >64 | >64 | | | |
| 97 | 32 | 2 | >64 | 8 | 8 | | | |
| 98 | 4 | 2 | 64 | 2 | 4 | | | |
| 99 | 64 | 16 | >64 | 64 | >64 | | | |
| 100 | 0.5 | 0.5 | >64 | 0.25 | 0.5 | | | |
| 101 | 0.125 | 0.125 | 2 | <=.0625 | <=.0625 | | | |
| 102 | 16 | 16 | 64 | 8 | 16 | | | |
| 103 | 0.5 | 0.25 | 4 | 0.25 | 0.25 | | | |
| 104^{‡} | >64 | >64 | >64 | >64 | >64 | | | |
| 105 | 1 | 2 | >64 | 2 | 2 | | | |
| 106 | <=.0625 | <=.0625 | 32 | <=.0625 | <=.0625 | | | |
| 107 | 64 | 64 | >64 | 64 | 16 | | | |
| 108 | <=.0625 | <=.0625 | 64 | <=.0625 | <=.0625 | | | |
| 109 | 1 | 0.5 | >64 | 0.25 | 1 | | | |
| 110 | >64 | 32 | >64 | >64 | >64 | | | |
| 111 | 4 | 2 | >64 | 1 | 1 | | | |
| 112* | 8 | 4 | >46.254 8 | 4 | 5.6568 | | | |
| 112 | 0.25 | <=.0625 | 4 | <=.0625 | <=.0625 | | | |
| 113 | 0.125 | <=.0625 | 16 | 0.125 | 0.25 | | | |
| 114 | 1 | 0.5 | >64 | 0.5 | 0.5 | | | |
| 115 | 32 | 8 | 64 | 8 | 16 | | | |
| 116 | | | | | | >64 | >64 | >64 |
| 117 | | | | | | >64 | >64 | >64 |
| 118 | | | | | | >64 | >64 | >64 |
| 119 | | | | | | >64 | >64 | >64 |
| 120 | | | | | | >64 | >64 | >64 |
| 121 | 2 | 1 | >64 | 1 | 1 | | | |
| 122 | 4 | 4 | 16 | 1 | 2 | | | |
| 123 | 32 | 16 | 32 | 16 | 32 | | | |
| 124 | | | | | | >64 | >64 | >64 |
| 125 | | | | | | >64 | >64 | 32 |
| 126 | >64 | 8 | >64 | 2 | 4 | | | |
| 127 | >64 | >64 | >64 | >64 | >64 | | | |
| 128 | >64 | >64 | >64 | >64 | >64 | | | |
| 129 | >64 | >64 | >64 | >64 | >64 | | | |
| 130 | >64 | >64 | >64 | >64 | >64 | | | |
| 131^{‡} | >64 | >64 | >64 | >64 | >64 | | | |
| 132^{‡} | >64 | >64 | >64 | >64 | >64 | | | |
| 133 | 0.25 | <=.0625 | 2 | 0.125 | <=.0625 | | | |
| 134 | <=.0625 | <=.0625 | 4 | <=.0625 | <=.0625 | | | |
| 135 | 0.25 | 0.25 | 8 | 0.125 | 0.25 | | | |
| 136 | 8 | 4 | 4 | 4 | 8 | | | |
| 137 | >64 | 64 | >64 | 64 | 64 | | | |
| 138 | 2 | 1 | 0.5 | 0.5 | 2 | | | |
| 139 | 2 | 2 | 16 | 4 | 2 | | | |
| 140 | 16 | 16 | 64 | 16 | 16 | | | |
| 141 | 2 | 1 | 32 | 0.5 | 1 | | | |
| 142 | 32 | 16 | >64 | 8 | 16 | | | |
| 143 | 1 | 0.5 | 2 | 0.5 | 1 | | | |
| 144 | <=.0625 | <=.0625 | 0.25 | <=.0625 | <=.0625 | | | |
| 145 | 0.25 | 1 | 4 | 0.5 | 1 | | | |
| 146 | 32 | 16 | >64 | 32 | 32 | | | |
| 147 | 8 | 1 | 4 | 1 | 1 | | | |
| 148 | 4 | 1 | 4 | 1 | 1 | | | |
| 149 | 8 | 2 | 4 | 2 | 2 | | | |
| 150 | 4 | 2 | 4 | 2 | 2 | | | |
| 151 | 8 | 4 | 32 | 2 | 4 | 2 | 64 | 2 |
| 152 | <=.0625 | 0.125 | 1 | <=.0625 | <=.0625 | | | |
| 152 | 0.25 | 0.125 | 2 | <=.0625 | 0.25 | I <= .0625 | 2 | 0.125 |
| 153 | 4 | 1 | 2 | 0.5 | 0.5 | | | |
| 154 | 0.25 | 0.0625 | 4 | 0.0625 | 0.0625 | | | |
| 155 | 1 | 0.125 | 4 | 0.25 | 0.125 | | | |
| 156 | 1 | 0.25 | >33 | 0.5 | 0.25 | | | |
| 157 | 4 | 1 | >33 | 1 | 0.5 | | | |
| 158 | | | | | | 32 | >64 | 32 |
| 159 | | | | | | 4 | 16 | 4 |
| 160 | | | | | | 2 | 4 | 2 |
| 162 | 64 | 64 | >64 | 32 | 64 | | | |
| 163 | >64 | 64 | >64 | >64 | >64 | | | |
| 164 | >64 | 64 | >64 | 64 | 64 | | | |
| 165 | >64 | 32 | >64 | 32 | 64 | | | |
| 166 | >64 | >64 | >64 | >64 | >64 | | | |
| 167 | >64 | >64 | >64 | >64 | >64 | | | |
| 168 | >64 | >64 | >64 | >64 | >64 | | | |
| 169 | >64 | >64 | >64 | >64 | >64 | | | |
| 170 | >64 | >64 | >64 | >64 | >64 | | | |
| 171 | 16 | 8 | 16 | 8 | 8 | | | |
| 172 | 4.00 | 2.00 | 32.0 | 1.00 | 2.00 | | | |
| 173 | <0.0625 | <0.0625 | 8.00 | <0.0625 | <0.0625 | | | |
| 174 | <0.0625 | <0.0625 | 4.00 | <0.0625 | <0.0625 | | | |
| 175 | 0.500 | <0.0625 | 4.00 | <0.0625 | <0.0625 | | | |
| 176 | 0.125 | <0.0625 | 4.00 | <0.0625 | <0.0625 | | | |
| 177 | 8.00 | 4.00 | 32.0 | 4.00 | 8.00 | | | |
| 178 | 8.00 | 2.00 | 32.0 | 2.00 | 4.00 | | | |
| 179 | 2.00 | 1.00 | 32.0 | 1.00 | 1.00 | | | |
| 180 | <0.0625 | <0.0625 | 16.0 | <0.0625 | <0.0625 | | | |
| 181 | <0.0625 | <0.0625 | 16.0 | <0.0625 | <0.0625 | | | |
| 182 | <0.0625 | <0.0625 | 1.00 | <0.0625 | <0.0625 | | | |
| 183 | 2.00 | 0.250 | 8.00 | 0.250 | 0.500 | | | |
| 184 | 0.250 | <0.0625 | 8.00 | <0.0625 | <0.0625 | | | |
| 185 | 8.00 | 2.00 | >64.0 | 8.00 | 4.00 | | | |
| 186 | <0.0625 | <0.0625 | 2.00 | <0.0625 | <0.0625 | | | |
| 187 | <0.0625 | <0.0625 | 1.00 | <0.0625 | <0.0625 | | | |
| 188 | <0.0625 | <0.0625 | 0.500 | <0.0625 | <0.0625 | | | |
| 189 | <0.0625 | <0.0625 | 0.125 | <0.0625 | <0.0625 | | | |
| 190 | <0.0625 | <0.0625 | 1.00 | <0.0625 | 0.125 | | | |
| 191 | 0.125 | <0.0625 | 1.00 | <0.0625 | <0.0625 | | | |
| 192 | 4.00 | 1.00 | 32.0 | 1.00 | 2.00 | | | |
| 193 | 0.500 | 0.125 | 64.0 | 0.125 | 0.125 | | | |
| 194 | 4.00 | 2.00 | >64.0 | 2.00 | 2.00 | | | |
| 195 | <0.0625 | <0.0625 | 1.00 | <0.0625 | <0.0625 | | | |
| 196 | 8.00 | 1.00 | 64.0 | 1.00 | 2.00 | | | |
| 197 | 0.500 | <0.0625 | 4.00 | <0.0625 | <0.0625 | | | |
| 198 | 0.125 | <0.0625 | >64.0 | <0.0625 | <0.0625 | | | |
| 199 | 0.125 | <0.0625 | >64.0 | <0.0625 | <0.0625 | | | |
| 200 | 32.0 | 8.00 | >64.0 | 8.00 | 8.00 | | | |
| 201 | 16.0 | 2.00 | 8.00 | 2.00 | 4.00 | | | |
| 202 | 8.00 | 2.00 | 64.0 | 2.00 | 4.00 | | | |
| 203 | 0.250 | 0.125 | 16.0 | 0.125 | 0.125 | | | |
| 204 | 0.250 | 0.125 | 4.00 | 0.125 | 0.250 | | | |
| 205 | <0.0625 | <0.0625 | 2.00 | <0.0625 | <0.0625 | | | |
| 206 | 4.00 | 2.00 | 16.0 | 2.00 | 4.00 | | | |
| 207 | <0.0625 | <0.0625 | 64.0 | <0.0625 | <0.0625 | | | |
| 208 | 64.0 | 32.0 | >64.0 | 32.0 | 64.0 | | | |
| 209 | <0.0625 | <0.0625 | 1.00 | <0.0625 | <0.0625 | | | |
| 210 | <0.0625 | <0.0625 | 16.0 | <0.0625 | <0.0625 | | | |
| 211 | <0.0625 | <0.0625 | 0.250 | <0.0625 | <0.0625 | | | |
| 212 | <0.0625 | <0.0625 | 2.00 | <0.0625 | <0.0625 | | | |
| 213 | <0.0625 | <0.0625 | 0.500 | <0.0625 | <0.0625 | | | |
| 214 | <0.0625 | <0.0625 | 2.00 | <0.0625 | <0.0625 | | | |
| 215 | <0.0625 | <0.0625 | 1.00 | <0.0625 | <0.0625 | | | |
| 216 | >64.0 | 16.0 | 64.0 | 8.00 | 32.0 | | | |
| 217 | 0.125 | 0.0600 | 2.00 | 0.125 | 0.0600 | | | |
| 218 | 0.125 | <0.0625 | 2.00 | <0.0625 | <0.0625 | | | |
| 219 | 0.250 | <0.0625 | 2.00 | <0.0625 | 0.125 | | | |
| 220 | 64.0 | 32.0 | >64.0 | 16.0 | 32.0 | | | |
| 221 | 1.00 | 0.250 | 4.00 | 0.250 | 0.250 | | | |
| 222 | 0.250 | <0.0625 | 2.00 | <0.0625 | <0.0625 | | | |
| 223 | 16.0 | 4.00 | 32.0 | 4.00 | 8.00 | | | |
| 224 | 2 | 0.25 | 8 | 0.25 | 0.5 | | | |
| 225 | 1 | 0.25 | 2 | 0.25 | 0.5 | | | |
| 226 | 16 | 4 | 32 | 4 | 8 | | | |
| 227 | 8 | 2 | 64 | 2 | 8 | | | |
| 229 | 2 | 1 | 32 | 1 | 2 | | | |
| 229 | 0.125 | 0.125 | 32 | 0.125 | 0.25 | | | |

| | | | | | | | | |
|---|---|---|---|---|---|---|---|---|
| * Example tested more than once; MIC data represents geometric mean. ^{†} Example had an MIC in the range of between 1 *µ*g/ml to 64 *µ*g/ml against *Streptococcus pneumoniae* 1531 for the doses tested. ^{‡} Example had an MIC in the range of between 16 *µ*g/ml to 64 *µ*g/ml against *Streptococcus pyogenes* 1079 for the doses tested. | | | | | | | | |

## Claims

1. A compound of the formula: 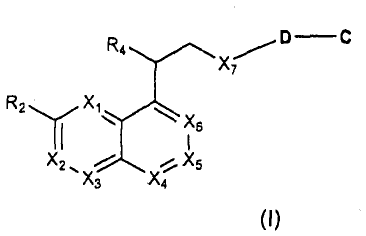 or a pharmaceutically acceptable salt or prodrug thereof or a hydrate or solvate of such compound, salt or prodrug wherein:
at least one of X₁, X₂, X₃, X₄, X₅, or X₆ is selected from N or N-oxide and the remaining are selected from N or CR₁;
each R₁ is independently selected from hydrogen, halogen, cyano, (C₁-C₆)alkyl, (C₁-C₆)alkoxy, halo(C₁-C₆)alkyl, amino, hydroxyl, thiol, or (C₁-C₆)alkylthio;
R₂ is independently selected from hydrogen, hydroxyl, halogen, amino, (C₁-C₆)alkyl, (C₁-C₆)alkylthio, (C₃-C₁₀)cycloalkyl, (C₃-C₁₀)cycloalkyl(C₁-C₆)alkyl, (C₁-C₆)alkyl(C₃-C₁₀)cycloalkyl, (C₂-C₉)heterocycloalkyl, (C₂-C₉)heterocyclo(C₁-C₆)alkyl, (C₆-C₁₀)aryloxy, (C₂-C₉)heterocycloxy, (C₂-C₉)heterocyclo(C₁-C₆)alkoxy, (C₁-C₆)alkoxy, (C₁-C₆)alkoxy(C₁-C₆)alkyl, fluoromethyl, difluoromethyl, trifluoromethyl, fluoromethoxy, difluoromethoxy, trifluoromethoxy, (C₃-C₁₀)cycloalkyloxy, (C₃-C₁₀)cycloalkylthio, (C₁-C₆)acyloxy, cyano, nitro, where any of the aforementioned groups (with the exception of hydrogen, halogen, hydroxyl, cyano, and nitro) is optionally substituted with at least one moiety selected from (C₁-C₆)alkyl, (C₃-C₁₀)cycloalkyl, (C₁-C₆)alkoxy, (C₃-C₁₀)cycloalkoxy, (C₆-C₁₀)aryl, (C₅-C₉)heteroaryl, carboxyl, (C₁-C₆)alkyloxycarbonyl, (C₃-C₁₀)cycloalkyloxycarbonyl, (C₁-C₆)acyl, halogen, halo(C₁-C₆)alkyl, halo(C₁-C₆)alkoxy, (C₁-C₆)alkylsulfonyl, aminocarbonyl, ((C₁-C₆)alkyl)aminocarbonyl, ((C₁-C₆)alkyl)₂aminocarbonyl, hydroxyl, (C₂-C₉)heterocycloxy, (C₆-C₁₀)aryloxy, or (C₁-C₆)acyloxy;
X₇ is selected from O, NR₅, CH₂, -S-, SO, or SO₂ or -CR₅H-;
R₄ is selected from hydrogen, hydroxyl, (C₁-C₆)alkoxy, fluoro, NH₂, ((C₁-C₆)alkyl)NH , ((C₁-C₆)alkyl)₂N or (C₂-C₉)heterocycloalkyl, cyano, or (C₁-C₆)alkylthio;
R₅ is selected from hydrogen, (C₁-C₆)alkyl, (C₁-C₆)alkyloxycarbonyl, aminocarbonyl, (C₁-C₆)alkylsutfonyl, or (C₁-C₆)alkylcarbonyl;
**D** is 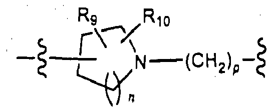
**C** is selected from 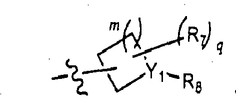 wherein " 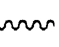 " indicates a point of attachment;
Y₁ is CR₆ where R₆ is selected from hydrogen, hydroxyl, halogen, (C₁-C₆)alkyl or R₇; or
Y₁ is N; and
wherein one of the carbon ring atoms of each of the foregoing C ring groups, together with the group to which it is attached, may optionally be replaced by -C(O)-;
each R₇ is independently selected from hydrogen, halogen, hydroxyl, (C₁-C₆)alkyl, (C₁-C₆)alkoxy, trifluoromethyl, trifluoromethoxy, or amino provided that when Y₁ is N and R₇ is hydroxyl, (C₁-C₆)alkoxy, amino, trifluoromethoxy , or halogen, R₇ may not be located on an atom adjacent to Y₁;
R₈ is selected from (C₆-C₁₀)aryl, (C₆-C₁₀)aryloxy, (C₆-C₁₀)aryl(C₁-C₆)alkyl, (C₆-C₁₀)aryl(C₁-C₆)alkoxy, (C₃-C₁₀)cycloalkyl, (C₃-C₁₀)cycloalkoxy, (C₃-C₁₀)cycloalkyl(C₁-C₆)alkoxy, C₃-C₁₀)cycloalkyl(C₁-C₆)alkyl, (C₅-C₉)heteroaryl(C₁-C₆)alkyl, (C₅-C₉)heteroaryl, (C₅-C₉)heteroaryl(C₁-C₆)alkoxy, (C₆-C₉)heteroaryloxy, (C₃-C₁₀)cycloalkoxy(C₁-C₆)alkyl, (C₂-C₉)heterocycloalkyl, (C₂-C₉)heterocycloxy, (C₂-C₉)heterocyclo(C₁-C₆)alkyl, (C₂-C₉)heterocyclo(C₁-C₆)alkoxy, where any of the aforementioned groups may be optionally substituted with 1 to 4 moieties each independently selected from halogen, (C₁-C₆)alkyl, (C₁-C₆)alkoxy, (C₁-C₆)alkoxy(C₁-C₆)alkyl, carboxyl, halo(C₁-C₆)alkyl, halo(C₁-C₆)alkoxy, thiol, (C₁-C₆)alkylthio, hydroxyl, nitro, cyano, amino, mono- or di-(C₁-C₆)alkylamino, (C₆-C₁₀)aryl, (C₅-C₉)heteroaryl, (C₁-C₆)alkoxycarbonyl, (C₁-C₆)alkoxycarbonyl(C₁-C₆)alkyl, (C₃-C₁₀)cycloalkyl, (C₁-C₆)alkylcarbonyl, (C₁-C₆)alkylsulfinyl, (C₁-C₆)alkylsulfonyl, aminocarbonyl, mono- and di-(C₁-C₆)alkylaminocarbonyl, (C₁-C₆)acylthio, or (C₁-C₆)acyloxy;
or R₇ and R₈ together with the atoms to which they are bonded form a three to eight membered saturated or unsaturated or aromatic ring system that may be monocyclic or bicyclic, wherein said ring system may optionally contain at least one heteroatom selected from nitrogen, oxygen or sulfur, and wherein said ring system may be optionally substituted with 1 to 4 moieties each independently selected from hydroxyl, halogen, cyano, (C₁-C₆)alkyl, halo(C₁-C₆)alkyl, (C₁-C₆)alkoxy, halo(C₁-C₆)alkoxy, (C₃-C₁₀)cycloalkyl, (C₃-C₁₀)cycloalkoxy, formyl, (C₁-C₆)acyl, (C₁-C₆)alkoxycarbonyl, (C₂-C₉)heterocycloalkyl, (C₆-C₁₀)aryl, or (C₅-C₉)heteroaryl;
R₉ is selected from carboxyl, (C₁-C₆)alkoxycarbonyl, aminocarbonyl, (C₁-C₆)alkylaminocarbonyl, (C₁-C₆)alkylsulfonylaminocarbonyl, hydroxyl, hydroxymethyl, or tetrazole;
R₁₀ is selected from hydrogen, halogen, hydroxyl, (C₁-C₆)alkyl or halo(C₁-C₆)alkyl;
*n* is 0, 1, 2, or 3;
*m* is 0, 1, 2, or 3;
*p* is 0 or 1; and
*q* is 0, 1 or 2.

2. A compound according to claim 1 wherein:
**D** is selected from

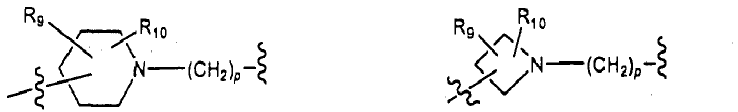

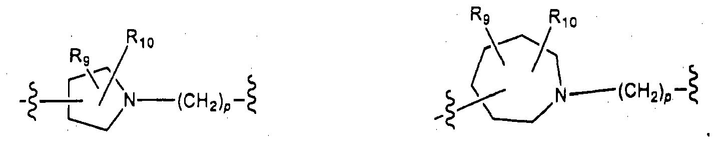

3. A compound according to claim 1 or 2 wherein:
C is selected from 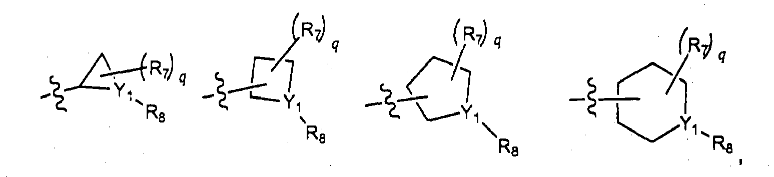
wherein one of the carbon ring atoms of each of the foregoing **C** ring groups, together with the group to which it is attached, may optionally be replaced by -C(O)-.

4. A compound according to claim 1, wherein:
**D** is selected from 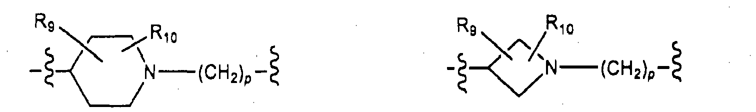 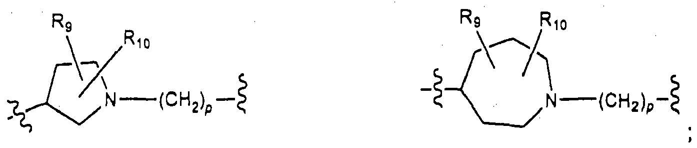 and
**C** is selected from

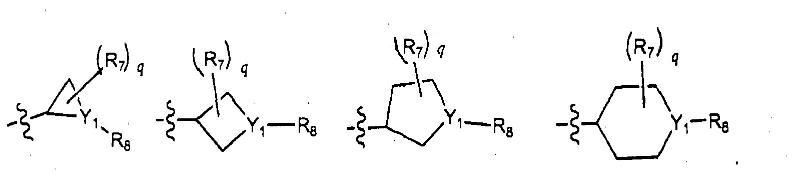

5. A compound according to claim 1 wherein:
two of X₁, X₂, X₃, X₄, X₅, or X₆ are independently selected from N or N-oxide provided that if any one of X₁, X₂, X₃, X₄, X₅, or X₆ is N-oxide, the remaining are independently selected from N or CR₁;
R₄ is selected from hydrogen, cyano, hydroxyl, (C₁-C₆)alkoxy, fluoro, NH₂, ((C₁-C₆)alkyl)NH-, ((C₁-C₆)alkyl)₂N or (C₂-C₉)heterocycloalkyl;
**D** is selected from: 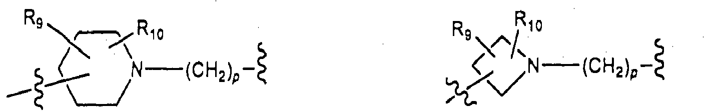 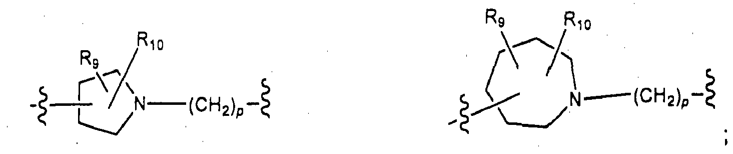 and
**C** is selected from

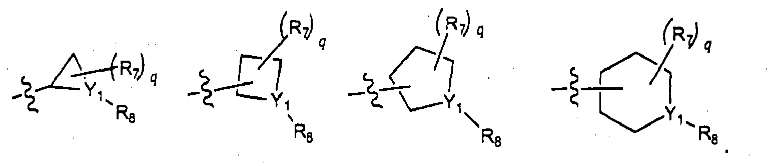

6. A compound according to claim 1, wherein:
two of X₁, X₂, X₃, X₄, X₅, or X₆ are independently selected from N or N-oxide, provided that if any one of X₁, X₂, X₃, X₄, X₅, or X₆ is N-oxide, the remaining are independently selected from N or CR₁;
**D** is selected from 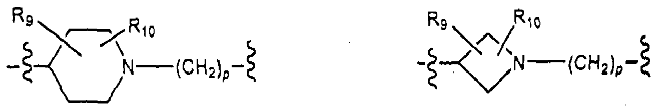 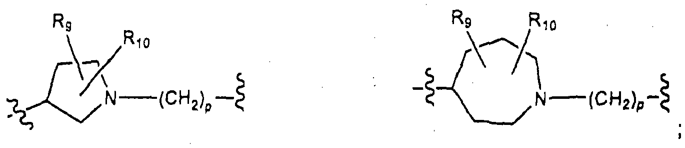 and
**C** is selected from

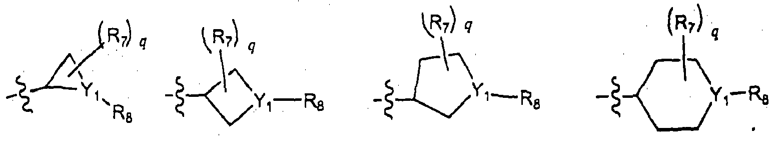

7. A compound according to claim 1, wherein:
X₄ is selected from N or N-oxide;
Y₁ is N;
**C** is selected from 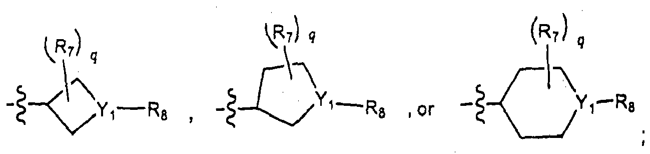 and
R₈ is (C₅-C₉)heteroaryl, (C₅-C₉)heteroaryl(C₁-C₆)alkyl, (C₂-C₉)heterocycloalkyl, (C₃-C₁₀)cycloalkyl, or (C₆-C₁₀)aryl(C₁-C₆)alkyl where any of the aforementioned groups is optionally substituted with 1 to 4 moieties each independently selected from halogen, cyano, (C₁-C₆)alkyl, (C₁-C₆)alkoxy, halo(C₁-C₆)alkyl, halo(C₁-C₆)a(koxy, or hydroxyl.

8. A compound according to claim 1 wherein:
X₄ is selected from N or N-oxide;
R₂ is (C₁-C₆)alkoxy or halo(C₁-C₆)alkoxy;
R₄ is selected from hydrogen, hydroxyl, cyano, (C₁-C₆)alkoxy, fluoro, NH₂, ((C₁-C₆)alkyl)NH-, ((C₁-C₆)alkyl)₂N or (C₂-C₉)heterocycloalkyl;
**C** is selected from 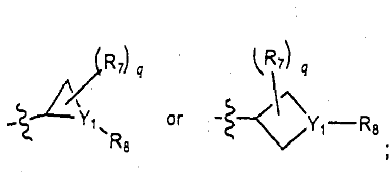 and
R₈ is (C₆-C₁₀)aryl, (C₆-C₁₀)aryl(C₁-C₆)alkyl, (C₆-C₁₀)aryloxy, (C₆-C₁₀)aryl(C₁-C₆)alkoxy, (C₃-C₁₀)cycloalkyl, (C₃-C₁₀)cycloalkyl(C₁-C₆)alkyl, (C₃-C₁₀)cycloalkyl(C₁-C₆)alkoxy, (C₅-C₉)heteroaryl, (C₅-C₉)heteroaryl(C₁-C₆)alkyl, (C₂-C₉)heterocycloalkyl, (C₂-C₉)heterocycloalkyl(C₁-C₆)alkyl, (C₂-C₈)heterocyclo(C₁-C₆)alkoxy, or (C₅-C₉)heteroaryloxy, where any of the aforementioned groups is optionally substituted with 1 to 4 moieties each independently selected from halogen, (C₁-C₆)alkyl, (C₁-C₆)alkoxy, cyano, (C₁-C₆)alkoxy(C₁-C₆)alkyl, halo(C₁-C₆)alkoxy, halo(C₁-C₆)alkyl, (C₃-C₁₀)cycloalkyl, (C₃-C₁₀)cycloalkyl(C₁-C₆)alkyl, or hydroxyl; or
R₇ and R₈ together with the atoms to which they are attached form at least a 5 membered spirocyclic ring or at least a 5 membered carbocylic, aromatic or heteroaromatic ring wherein any of the aforementioned ring systems may be monocyclic or bicyclic, wherein said ring system may optionally contain at least one heteroatom selected from nitrogen, oxygen or sulfur, and wherein said ring systems is optionally substituted with 1 to 4 moieties each independently selected from amino, hydroxyl, halogen, cyano, (C₁-C₆)alkyl, halo(C₁-C₆)alkyl, (C₁-C₆)alkoxy, halo(C₁-C₆)alkoxy, (C₃-C₁₀)cycloalkyl, (C₃-C₁₀)cycloalkyl(C₁-C₆)alkyl, (C₂-C₉)heterocycloalkyl, (C₆-C₁₀)aryl, or (C₅-C₉)heteroaryl.

9. A compound according to claim 1 selected from:
(3R,4R)-4-(3-hydroxy-3-(6-methoxyquinolin-4-yl)propyl)-1-(3-phenylcyclobutyl)piperidine-3-carboxylic acid;
(3R,4R)-4-(3-(3-fluoro-6-methoxyquinolin-4-yl)-3-hydroxypropyl)-1-(3-phenylcyclobutyl)piperidine-3-carboxylic acid;
(3R,4R)-4-(3-(3-chloro-6-methoxyquinolin-4-yl)-3-hydroxypropyl)-1-(3-phenylcyclobutyl)piperidine-3-carboxylic acid;
(3R,4R)-1-(3-(2,6-difluorophenyl)cyclobutyl)-4-(3-hydroxy-3-(6-methoxyquinolin-4-yl)propyl)piperidine-3-carboxylic acid;
(3R,4R)-1-(3-(2,6-difluorophenyl)cyclobutyl)-4-(3-(3-fluoro-6-methoxyquinolin-4-yl)-3-hydroxypropyl)piperidine-3-carboxylic acid;
(3R,4R)-4-(3-(3-chloro-6-methoxyquinolin-4-yl)-3-hydroxypropyl)-1-(3-(2,6-difluorophenyl)cyclobutyl)piperidine-3-carboxylic acid;
(3R,4R)-1-(3-(2,6-difluorophenyl)cyclobutyl)-4-(3-(3-fluoro-6-methoxy-1,5-naphthyridin-4-yl)-3-hydroxypropyl)piperidine-3-carboxylic acid;
(3R,4R)-1-(3-(2,5-difluorophenyl)cyclobutyl)-4-((S)-3-hydroxy-3-(6-methoxyquinolin-4-yl)propyl)piperidine-3-carboxylic acid;
(3R,4R)-1-(3-(2,5-difluorophenyl)cyclobutyl)-4-((S)-3-(3-fluoro-6-methoxyquinolin-4-yl)-3-hydroxypropyl)piperidine-3-carboxylic acid;
(3R,4R)-4-(3-(3-chloro-6-methoxyquinolin-4-yl)-3-hydroxypropyl)-1-(3-(2,5-difluorophenyl)cyclobutyl)piperidine-3-carboxylic acid;
(3R,4R)-1-(3-(2,5-difluorophenyl)cyclobutyl)-4-(3-(3-fluoro-6-methoxy-1,5-naphthyridin-4-yl)-3-hydroxypropyl)piperidine-3-carboxylic acid;
(3R,4R)-1-[3-(3,5-difluorophenyl)cyclobutyl]-4-[(3S)-3-hydroxy-3-(6-methoxyquinolin-4-yl)propyl]piperidine-3-carboxylic acid;
(3R,4R)-1-[3-(3,5-difluorophenyl)cyclobutyl]-4-[(3)-3-(3-fluoro-6-methoxyquinolin-4-yl)-3-hydroxypropyl]piperidine-3-carboxylic acid;
3-(3-(3-chloro-6-methoxyquinolin-4-yl)propyl)-1-(3-(2,5-difluorophenyl)cyclobutyl)pyrrolidine-3-carboxylic acid; or
(3R,4R)-1-[3-(3,5-difluorophenyl)cyclobutyl]-4-[3-(3-fluoro-6-methoxy-1,5-naphthyridin-4-yl)-3-hydroxypropyl]piperidine-3-carboxylic acid.

10. A process for the preparation of a compound, or a pharmaceutically acceptable salt thereof, as claimed in claim 1, comprising condensing a compound of the formula 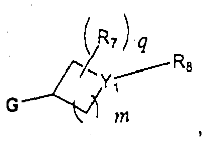 with a heterocyclic derivative of the general formula 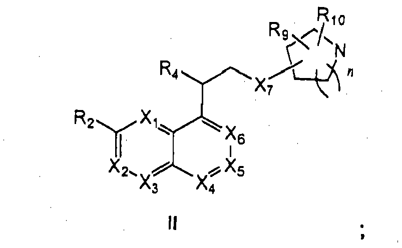 wherein X₁, X₂, X₃, X₄, X₅, X₆, X₇, R₁, R₂, R₄, R₅, R₆, R₇, R₈, R₉, R₁₀, Y₁, *n, m*, *p* and *q* are as defined in claim 1 and G is selected from oxo or 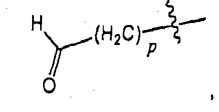 or **G** is a leaving group selected from tosylate, mesylate, triflate, iodo, bromo or chloro.

11. A process for the preparation of a compound, or a pharmaceutically acceptable salt thereof, as claimed in claim 1, comprising condensing a compound of the formula 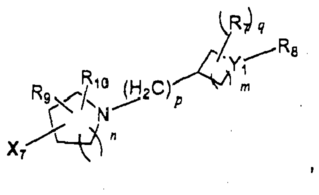 with a bicyclic derivative of the general formula 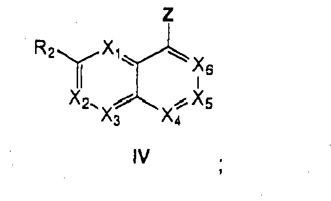 wherein X₁, X₂, X₃, X₄, X₅, X₆, R₁, R₂, R₄, R₅, R₆, R₇, R₈, R₉, R₁₀, Y₁, *n*, *m*, *p* and *q* are as defined in claim 1; **X₇** is amino; and **Z** is selected from
oxirane or

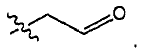

12. A pharmaceutical composition comprising:
a compound of Formula I according to claim 1 or a pharmaceutically acceptable salt or prodrug, or a solvate or hydrate of said compound, salt or prodrug; and
a pharmaceutically acceptable carrier, vehicle, diluent or excipient.

13. A pharmaceutical composition comprising:
a compound of Formula I, according to claim 1 or a pharmaceutically acceptable salt or prodrug, or a solvate or hydrate of said compound, salt or prodrug;
a second therapeutic agent; and
a pharmaceutically acceptable carrier, vehicle, diluent or excipient.

14. A method of treating or preventing bacterial infections in a mammal, including a human, in need of such treatment comprising administering to said mammal a therapeutically effective amount of a compound of the Formula I according to claim 1 or a pharmaceutically acceptable salt or prodrug, or a solvate or hydrate of said compound, salt or prodrug.

15. A method of treating or preventing bacterial infections in a mammal, including a human, in need of such treatment comprising administering to said mammal a therapeutically effective amount of a compound of the Formula I according to claim 1, or a pharmaceutically acceptable salt or prodrug, or a solvate or hydrate of said compound, salt or prodrug, in combination with a second therapeutic agent.
